# EUROPEAN PATENT APPLICATION

(11) **EP 2 522 678 A1**
(43) Date of publication of application: **14.11.2012**
(21) Application number: 12165672.2
(22) Date of filing: 15.05.2007
(51) Int. Cl.: C07K 16/10, A61K 39/42, A61P 31/16, G01N 33/68, C12Q 1/68

(54) **Neutralizing antibodies to influenza viruses**

(30) Priority: 15.05.2006 US 800787 P; 30.10.2006 US 855679 P
(62) Divisional of application: 07762203.3
(71) Applicant: Sea Lane Biotechnologies, LLC, CA 94027 (US)
(72) Inventor: Bhatt, Ramesh, R., Belmont, CA 94002 (US); Horowitz, Lawrence, Atherton, CA 94027 (US); Kashyap, Arun K., Newark, CA 94539 (US)
(74) Representative: Denison, Christopher Marcus

(57) **Abstract**

The present invention concerns methods and means for identifying, producing, and engineering neutralizing anti-bodies against influenza A viruses, and to the neutralizing antibodies produced. In particular, the invention concerns neutralizing antibodies against various influenza A virus subtypes, including neutralizing antibodies against two or more of H1, H2, H3, H5, H7 and H9, such as, for example all of H1, H2, H3, and H5 subtypes, and methods and means for making such antibodies. More specifically, the invention concerns antibodies capable of neutralizing more than one, preferably all, isolates of an influenza A virus subtype.

SEQUENCE LISTING

<110> Sea Lane Biotechnologies, LLC.
      Lawrence Horowitz
      Ramesh Bhatt
      Arun K. Kashyap

<120> Neutralizing Antibodies To Influenza
  Viruses

<130> CMD/FP6820005

<140> Divisional application of 07762203.3
<141> 2007-05-15

<150> 07762203.3
<151> 2007-05-15

<150> PCT/US2007/068983
<151> 2007-05-15

<150> 60/800,787
<151> 2006-05-15

<150> 60/855,697
<151> 2006-10-30

<160> 84

<170> FastSEQ for Windows Version 4.0

<210> 1
<211> 562
<212> PRT
<213> Influenza A virus

<400> 1
Met Ala Ile Ile Tyr Leu Ile Leu Leu Phe Thr Ala Val Arg Gly Asp
 1               5                  10                  15
Gln Ile Cys Ile Gly Tyr His Ala Asn Asn Ser Thr Glu Lys Val Asp
            20                  25                  30
Thr Ile Leu Glu Arg Asn Val Thr Val Thr His Ala Lys Asp Ile Leu
        35                  40                  45
Glu Lys Thr His Asn Gly Lys Leu Cys Lys Leu Asn Gly Ile Pro Pro
    50                  55                  60
Leu Glu Leu Gly Asp Cys Ser Ile Ala Gly Trp Leu Leu Gly Asn Pro
65                  70                  75                  80
Glu Cys Asp Arg Leu Leu Ser Val Pro Glu Trp Ser Tyr Ile Met Glu
                85                  90                  95
Lys Glu Asn Pro Arg Asp Gly Leu Cys Tyr Pro Gly Ser Phe Asn Asp
            100                 105                 110
Tyr Glu Glu Leu Lys His Leu Leu Ser Ser Val Lys His Phe Glu Lys
        115                 120                 125
Val Lys Ile Leu Pro Lys Asp Arg Trp Thr Gln His Thr Thr Thr Gly
    130                 135                 140
Gly Ser Arg Ala Cys Ala Val Ser Gly Asn Pro Ser Phe Phe Arg Asn
145                 150                 155                 160
Met Val Trp Leu Thr Glu Lys Gly Ser Asn Tyr Pro Val Ala Lys Gly
                165                 170                 175
Ser Tyr Asn Asn Thr Ser Gly Glu Gln Met Leu Ile Ile Trp Gly Val
            180                 185                 190
His His Pro Asn Asp Glu Lys Glu Gln Arg Thr Leu Tyr Gln Asn Val
        195                 200                 205
Gly Thr Tyr Val Ser Val Gly Thr Ser Thr Leu Asn Lys Arg Ser Thr

210                 215                 220
Pro Asp Ile Ala Thr Arg Pro Lys Val Asn Gly Leu Gly Ser Arg Met
225                 230                 235                 240
Glu Phe Ser Trp Thr Leu Leu Asp Met Trp Asp Thr Ile Asn Phe Glu
                245                 250                 255
Ser Thr Gly Asn Leu Ile Ala Pro Glu Tyr Gly Phe Lys Ile Ser Lys
            260                 265                 270
Arg Gly Ser Ser Gly Ile Met Lys Thr Glu Gly Thr Leu Glu Asn Cys
        275                 280                 285
Glu Thr Lys Cys Gln Thr Pro Leu Gly Ala Ile Asn Thr Thr Leu Pro
    290                 295                 300
Phe His Asn Val His Pro Leu Thr Ile Gly Glu Cys Pro Lys Tyr Val
305                 310                 315                 320
Lys Ser Glu Lys Leu Val Leu Ala Thr Gly Leu Arg Asn Val Pro Gln
                325                 330                 335
Ile Glu Ser Arg Gly Leu Phe Gly Ala Ile Ala Gly Phe Ile Glu Gly
            340                 345                 350
Gly Trp Gln Gly Met Ile Asp Gly Trp Tyr Gly Tyr His His Ser Asn
        355                 360                 365
Asp Gln Gly Ser Gly Tyr Ala Ala Asp Lys Glu Ser Thr Gln Lys Ala
    370                 375                 380
Phe Asp Gly Ile Thr Asn Lys Val Asn Ser Val Ile Glu Lys Met Asn
385                 390                 395                 400
Thr Gln Phe Glu Ala Val Gly Lys Glu Phe Ser Asn Leu Glu Arg Arg
                405                 410                 415
Leu Glu Asn Leu Asn Lys Lys Met Glu Asp Gly Phe Leu Asp Val Trp
            420                 425                 430
Thr Tyr Asn Ala Glu Leu Leu Val Leu Met Glu Asn Glu Arg Thr Leu
        435                 440                 445
Asp Phe His Asp Ser Asn Val Lys Asn Leu Tyr Asp Lys Val Arg Met
    450                 455                 460
Gln Leu Arg Asp Asn Val Lys Glu Leu Gly Asn Gly Cys Phe Glu Phe
465                 470                 475                 480
Tyr His Lys Cys Asp Asp Glu Cys Met Asn Ser Val Lys Asn Gly Thr
                485                 490                 495
Tyr Asp Tyr Pro Lys Tyr Glu Glu Glu Ser Lys Leu Asn Arg Asn Glu
            500                 505                 510
Ile Lys Gly Val Lys Leu Ser Ser Met Gly Val Tyr Gln Ile Leu Ala
        515                 520                 525
Ile Tyr Ala Thr Val Ala Gly Ser Leu Ser Leu Ala Ile Met Met Ala
    530                 535                 540
Gly Ile Ser Phe Trp Met Cys Ser Asn Gly Ser Leu Gln Cys Arg Ile
545                 550                 555                 560
Cys Ile

<210> 2
<211> 561
<212> PRT
<213> Influenza A virus

<400> 2
Met Tyr Lys Val Val Val Ile Ile Ala Leu Leu Gly Ala Val Lys Gly
1               5                   10                  15
Leu Asp Arg Ile Cys Leu Gly His His Ala Val Ala Asn Gly Thr Ile
            20                  25                  30
Val Lys Thr Leu Thr Asn Glu Gln Glu Glu Val Thr Asn Ala Thr Glu
        35                  40                  45
Thr Val Glu Ser Thr Asn Leu Asn Lys Leu Cys Met Lys Gly Arg Ser
    50                  55                  60
Tyr Lys Asp Leu Gly Asn Cys His Pro Val Gly Met Leu Ile Gly Thr
65                  70                  75                  80
Pro Val Cys Asp Pro His Leu Thr Gly Thr Trp Asp Thr Leu Ile Glu
                85                  90                  95

Arg Glu Asn Ala Ile Ala His Cys Tyr Pro Gly Ala Thr Ile Asn Glu
            100                 105                 110
Glu Ala Leu Arg Gln Lys Ile Met Glu Ser Gly Gly Ile Ser Lys Met
        115                 120                 125
Ser Thr Gly Phe Thr Tyr Gly Ser Ser Ile Thr Ser Ala Gly Thr Thr
    130                 135                 140
Lys Ala Cys Met Arg Asn Gly Gly Asp Ser Phe Tyr Ala Glu Leu Lys
145                 150                 155                 160
Trp Leu Val Ser Lys Thr Lys Gly Gln Asn Phe Pro Gln Thr Thr Asn
                165                 170                 175
Thr Tyr Arg Asn Thr Asp Thr Ala Glu His Leu Ile Ile Trp Gly Ile
            180                 185                 190
His His Pro Ser Ser Thr Gln Glu Lys Asn Asp Leu Tyr Gly Thr Gln
        195                 200                 205
Ser Leu Ser Ile Ser Val Glu Ser Ser Thr Tyr Gln Asn Asn Phe Val
    210                 215                 220
Pro Val Val Gly Ala Arg Pro Gln Val Asn Gly Gln Ser Gly Arg Ile
225                 230                 235                 240
Asp Phe His Trp Thr Leu Val Gln Pro Gly Asp Asn Ile Thr Phe Ser
                245                 250                 255
Asp Asn Gly Gly Leu Ile Ala Pro Ser Arg Val Ser Lys Leu Thr Gly
            260                 265                 270
Arg Asp Leu Gly Ile Gln Ser Glu Ala Leu Ile Asp Asn Ser Cys Glu
        275                 280                 285
Ser Lys Cys Phe Trp Arg Gly Gly Ser Ile Asn Thr Lys Leu Pro Phe
    290                 295                 300
Gln Asn Leu Ser Pro Arg Thr Val Gly Gln Cys Pro Lys Tyr Val Asn
305                 310                 315                 320
Gln Arg Ser Leu Leu Leu Ala Thr Gly Met Arg Asn Val Pro Glu Val
                325                 330                 335
Val Gln Gly Arg Gly Leu Phe Gly Ala Ile Ala Gly Phe Ile Glu Asn
            340                 345                 350
Gly Trp Glu Gly Met Val Asp Gly Trp Tyr Gly Phe Arg His Gln Asn
        355                 360                 365
Ala Gln Gly Thr Gly Gln Ala Ala Asp Tyr Lys Ser Thr Gln Ala Ala
    370                 375                 380
Ile Asp Gln Ile Thr Gly Lys Leu Asn Arg Leu Ile Glu Lys Thr Asn
385                 390                 395                 400
Thr Glu Phe Glu Ser Ile Glu Ser Glu Phe Ser Glu Thr Glu His Gln
                405                 410                 415
Ile Gly Asn Val Ile Asn Trp Thr Lys Asp Ser Ile Thr Asp Ile Trp
            420                 425                 430
Thr Tyr Asn Ala Glu Leu Leu Val Ala Met Glu Asn Gln His Thr Ile
        435                 440                 445
Asp Met Ala Asp Ser Glu Met Leu Asn Leu Tyr Glu Arg Val Arg Lys
    450                 455                 460
Gln Leu Arg Gln Asn Ala Glu Glu Asp Gly Lys Gly Cys Phe Glu Ile
465                 470                 475                 480
Tyr His Thr Cys Asp Asp Ser Cys Met Glu Ser Ile Arg Asn Asn Thr
                485                 490                 495
Tyr Asp His Ser Gln Tyr Arg Glu Glu Ala Leu Leu Asn Arg Leu Asn
            500                 505                 510
Ile Asn Pro Val Lys Leu Ser Ser Gly Tyr Lys Asp Ile Ile Leu Trp
        515                 520                 525
Phe Ser Phe Gly Glu Ser Cys Phe Val Leu Leu Ala Val Val Met Gly
    530                 535                 540
Leu Val Phe Phe Cys Leu Lys Asn Gly Asn Met Arg Cys Thr Ile Cys
545                 550                 555                 560
Ile

<210> 3
<211> 570
<212> PRT

<213> Influenza A virus

<400> 3
Met Asn Thr Gln Ile Ile Val Ile Leu Val Leu Gly Leu Ser Met Val
1               5                   10                  15
Lys Ser Asp Lys Ile Cys Leu Gly His His Ala Val Ala Asn Gly Thr
            20                  25                  30
Lys Val Asn Thr Leu Thr Glu Arg Gly Val Glu Val Val Asn Ala Thr
        35                  40                  45
Glu Thr Val Glu Ile Thr Gly Ile Asp Lys Val Cys Thr Lys Gly Lys
    50                  55                  60
Lys Ala Val Asp Leu Gly Ser Cys Gly Ile Leu Gly Thr Ile Ile Gly
65                  70                  75                  80
Pro Pro Gln Cys Asp Leu His Leu Glu Phe Lys Ala Asp Leu Ile Ile
                85                  90                  95
Glu Arg Arg Asn Ser Ser Asp Ile Cys Tyr Pro Gly Arg Phe Thr Asn
            100                 105                 110
Glu Glu Ala Leu Arg Gln Ile Ile Arg Glu Ser Gly Gly Ile Asp Lys
        115                 120                 125
Glu Ser Met Gly Phe Arg Tyr Ser Gly Ile Arg Thr Asp Gly Ala Thr
    130                 135                 140
Ser Ala Cys Lys Arg Thr Val Ser Ser Phe Tyr Ser Glu Met Lys Trp
145                 150                 155                 160
Leu Ser Ser Ser Met Asn Asn Gln Val Phe Pro Gln Leu Asn Gln Thr
                165                 170                 175
Tyr Arg Asn Thr Arg Lys Glu Pro Ala Leu Ile Val Trp Gly Val His
            180                 185                 190
His Ser Ser Ser Leu Asp Glu Gln Asn Lys Leu Tyr Gly Thr Gly Asn
        195                 200                 205
Lys Leu Ile Thr Val Gly Ser Ser Lys Tyr Gln Gln Ser Phe Ser Pro
    210                 215                 220
Ser Pro Gly Ala Arg Pro Lys Val Asn Gly Gln Ala Gly Arg Ile Asp
225                 230                 235                 240
Phe His Trp Met Leu Leu Asp Pro Gly Asp Thr Val Thr Phe Thr Phe
                245                 250                 255
Asn Gly Ala Phe Ile Ala Pro Asp Arg Ala Thr Phe Leu Arg Ser Asn
            260                 265                 270
Ala Pro Ser Gly Ile Glu Tyr Asn Gly Lys Ser Leu Gly Ile Gln Ser
        275                 280                 285
Asp Ala Gln Ile Asp Glu Ser Cys Glu Gly Glu Cys Phe Tyr Ser Gly
    290                 295                 300
Gly Thr Ile Asn Ser Pro Leu Pro Phe Gln Asn Ile Asp Ser Arg Ala
305                 310                 315                 320
Val Gly Lys Cys Pro Arg Tyr Val Lys Gln Ser Ser Leu Pro Leu Ala
                325                 330                 335
Leu Gly Met Lys Asn Val Pro Glu Lys Ile Arg Thr Arg Gly Leu Phe
            340                 345                 350
Gly Ala Ile Ala Gly Phe Ile Glu Asn Gly Trp Glu Gly Leu Ile Asp
        355                 360                 365
Gly Trp Tyr Gly Phe Arg His Gln Asn Ala Gln Gly Gln Gly Thr Ala
    370                 375                 380
Ala Asp Tyr Lys Ser Thr Gln Ala Ala Ile Asp Gln Ile Thr Gly Lys
385                 390                 395                 400
Leu Asn Arg Leu Ile Glu Lys Thr Asn Lys Gln Phe Glu Leu Ile Asp
                405                 410                 415
Asn Glu Phe Thr Glu Val Glu Gln Gln Ile Gly Asn Val Ile Asn Trp
            420                 425                 430
Thr Arg Asp Ser Leu Thr Glu Ile Trp Ser Tyr Asn Ala Glu Leu Leu
        435                 440                 445
Val Ala Met Glu Asn Gln His Thr Ile Asp Leu Ala Asp Ser Glu Met
    450                 455                 460
Asn Lys Leu Tyr Glu Arg Val Arg Arg Gln Leu Arg Glu Asn Ala Glu
465                 470                 475                 480
Glu Asp Gly Thr Gly Cys Phe Glu Ile Phe His Arg Cys Asp Asp Gln
                485                 490                 495

Cys Met Glu Ser Ile Arg Asn Asn Thr Tyr Asn His Thr Glu Tyr Arg
                500                 505                 510
Gln Glu Ala Leu Gln Asn Arg Ile Met Ile Asn Pro Val Lys Leu Ser
            515                 520                 525
Ser Gly Tyr Lys Asp Val Ile Leu Trp Phe Ser Phe Gly Ala Ser Cys
    530                 535                 540
Val Met Leu Leu Ala Ile Ala Met Gly Leu Ile Phe Met Cys Val Lys
545                 550                 555                 560
Asn Gly Asn Leu Arg Cys Thr Ile Cys Ile
                565                 570

<210> 4
<211> 560
<212> PRT
<213> Influenza A virus

<400> 4
Met Asn Thr Gln Ile Leu Ile Leu Ala Leu Val Ala Ile Ile Pro Thr
1               5                   10                  15
Asn Ala Asp Lys Ile Cys Leu Gly His His Ala Val Ser Asn Gly Ala
            20                  25                  30
Lys Val Asn Thr Leu Thr Glu Arg Gly Val Glu Val Val Asn Ala Thr
        35                  40                  45
Glu Thr Val Glu Arg Thr Asn Val Pro Arg Ile Cys Ser Lys Gly Lys
    50                  55                  60
Arg Thr Val Asp Leu Gly Gln Cys Gly Leu Leu Gly Thr Ile Thr Gly
65                  70                  75                  80
Pro Pro Gln Cys Asp Gln Phe Leu Glu Phe Ser Ala Asp Leu Ile Ile
                85                  90                  95
Glu Arg Arg Glu Gly Asn Asp Val Cys Tyr Pro Gly Lys Phe Val Asn
            100                 105                 110
Glu Glu Ala Leu Arg Gln Ile Leu Arg Lys Ser Gly Gly Ile Asp Lys
        115                 120                 125
Glu Thr Met Gly Phe Thr Tyr Ser Gly Ile Arg Thr Asn Gly Ala Thr
    130                 135                 140
Ser Ala Cys Arg Arg Ser Gly Ser Ser Phe Tyr Ala Glu Met Lys Trp
145                 150                 155                 160
Leu Leu Ser Asn Thr Asp Asn Ala Ala Phe Pro Gln Met Thr Lys Ser
                165                 170                 175
Tyr Lys Asn Ile Arg Lys Asp Pro Ala Leu Ile Ile Trp Gly Ile His
            180                 185                 190
His Ser Gly Ser Thr Ala Glu Gln Thr Lys Leu Tyr Gly Ser Gly Asn
        195                 200                 205
Lys Leu Ile Thr Val Gly Ser Ser Asn Tyr Gln Gln Ser Phe Val Pro
    210                 215                 220
Ser Pro Gly Ala Arg Pro Gln Val Asn Gly Gln Ser Gly Arg Ile Asp
225                 230                 235                 240
Phe His Trp Leu Met Leu Asn Pro Asn Asp Thr Val Thr Phe Ser Phe
                245                 250                 255
Asn Gly Ala Phe Ile Ala Pro Asp Arg Ala Ser Phe Leu Arg Gly Lys
            260                 265                 270
Ser Met Gly Ile Gln Ser Glu Val Gln Val Asp Ala Asn Cys Glu Gly
        275                 280                 285
Asp Cys Tyr His Ser Gly Gly Thr Ile Leu Ser Ser Leu Pro Phe Gln
    290                 295                 300
Asn Ile Asn Ser Arg Thr Val Gly Glu Cys Pro Arg Tyr Val Lys Gln
305                 310                 315                 320
Glu Ser Leu Leu Leu Ala Thr Gly Met Lys Asn Val Pro Glu Ile Pro
                325                 330                 335
Lys Gly Arg Gly Leu Phe Gly Ala Ile Ala Gly Phe Ile Glu Asn Gly
            340                 345                 350
Trp Glu Gly Leu Val Asp Gly Trp Tyr Gly Phe Arg His Gln Asn Ala
        355                 360                 365
Gln Gly Glu Gly Thr Ala Ala Asp Tyr Lys Ser Thr Gln Ser Ala Ile

370                 375                 380
Asp Gln Ile Thr Gly Lys Leu Asn Arg Leu Ile Glu Lys Thr Asn Gln
385                 390                 395                 400
Gln Phe Glu Leu Ile Asp Asn Glu Phe Thr Glu Val Glu Lys Gln Ile
                405                 410                 415
Gly Asn Val Ile Asn Trp Thr Arg Asp Ser Leu Thr Glu Met Trp Ser
            420                 425                 430
Tyr Asn Ala Glu Leu Leu Val Ala Met Glu Asn Gln His Thr Ile Asp
        435                 440                 445
Leu Ala Asp Ser Glu Met Asn Lys Leu Tyr Glu Arg Val Arg Arg Gln
    450                 455                 460
Leu Arg Glu Asn Ala Glu Glu Asp Gly Thr Gly Cys Phe Glu Ile Phe
465                 470                 475                 480
His Lys Cys Asp Asp Asp Cys Met Ala Ser Ile Arg Asn Asn Thr Tyr
                485                 490                 495
Asp His Ser Lys Tyr Arg Glu Glu Ala Ile Gln Asn Arg Ile Gln Ile
            500                 505                 510
Asp Pro Val Lys Leu Ser Ser Gly Tyr Lys Asp Val Ile Leu Trp Phe
        515                 520                 525
Ser Phe Gly Ala Ser Cys Phe Ile Leu Leu Ala Ile Ala Met Gly Leu
    530                 535                 540
Val Phe Ile Cys Val Lys Asn Gly Asn Met Arg Cys Thr Ile Cys Ile
545                 550                 555                 560


<210> 5
<211> 566
<212> PRT
<213> Influenza A virus

<400> 5
Met Glu Ala Arg Leu Leu Val Leu Leu Cys Ala Phe Ala Ala Thr Asn
1               5                   10                  15
Ala Asp Thr Ile Cys Ile Gly Tyr His Ala Asn Asn Ser Thr Asp Thr
            20                  25                  30
Val Asp Thr Val Leu Glu Lys Asn Val Thr Val Thr His Ser Val Asn
        35                  40                  45
Leu Leu Glu Asp Ser His Asn Gly Lys Leu Cys Lys Leu Lys Gly Ile
    50                  55                  60
Ala Pro Leu Gln Leu Gly Lys Cys Asn Ile Ala Gly Trp Leu Leu Gly
65                  70                  75                  80
Asn Pro Glu Cys Asp Leu Leu Leu Thr Ala Ser Ser Trp Ser Tyr Ile
                85                  90                  95
Val Glu Thr Ser Asn Ser Glu Asn Gly Thr Cys Tyr Pro Gly Asp Phe
            100                 105                 110
Ile Asp Tyr Glu Glu Leu Arg Glu Gln Leu Ser Ser Val Ser Ser Phe
        115                 120                 125
Glu Lys Phe Glu Ile Phe Pro Lys Thr Ser Ser Trp Pro Asn His Glu
    130                 135                 140
Thr Thr Lys Gly Val Thr Ala Ala Cys Ser Tyr Ala Gly Ala Ser Ser
145                 150                 155                 160
Phe Tyr Arg Asn Leu Leu Trp Leu Thr Lys Lys Gly Ser Ser Tyr Pro
                165                 170                 175
Lys Leu Ser Lys Ser Tyr Val Asn Asn Lys Gly Lys Glu Val Leu Val
            180                 185                 190
Leu Trp Gly Val His His Pro Pro Thr Gly Thr Asp Gln Gln Ser Leu
        195                 200                 205
Tyr Gln Asn Ala Asp Ala Tyr Val Ser Val Gly Ser Ser Lys Tyr Asn
    210                 215                 220
Arg Arg Phe Thr Pro Glu Ile Ala Ala Arg Pro Lys Val Arg Asp Gln
225                 230                 235                 240
Ala Gly Arg Met Asn Tyr Tyr Trp Thr Leu Leu Glu Pro Gly Asp Thr
                245                 250                 255
Ile Thr Phe Glu Ala Thr Gly Asn Leu Ile Ala Pro Trp Tyr Ala Phe
            260                 265                 270

Ala Leu Asn Arg Gly Ser Gly Ser Gly Ile Ile Thr Ser Asp Ala Pro
            275                 280                 285
Val His Asp Cys Asn Thr Lys Cys Gln Thr Pro His Gly Ala Ile Asn
    290                 295                 300
Ser Ser Leu Pro Phe Gln Asn Ile His Pro Val Thr Ile Gly Glu Cys
305                 310                 315                 320
Pro Lys Tyr Val Arg Ser Thr Lys Leu Arg Met Ala Thr Gly Leu Arg
                325                 330                 335
Asn Ile Pro Ser Ile Gln Ser Arg Gly Leu Phe Gly Ala Ile Ala Gly
            340                 345                 350
Phe Ile Glu Gly Gly Trp Thr Gly Met Ile Asp Gly Trp Tyr Gly Tyr
        355                 360                 365
His His Gln Asn Glu Gln Gly Ser Gly Tyr Ala Ala Asp Gln Lys Ser
    370                 375                 380
Thr Gln Asn Ala Ile Asp Gly Ile Thr Asn Lys Val Asn Ser Val Ile
385                 390                 395                 400
Glu Lys Met Asn Thr Gln Phe Thr Ala Val Gly Lys Glu Phe Asn Asn
                405                 410                 415
Leu Glu Arg Arg Ile Glu Asn Leu Asn Lys Lys Val Asp Asp Gly Phe
            420                 425                 430
Leu Asp Ile Trp Thr Tyr Asn Ala Glu Leu Leu Val Leu Leu Glu Asn
        435                 440                 445
Glu Arg Thr Leu Asp Phe His Asp Ser Asn Val Arg Asn Leu Tyr Glu
    450                 455                 460
Lys Val Lys Ser Gln Leu Lys Asn Asn Ala Lys Glu Ile Gly Asn Gly
465                 470                 475                 480
Cys Phe Glu Phe Tyr His Lys Cys Asp Asp Ala Cys Met Glu Ser Val
                485                 490                 495
Arg Asn Gly Thr Tyr Asp Tyr Pro Lys Tyr Ser Glu Glu Ser Lys Leu
            500                 505                 510
Asn Arg Glu Glu Ile Asp Gly Val Lys Leu Glu Ser Met Gly Val Tyr
        515                 520                 525
Gln Ile Leu Ala Ile Tyr Ser Thr Val Ala Ser Ser Leu Val Leu Leu
    530                 535                 540
Val Ser Leu Gly Ala Ile Ser Phe Trp Met Cys Ser Asn Gly Ser Leu
545                 550                 555                 560
Gln Cys Arg Ile Cys Ile
                565

<210> 6
<211> 552
<212> PRT
<213> Influenza A virus

<400> 6
Asp Gln Ile Cys Ile Gly Tyr His Ala Asn Asn Ser Thr Glu Gln Val
1               5                   10                  15
Asp Thr Ile Met Glu Lys Asn Val Thr Val Thr His Ala Gln Asp Ile
            20                  25                  30
Leu Glu Arg Thr His Asn Gly Lys Leu Cys Asp Leu Asn Gly Val Lys
        35                  40                  45
Pro Leu Ile Leu Arg Asp Cys Ser Val Ala Gly Trp Leu Leu Gly Asn
    50                  55                  60
Pro Met Cys Asp Glu Phe Ile Asn Val Pro Glu Trp Ser Tyr Ile Val
65                  70                  75                  80
Glu Lys Ala Ser Pro Ala Asn Asp Leu Cys Tyr Pro Gly Asn Phe Asn
                85                  90                  95
Asp Tyr Glu Glu Leu Lys His Leu Leu Ser Arg Ile Asn His Phe Glu
            100                 105                 110
Lys Ile Gln Ile Ile Pro Lys Ser Ser Trp Ser Asn His Asp Ala Ser
        115                 120                 125
Ser Gly Val Ser Ser Ala Cys Pro Tyr Leu Gly Arg Ser Ser Phe Phe
    130                 135                 140
Arg Asn Val Val Trp Leu Ile Lys Lys Asn Ser Ser Tyr Pro Thr Ile

145                 150                 155                 160
Lys Arg Ser Tyr Asn Asn Thr Asn Gln Glu Asp Leu Leu Val Leu Trp
                165                 170                 175
Gly Ile His His Pro Asn Asp Ala Ala Glu Gln Thr Lys Leu Tyr Gln
            180                 185                 190
Asn Pro Thr Thr Tyr Ile Ser Val Gly Thr Ser Thr Leu Asn Gln Arg
        195                 200                 205
Leu Val Pro Glu Ile Ala Thr Arg Pro Lys Val Asn Gly Gln Ser Gly
    210                 215                 220
Arg Met Glu Phe Phe Trp Thr Ile Leu Lys Pro Asn Asp Ala Ile Asn
225                 230                 235                 240
Phe Glu Ser Asn Gly Asn Phe Ile Ala Pro Glu Tyr Ala Tyr Lys Ile
                245                 250                 255
Val Lys Lys Gly Asp Ser Thr Ile Met Lys Ser Glu Leu Glu Tyr Gly
            260                 265                 270
Asn Cys Asn Thr Lys Cys Gln Thr Pro Met Gly Ala Ile Asn Ser Ser
        275                 280                 285
Met Pro Phe His Asn Ile His Pro Leu Thr Ile Gly Glu Cys Pro Lys
    290                 295                 300
Tyr Val Lys Ser Asn Arg Leu Val Leu Ala Thr Gly Leu Arg Asn Thr
305                 310                 315                 320
Pro Gln Arg Glu Arg Arg Arg Lys Lys Arg Gly Leu Phe Gly Ala Ile
                325                 330                 335
Ala Gly Phe Ile Glu Gly Gly Trp Gln Gly Met Val Asp Gly Trp Tyr
            340                 345                 350
Gly Tyr His His Ser Asn Glu Gln Gly Ser Gly Tyr Ala Ala Asp Gln
        355                 360                 365
Glu Ser Thr Gln Lys Ala Ile Asp Gly Val Thr Asn Lys Val Asn Ser
    370                 375                 380
Ile Ile Asn Lys Met Asn Thr Gln Phe Glu Ala Val Gly Arg Glu Phe
385                 390                 395                 400
Asn Asn Leu Glu Arg Arg Ile Glu Asn Leu Asn Lys Lys Met Glu Asp
                405                 410                 415
Gly Phe Leu Asp Val Trp Thr Tyr Asn Ala Glu Leu Leu Val Leu Met
            420                 425                 430
Glu Asn Glu Arg Thr Leu Asp Phe His Asp Ser Asn Val Lys Asn Leu
        435                 440                 445
Tyr Asp Lys Val Arg Leu Gln Leu Arg Asp Asn Ala Lys Glu Leu Gly
    450                 455                 460
Asn Gly Cys Phe Glu Phe Tyr His Lys Cys Asp Asn Glu Cys Met Glu
465                 470                 475                 480
Ser Val Lys Asn Gly Thr Tyr Asp Tyr Pro Gln Tyr Ser Glu Glu Ala
                485                 490                 495
Arg Leu Asn Arg Glu Glu Ile Ser Gly Val Lys Leu Glu Ser Met Gly
            500                 505                 510
Thr Tyr Gln Ile Leu Ser Ile Tyr Ser Thr Val Ala Ser Ser Leu Ala
        515                 520                 525
Leu Ala Ile Met Val Ala Gly Leu Ser Leu Trp Met Cys Ser Asn Gly
    530                 535                 540
Ser Leu Gln Cys Arg Ile Cys Ile
545                 550

<210> 7
<211> 564
<212> PRT
<213> Influenza A virus

<400> 7
Met Leu Ser Ile Val Ile Leu Phe Leu Leu Ile Ala Glu Asn Ser Ser
1               5                   10                  15
Gln Asn Tyr Thr Gly Asn Pro Val Ile Cys Met Gly His His Ala Val
            20                  25                  30
Ala Asn Gly Thr Met Val Lys Thr Leu Ala Asp Asp Gln Val Glu Val
        35                  40                  45

Val Thr Ala Gln Glu Leu Val Glu Ser Gln Asn Leu Pro Glu Leu Cys
50                  55                  60
Pro Ser Pro Leu Arg Leu Val Asp Gly Gln Thr Cys Asp Ile Ile Asn
65                  70                  75                  80
Gly Ala Leu Gly Ser Pro Gly Cys Asp His Leu Asn Gly Ala Glu Trp
                85                  90                  95
Asp Val Phe Ile Glu Arg Pro Asn Ala Val Asp Thr Cys Tyr Pro Phe
            100                 105                 110
Asp Val Pro Glu Tyr Gln Ser Leu Arg Ser Ile Leu Ala Asn Asn Gly
        115                 120                 125
Lys Phe Glu Phe Ile Ala Glu Glu Phe Gln Trp Asn Thr Val Lys Gln
    130                 135                 140
Asn Gly Lys Ser Gly Ala Cys Lys Arg Ala Asn Val Asp Asp Phe Phe
145                 150                 155                 160
Asn Arg Leu Asn Trp Leu Val Lys Ser Asp Gly Asn Ala Tyr Pro Phe
                165                 170                 175
Gln Asn Leu Thr Lys Ile Asn Asn Gly Asp Tyr Ala Arg Leu Tyr Ile
            180                 185                 190
Trp Gly Val His His Pro Ser Thr Ser Thr Glu Gln Ile Asn Leu Tyr
        195                 200                 205
Lys Asn Asn Pro Gly Arg Val Thr Val Ser Thr Lys Thr Ser Gln Thr
    210                 215                 220
Ser Val Val Pro Asp Ile Gly Ser Arg Pro Leu Val Arg Gly Gln Ser
225                 230                 235                 240
Gly Arg Val Ser Phe Tyr Trp Thr Ile Val Glu Pro Gly Asp Leu Ile
                245                 250                 255
Val Phe Asn Thr Ile Gly Asn Leu Ile Ala Pro Arg Gly His Tyr Lys
            260                 265                 270
Leu Asn Asn Gln Lys Lys Ser Thr Ile Leu Asn Thr Ala Ile Pro Ile
        275                 280                 285
Gly Ser Cys Val Ser Lys Cys His Thr Asp Lys Gly Ser Leu Ser Thr
    290                 295                 300
Thr Lys Pro Phe Gln Asn Ile Ser Arg Ile Ala Val Gly Asp Cys Pro
305                 310                 315                 320
Arg Tyr Val Lys Gln Gly Ser Leu Lys Leu Ala Thr Gly Met Arg Asn
                325                 330                 335
Ile Pro Glu Lys Ala Ser Arg Gly Leu Phe Gly Ala Ile Ala Gly Phe
            340                 345                 350
Ile Glu Asn Gly Trp Gln Gly Leu Ile Asp Gly Trp Tyr Gly Phe Arg
        355                 360                 365
His Gln Asn Ala Glu Gly Thr Gly Thr Ala Ala Asp Leu Lys Ser Thr
    370                 375                 380
Gln Ala Ala Ile Asn Gln Ile Asn Gly Lys Leu Asn Arg Leu Ile Glu
385                 390                 395                 400
Lys Thr Asn Asp Lys Tyr His Gln Ile Glu Lys Glu Phe Glu Gln Val
                405                 410                 415
Glu Gly Arg Ile Gln Asp Leu Glu Asn Tyr Val Glu Asp Thr Lys Ile
            420                 425                 430
Asp Leu Trp Ser Tyr Asn Ala Glu Leu Leu Val Ala Leu Glu Asn Gln
        435                 440                 445
His Thr Ile Asp Val Thr Asp Ser Glu Met Asn Lys Leu Phe Glu Arg
    450                 455                 460
Val Arg Arg Gln Leu Arg Glu Asn Ala Glu Asp Lys Gly Asn Gly Cys
465                 470                 475                 480
Phe Glu Ile Phe His Lys Cys Asp Asn Asn Cys Ile Glu Ser Ile Arg
                485                 490                 495
Asn Gly Thr Tyr Asp His Asp Ile Tyr Arg Asp Glu Ala Ile Asp Asn
            500                 505                 510
Arg Phe Gln Ile Gln Gly Val Lys Leu Thr Gln Gly Tyr Lys Asp Ile
        515                 520                 525
Ile Leu Trp Ile Ser Phe Ser Ile Ser Cys Phe Leu Leu Val Ala Leu
    530                 535                 540
Leu Leu Ala Phe Ile Leu Trp Ala Cys Gln Asn Gly Asn Ile Arg Cys
545                 550                 555                 560
Gln Ile Cys Ile

<210> 8
<211> 566
<212> PRT
<213> Influenza A virus

<400> 8
Met Lys Thr Ile Ile Ala Leu Ser Tyr Ile Phe Cys Leu Ala Leu Gly
1               5                   10                  15
Gln Asp Leu Pro Gly Asn Asp Asn Ser Thr Ala Thr Leu Cys Leu Gly
            20                  25                  30
His His Ala Val Pro Asn Gly Thr Leu Val Lys Thr Ile Thr Asp Asp
        35                  40                  45
Gln Ile Glu Val Thr Asn Ala Thr Glu Leu Val Gln Ser Ser Ser Thr
    50                  55                  60
Gly Lys Ile Cys Asn Asn Pro His Arg Ile Leu Asp Gly Ile Asp Cys
65                  70                  75                  80
Thr Leu Ile Asp Ala Leu Leu Gly Asp Pro His Cys Asp Val Phe Gln
                85                  90                  95
Asn Glu Thr Trp Asp Leu Phe Val Glu Arg Ser Lys Ala Phe Ser Asn
            100                 105                 110
Cys Tyr Pro Tyr Asp Val Pro Asp Tyr Ala Ser Leu Arg Ser Leu Val
        115                 120                 125
Ala Ser Ser Gly Thr Leu Glu Phe Ile Thr Glu Gly Phe Thr Trp Thr
    130                 135                 140
Gly Val Thr Gln Asn Gly Gly Ser Asn Ala Cys Lys Arg Gly Pro Gly
145                 150                 155                 160
Ser Gly Phe Phe Ser Arg Leu Asn Trp Leu Thr Lys Ser Gly Ser Thr
                165                 170                 175
Tyr Pro Val Leu Asn Val Thr Met Pro Asn Asn Asp Asn Phe Asp Lys
            180                 185                 190
Leu Tyr Ile Trp Gly Val His His Pro Ser Thr Asn Gln Glu Gln Thr
        195                 200                 205
Ser Leu Tyr Val Gln Ala Ser Gly Arg Val Thr Val Ser Thr Arg Arg
    210                 215                 220
Ser Gln Gln Thr Ile Ile Pro Asn Ile Gly Ser Arg Pro Trp Val Arg
225                 230                 235                 240
Gly Leu Ser Ser Arg Ile Ser Ile Tyr Trp Thr Ile Val Lys Pro Gly
                245                 250                 255
Asp Val Leu Val Ile Asn Ser Asn Gly Asn Leu Ile Ala Pro Arg Gly
            260                 265                 270
Tyr Phe Lys Met Arg Thr Gly Lys Ser Ser Ile Met Arg Ser Asp Ala
        275                 280                 285
Pro Ile Asp Thr Cys Ile Ser Glu Cys Ile Thr Pro Asn Gly Ser Ile
    290                 295                 300
Pro Asn Asp Lys Pro Phe Gln Asn Val Asn Lys Ile Thr Tyr Gly Ala
305                 310                 315                 320
Cys Pro Lys Tyr Val Lys Gln Asn Thr Leu Lys Leu Ala Thr Gly Met
                325                 330                 335
Arg Asn Val Pro Glu Lys Gln Thr Arg Gly Leu Phe Gly Ala Ile Ala
            340                 345                 350
Gly Phe Ile Glu Asn Gly Trp Glu Gly Met Ile Asp Gly Trp Tyr Gly
        355                 360                 365
Phe Arg His Gln Asn Ser Glu Gly Thr Gly Gln Ala Ala Asp Leu Lys
    370                 375                 380
Ser Thr Gln Ala Ala Ile Asp Gln Ile Asn Gly Lys Leu Asn Arg Val
385                 390                 395                 400
Ile Glu Lys Thr Asn Glu Lys Phe His Gln Ile Glu Lys Glu Phe Ser
                405                 410                 415
Glu Val Glu Gly Arg Ile Gln Asp Leu Glu Lys Tyr Val Glu Asp Thr
            420                 425                 430
Lys Ile Asp Leu Trp Ser Tyr Asn Ala Glu Leu Leu Val Ala Leu Glu
        435                 440                 445

Asn Gln His Thr Ile Asp Leu Thr Asp Ser Glu Met Asn Lys Leu Phe
    450                 455                 460
Glu Lys Thr Arg Arg Gln Leu Arg Glu Asn Ala Glu Asp Met Gly Asn
465                 470                 475                 480
Gly Cys Phe Lys Ile Tyr His Lys Cys Asp Asn Ala Cys Ile Glu Ser
                485                 490                 495
Ile Arg Asn Gly Thr Tyr Asp His Asp Val Tyr Arg Asp Glu Ala Leu
            500                 505                 510
Asn Asn Arg Phe Gln Ile Lys Gly Val Glu Leu Lys Ser Gly Tyr Lys
        515                 520                 525
Asp Trp Ile Leu Trp Ile Ser Phe Ala Ile Ser Cys Phe Leu Leu Cys
    530                 535                 540
Val Val Leu Leu Gly Phe Ile Met Trp Ala Cys Gln Arg Gly Asn Ile
545                 550                 555                 560
Arg Cys Asn Ile Cys Ile
                565

<210> 9
<211> 566
<212> PRT
<213> Influenza A virus

<400> 9
Met Ile Ala Ile Ile Ile Leu Ala Ile Val Ala Ser Thr Ser Lys Ser
 1               5                  10                  15
Asp Lys Ile Cys Ile Gly Tyr His Ala Asn Asn Ser Thr Thr Gln Val
            20                  25                  30
Asp Thr Ile Leu Glu Lys Asn Val Thr Val Thr His Ser Val Glu Leu
        35                  40                  45
Leu Glu Ser Gln Lys Glu Glu Arg Phe Cys Arg Val Leu Asn Lys Ala
    50                  55                  60
Pro Leu Asp Leu Lys Gly Cys Thr Ile Glu Gly Trp Ile Leu Gly Asn
65                  70                  75                  80
Pro Gln Cys Asp Ile Leu Leu Gly Asp Gln Arg Trp Ser Tyr Ile Val
                85                  90                  95
Glu Arg Pro Gly Ala Gln Asn Gly Ile Cys Tyr Pro Gly Ile Leu Asn
            100                 105                 110
Glu Leu Glu Glu Leu Lys Ala Leu Ile Gly Ser Gly Glu Arg Val Gln
        115                 120                 125
Arg Phe Glu Met Phe Pro Lys Ser Thr Trp Ala Gly Val Asp Thr Ser
    130                 135                 140
Arg Gly Val Thr Lys Ala Cys Pro Tyr Ile Ser Gly Ser Ser Phe Tyr
145                 150                 155                 160
Gly Asn Leu Leu Trp Ile Ile Lys Thr Glu Ser Ala Ala Tyr Pro Val
                165                 170                 175
Ile Lys Gly Thr Tyr Asn Asn Thr Gly Ser Gln Pro Ile Leu Tyr Phe
            180                 185                 190
Trp Gly Val His His Pro Pro Asp Thr Asn Glu Gln Asn Thr Leu Tyr
        195                 200                 205
Gly Ser Gly Asp Arg Tyr Val Arg Met Gly Thr Glu Ser Met Asn Phe
    210                 215                 220
Ala Lys Ser Pro Glu Ile Ala Ala Arg Pro Ala Val Asn Gly Gln Arg
225                 230                 235                 240
Gly Arg Ile Asp Tyr Tyr Trp Ser Val Leu Lys Pro Gly Glu Thr Leu
                245                 250                 255
Asn Val Glu Ser Asn Gly Asn Leu Ile Ala Pro Trp Tyr Ala Tyr Lys
            260                 265                 270
Phe Thr Ser Ser Asn Asn Lys Gly Ala Val Phe Lys Ser Asn Leu Pro
        275                 280                 285
Ile Glu Asn Cys Asp Ala Val Cys Gln Thr Val Ala Gly Ala Leu Arg
    290                 295                 300
Thr Asn Lys Thr Phe Gln Asn Val Ser Pro Leu Trp Ile Gly Glu Cys
305                 310                 315                 320
Pro Lys Tyr Val Lys Ser Asp Ser Leu Arg Leu Ala Thr Gly Leu Arg

325                 330                 335
Asn Val Pro Gln Ala Glu Thr Arg Gly Leu Phe Gly Ala Ile Ala Gly
            340                 345                 350
Phe Ile Glu Gly Gly Trp Thr Gly Met Ile Asp Gly Trp Tyr Gly Tyr
        355                 360                 365
His His Glu Asn Ser Gln Gly Ser Gly Tyr Ala Ala Asp Lys Glu Ser
    370                 375                 380
Thr Gln Lys Ala Ile Asp Gly Ile Thr Asn Lys Val Asn Ser Ile Ile
385                 390                 395                 400
Asp Lys Met Asn Thr Gln Phe Glu Ala Val Asp His Glu Phe Ser Asn
                405                 410                 415
Leu Glu Arg Arg Val Asp Asn Leu Asn Lys Arg Met Glu Asp Gly Phe
            420                 425                 430
Leu Asp Val Trp Thr Tyr Asn Ala Glu Leu Leu Val Leu Leu Glu Asn
        435                 440                 445
Glu Arg Thr Leu Asp Leu His Asp Ala Asn Val Lys Asn Leu Tyr Glu
    450                 455                 460
Lys Val Lys Ser Gln Leu Arg Asp Asn Ala Lys Asp Leu Gly Asn Gly
465                 470                 475                 480
Cys Phe Glu Phe Trp His Lys Cys Asp Asp Glu Cys Ile Asn Ser Val
                485                 490                 495
Lys Asn Gly Thr Tyr Asp Tyr Pro Lys Tyr Gln Asp Glu Ser Lys Leu
            500                 505                 510
Asn Arg Gln Glu Ile Asp Ser Val Lys Leu Glu Asn Leu Gly Val Tyr
        515                 520                 525
Gln Ile Leu Ala Ile Tyr Ser Thr Val Ser Ser Gly Leu Val Leu Val
    530                 535                 540
Gly Leu Ile Ile Ala Met Gly Leu Trp Met Cys Ser Asn Gly Ser Met
545                 550                 555                 560
Pro Cys Lys Ile Cys Ile
                565

<210> 10
<211> 565
<212> PRT
<213> Influenza A virus

<400> 10
Met Ala Val Lys Val Leu His Leu Leu Ile Ile Val Leu Gly Arg Tyr
1               5                   10                  15
Ser Ile Ala Asp Lys Ile Cys Ile Gly Tyr Leu Ser Asn Asn Ser Ser
            20                  25                  30
Asp Thr Val Asp Thr Leu Thr Glu Asn Gly Val Pro Val Thr Ser Ser
        35                  40                  45
Ile Asp Leu Val Glu Thr Asn His Thr Gly Thr Tyr Cys Ser Leu Asn
    50                  55                  60
Gly Ile Ser Pro Ile His Leu Gly Asp Cys Ser Phe Glu Gly Trp Ile
65                  70                  75                  80
Val Gly Asn Pro Ser Cys Ala Thr Asn Ile Asn Ile Arg Glu Trp Ser
                85                  90                  95
Tyr Leu Ile Glu Asp Pro Asn Ala Pro Asn Lys Leu Cys Phe Pro Gly
            100                 105                 110
Glu Leu Asp Asn Asn Gly Glu Leu Arg His Leu Phe Ser Gly Val Asn
        115                 120                 125
Ser Phe Ser Arg Thr Glu Leu Ile Ser Pro Ser Lys Trp Gly Asp Val
    130                 135                 140
Leu Asp Gly Val Thr Ala Ser Cys Leu Asp Lys Gly Ala Ser Ser Phe
145                 150                 155                 160
Tyr Arg Asn Leu Val Trp Leu Val Lys Gln Asn Asp Arg Tyr Pro Val
                165                 170                 175
Val Arg Gly Asp Tyr Asn Asn Thr Thr Gly Arg Asp Val Leu Val Leu
            180                 185                 190
Trp Gly Ile His His Pro Asp Thr Glu Thr Thr Ala Thr Lys Leu Tyr
        195                 200                 205

Val Asn Lys Asn Pro Tyr Thr Leu Val Ser Thr Lys Glu Trp Ser Lys
    210                 215                 220
Arg Tyr Glu Leu Glu Ile Gly Thr Arg Ile Gly Asp Gly Gln Arg Ser
225                 230                 235                 240
Trp Met Lys Ile Tyr Trp His Leu Met His Pro Gly Glu Arg Ile Met
                245                 250                 255
Phe Glu Ser Asn Gly Gly Leu Leu Ala Pro Arg Tyr Gly Tyr Ile Ile
            260                 265                 270
Glu Lys Tyr Gly Thr Gly Arg Ile Phe Gln Ser Gly Ile Arg Met Ala
        275                 280                 285
Lys Cys Asn Thr Lys Cys Gln Thr Ser Met Gly Gly Val Asn Thr Asn
    290                 295                 300
Lys Thr Phe Gln Asn Ile Glu Arg Asn Ala Leu Gly Asp Cys Pro Lys
305                 310                 315                 320
Tyr Ile Lys Ser Gly Gln Leu Lys Leu Ala Thr Gly Leu Arg Asn Val
                325                 330                 335
Pro Ser Ile Gly Glu Arg Gly Leu Phe Gly Ala Ile Ala Gly Phe Ile
            340                 345                 350
Glu Gly Gly Trp Pro Gly Leu Ile Asn Gly Trp Tyr Gly Phe Gln His
        355                 360                 365
Gln Asn Glu Gln Gly Thr Gly Ile Ala Ala Asp Lys Ala Ser Thr Gln
    370                 375                 380
Lys Ala Ile Asn Glu Ile Thr Thr Lys Ile Asn Asn Ile Ile Glu Lys
385                 390                 395                 400
Met Asn Gly Asn Tyr Asp Ser Ile Arg Gly Glu Phe Asn Gln Val Glu
                405                 410                 415
Lys Arg Ile Asn Met Leu Ala Asp Arg Val Asp Asp Ala Val Thr Asp
            420                 425                 430
Ile Trp Ser Tyr Asn Ala Lys Leu Leu Val Leu Ile Glu Asn Asp Arg
        435                 440                 445
Thr Leu Asp Leu His Asp Ala Asn Val Lys Asn Leu His Glu Gln Val
    450                 455                 460
Lys Arg Ala Leu Lys Asn Asn Ala Ile Asp Glu Gly Asp Gly Cys Phe
465                 470                 475                 480
Asn Leu Leu His Lys Cys Asn Asp Ser Cys Met Glu Thr Ile Arg Asn
                485                 490                 495
Gly Thr Tyr Asn His Glu Asp Tyr Arg Glu Glu Ser Gln Leu Lys Arg
            500                 505                 510
Gln Glu Ile Glu Gly Ile Lys Leu Lys Thr Glu Asp Asn Val Tyr Lys
        515                 520                 525
Val Leu Ser Ile Tyr Ser Cys Ile Ala Ser Ser Ile Val Met Val Gly
    530                 535                 540
Leu Ile Leu Ala Phe Ile Met Trp Ala Cys Ser Ser Gly Asn Cys Arg
545                 550                 555                 560
Phe Asn Val Cys Ile
                565

<210> 11
<211> 565
<212> PRT
<213> Influenza A virus

<400> 11
Met Glu Lys Phe Ile Ala Ile Ala Thr Leu Ala Ser Thr Asn Ala Tyr
1               5                   10                  15
Asp Arg Ile Cys Ile Gly Tyr Gln Ser Asn Asn Ser Thr Asp Thr Val
            20                  25                  30
Asn Thr Leu Ile Glu Gln Asn Val Pro Val Thr Gln Thr Met Glu Leu
        35                  40                  45
Val Glu Thr Glu Lys His Pro Ala Tyr Cys Asn Thr Asp Leu Gly Ala
    50                  55                  60
Pro Leu Glu Leu Arg Asp Cys Lys Ile Glu Ala Val Ile Tyr Gly Asn
65                  70                  75                  80
Pro Lys Cys Asp Ile His Leu Lys Asp Gln Gly Trp Ser Tyr Ile Val

85                  90                  95
Glu Arg Pro Ser Ala Pro Glu Gly Met Cys Tyr Pro Gly Ser Val Glu
            100                 105                 110
Asn Leu Glu Glu Leu Arg Phe Val Phe Ser Ser Ala Ala Ser Tyr Lys
        115                 120                 125
Arg Ile Arg Leu Phe Asp Tyr Ser Arg Trp Asn Val Thr Arg Ser Gly
    130                 135                 140
Thr Ser Lys Ala Cys Asn Ala Ser Thr Gly Gly Gln Ser Phe Tyr Arg
145                 150                 155                 160
Ser Ile Asn Trp Leu Thr Lys Lys Glu Pro Asp Thr Tyr Asp Phe Asn
                165                 170                 175
Glu Gly Ala Tyr Val Asn Asn Glu Asp Gly Asp Ile Ile Phe Leu Trp
            180                 185                 190
Gly Ile His His Pro Pro Asp Thr Lys Glu Gln Thr Thr Leu Tyr Lys
        195                 200                 205
Asn Ala Asn Thr Leu Ser Ser Val Thr Thr Asn Thr Ile Asn Arg Ser
    210                 215                 220
Phe Gln Pro Asn Ile Gly Pro Arg Pro Leu Val Arg Gly Gln Gln Gly
225                 230                 235                 240
Arg Met Asp Tyr Tyr Trp Gly Ile Leu Lys Arg Gly Glu Thr Leu Lys
                245                 250                 255
Ile Arg Thr Asn Gly Asn Leu Ile Ala Pro Glu Phe Gly Tyr Leu Leu
            260                 265                 270
Lys Gly Glu Ser Tyr Gly Arg Ile Ile Gln Asn Glu Asp Ile Pro Ile
        275                 280                 285
Gly Asn Cys Asn Thr Lys Cys Gln Thr Tyr Ala Gly Ala Ile Asn Ser
    290                 295                 300
Ser Lys Pro Phe Gln Asn Ala Ser Arg His Tyr Met Gly Glu Cys Pro
305                 310                 315                 320
Lys Tyr Val Lys Lys Ala Ser Leu Arg Leu Ala Val Gly Leu Arg Asn
                325                 330                 335
Thr Pro Ser Val Glu Pro Arg Gly Leu Phe Gly Ala Ile Ala Gly Phe
            340                 345                 350
Ile Glu Gly Gly Trp Ser Gly Met Ile Asp Gly Trp Tyr Gly Phe His
        355                 360                 365
His Ser Asn Ser Glu Gly Thr Gly Met Ala Ala Asp Gln Lys Ser Thr
    370                 375                 380
Gln Glu Ala Ile Asp Lys Ile Thr Asn Lys Val Asn Asn Ile Val Asp
385                 390                 395                 400
Lys Met Asn Arg Glu Phe Glu Val Val Asn His Glu Phe Ser Glu Val
                405                 410                 415
Glu Lys Arg Ile Asn Met Ile Asn Asp Lys Ile Asp Asp Gln Ile Glu
            420                 425                 430
Asp Leu Trp Ala Tyr Asn Ala Glu Leu Leu Val Leu Leu Glu Asn Gln
        435                 440                 445
Lys Thr Leu Asp Glu His Asp Ser Asn Val Lys Asn Leu Phe Asp Glu
    450                 455                 460
Val Lys Arg Arg Leu Ser Ala Asn Ala Ile Asp Ala Gly Asn Gly Cys
465                 470                 475                 480
Phe Asp Ile Leu His Lys Cys Asp Asn Glu Cys Met Glu Thr Ile Lys
                485                 490                 495
Asn Gly Thr Tyr Asp His Lys Glu Tyr Glu Glu Glu Ala Lys Leu Glu
            500                 505                 510
Arg Ser Lys Ile Asn Gly Val Lys Leu Glu Glu Asn Thr Thr Tyr Lys
        515                 520                 525
Ile Leu Ser Ile Tyr Ser Thr Val Ala Ala Ser Leu Cys Leu Ala Ile
    530                 535                 540
Leu Ile Ala Gly Gly Leu Ile Leu Gly Met Gln Asn Gly Ser Cys Arg
545                 550                 555                 560
Cys Met Phe Cys Ile
                565

<210> 12
<211> 565

<212> PRT
<213> Influenza A virus

<400> 12
Met Glu Lys Thr Leu Leu Phe Ala Ala Ile Phe Leu Cys Val Lys Ala
1               5                   10                  15
Asp Glu Ile Cys Ile Gly Tyr Leu Ser Asn Asn Ser Thr Asp Lys Val
            20                  25                  30
Asp Thr Ile Ile Glu Asn Asn Val Thr Val Thr Ser Ser Val Glu Leu
        35                  40                  45
Val Glu Thr Glu His Thr Gly Ser Phe Cys Ser Ile Asn Gly Lys Gln
    50                  55                  60
Pro Ile Ser Leu Gly Asp Cys Ser Phe Ala Gly Trp Ile Leu Gly Asn
65                  70                  75                  80
Pro Met Cys Asp Glu Leu Ile Gly Lys Thr Ser Trp Ser Tyr Ile Val
                85                  90                  95
Glu Lys Pro Asn Pro Thr Asn Gly Ile Cys Tyr Pro Gly Thr Leu Glu
            100                 105                 110
Ser Glu Glu Glu Leu Arg Leu Lys Phe Ser Gly Val Leu Glu Phe Asn
        115                 120                 125
Lys Phe Glu Val Phe Thr Ser Asn Gly Trp Gly Ala Val Asn Ser Gly
    130                 135                 140
Val Gly Val Thr Ala Ala Cys Lys Phe Gly Gly Ser Asn Ser Phe Phe
145                 150                 155                 160
Arg Asn Met Val Trp Leu Ile His Gln Ser Gly Thr Tyr Pro Val Ile
                165                 170                 175
Lys Arg Thr Phe Asn Asn Thr Lys Gly Arg Asp Val Leu Ile Val Trp
            180                 185                 190
Gly Ile His His Pro Ala Thr Leu Thr Glu His Gln Asp Leu Tyr Lys
        195                 200                 205
Lys Asp Ser Ser Tyr Val Ala Val Gly Ser Glu Thr Tyr Asn Arg Arg
    210                 215                 220
Phe Thr Pro Glu Ile Asn Thr Arg Pro Arg Val Asn Gly Gln Ala Gly
225                 230                 235                 240
Arg Met Thr Phe Tyr Trp Lys Ile Val Lys Pro Gly Glu Ser Ile Thr
                245                 250                 255
Phe Glu Ser Asn Gly Ala Phe Leu Ala Pro Arg Tyr Ala Phe Glu Ile
            260                 265                 270
Val Ser Val Gly Asn Gly Lys Leu Phe Arg Ser Glu Leu Asn Ile Glu
        275                 280                 285
Ser Cys Ser Thr Lys Cys Gln Thr Glu Ile Gly Gly Ile Asn Thr Asn
    290                 295                 300
Lys Ser Phe His Asn Val His Arg Asn Thr Ile Gly Asp Cys Pro Lys
305                 310                 315                 320
Tyr Val Asn Val Lys Ser Leu Lys Leu Ala Thr Gly Pro Arg Asn Val
                325                 330                 335
Pro Ala Ile Ala Ser Arg Gly Leu Phe Gly Ala Ile Ala Gly Phe Ile
            340                 345                 350
Glu Gly Gly Trp Pro Gly Leu Ile Asn Gly Trp Tyr Gly Phe Gln His
        355                 360                 365
Arg Asp Glu Glu Gly Thr Gly Ile Ala Ala Asp Lys Glu Ser Thr Gln
    370                 375                 380
Lys Ala Ile Asp Gln Ile Thr Ser Lys Val Asn Asn Ile Val Asp Arg
385                 390                 395                 400
Met Asn Thr Asn Phe Glu Ser Val Gln His Glu Phe Ser Glu Ile Glu
                405                 410                 415
Glu Arg Ile Asn Gln Leu Ser Lys His Val Asp Asp Ser Val Val Asp
            420                 425                 430
Ile Trp Ser Tyr Asn Ala Gln Leu Leu Val Leu Leu Glu Asn Glu Lys
        435                 440                 445
Thr Leu Asp Leu His Asp Ser Asn Val Arg Asn Leu His Glu Lys Val
    450                 455                 460
Arg Arg Met Leu Lys Asp Asn Ala Lys Asp Glu Gly Asn Gly Cys Phe
465                 470                 475                 480
Thr Phe Tyr His Lys Cys Asp Asn Lys Cys Ile Glu Arg Val Arg Asn

                485                 490                 495
Gly Thr Tyr Asp His Lys Glu Phe Glu Glu Glu Ser Lys Ile Asn Arg
            500                 505                 510
Gln Glu Ile Glu Gly Val Lys Leu Asp Ser Ser Gly Asn Val Tyr Lys
        515                 520                 525
Ile Leu Ser Ile Tyr Ser Cys Ile Ala Ser Ser Leu Val Leu Ala Ala
    530                 535                 540
Leu Ile Met Gly Phe Met Phe Trp Ala Cys Ser Asn Gly Ser Cys Arg
545                 550                 555                 560
Cys Thr Ile Cys Ile
                565

<210> 13
<211> 564
<212> PRT
<213> Influenza A virus

<400> 13
Met Glu Lys Phe Ile Ile Leu Ser Thr Val Leu Ala Ala Ser Phe Ala
 1               5                  10                  15
Tyr Asp Lys Ile Cys Ile Gly Tyr Gln Thr Asn Asn Ser Thr Glu Thr
            20                  25                  30
Val Asn Thr Leu Ser Glu Gln Asn Val Pro Val Thr Gln Val Glu Glu
        35                  40                  45
Leu Val His Arg Gly Ile Asp Pro Ile Leu Cys Gly Thr Glu Leu Gly
    50                  55                  60
Ser Pro Leu Val Leu Asp Asp Cys Ser Leu Glu Gly Leu Ile Leu Gly
65                  70                  75                  80
Asn Pro Lys Cys Asp Leu Tyr Leu Asn Gly Arg Glu Trp Ser Tyr Ile
                85                  90                  95
Val Glu Arg Pro Lys Glu Met Glu Gly Val Cys Tyr Pro Gly Ser Ile
            100                 105                 110
Glu Asn Gln Glu Glu Leu Arg Ser Leu Phe Ser Ser Ile Lys Lys Tyr
        115                 120                 125
Glu Arg Val Lys Met Phe Asp Phe Thr Lys Trp Asn Val Thr Tyr Thr
    130                 135                 140
Gly Thr Ser Lys Ala Cys Asn Asn Thr Ser Asn Gln Gly Ser Phe Tyr
145                 150                 155                 160
Arg Ser Met Arg Trp Leu Thr Leu Lys Ser Gly Gln Phe Pro Val Gln
                165                 170                 175
Thr Asp Glu Tyr Lys Asn Thr Arg Asp Ser Asp Ile Val Phe Thr Trp
            180                 185                 190
Ala Ile His His Pro Pro Thr Ser Asp Glu Gln Val Lys Leu Tyr Lys
        195                 200                 205
Asn Pro Asp Thr Leu Ser Ser Val Thr Thr Val Glu Ile Asn Arg Ser
    210                 215                 220
Phe Lys Pro Asn Ile Gly Pro Arg Pro Leu Val Arg Gly Gln Gln Gly
225                 230                 235                 240
Arg Met Asp Tyr Tyr Trp Ala Val Leu Lys Pro Gly Gln Thr Val Lys
                245                 250                 255
Ile Gln Thr Asn Gly Asn Leu Ile Ala Pro Glu Tyr Gly His Leu Ile
            260                 265                 270
Thr Gly Lys Ser His Gly Arg Ile Leu Lys Asn Asn Leu Pro Met Gly
        275                 280                 285
Gln Cys Val Thr Glu Cys Gln Leu Asn Glu Gly Val Met Asn Thr Ser
    290                 295                 300
Lys Pro Phe Gln Asn Thr Ser Lys His Tyr Ile Gly Lys Cys Pro Lys
305                 310                 315                 320
Tyr Ile Pro Ser Gly Ser Leu Lys Leu Ala Ile Gly Leu Arg Asn Val
                325                 330                 335
Pro Gln Val Gln Asp Arg Gly Leu Phe Gly Ala Ile Ala Gly Phe Ile
            340                 345                 350
Glu Gly Gly Trp Pro Gly Leu Val Ala Gly Trp Tyr Gly Phe Gln His
        355                 360                 365

Gln Asn Ala Glu Gly Thr Gly Ile Ala Ala Asp Arg Asp Ser Thr Gln
    370                 375                 380
Arg Ala Ile Asp Asn Met Gln Asn Lys Leu Asn Asn Val Ile Asp Lys
385                 390                 395                 400
Met Asn Lys Gln Phe Glu Val Val Asn His Glu Phe Ser Glu Val Glu
                405                 410                 415
Ser Arg Ile Asn Met Ile Asn Ser Lys Ile Asp Asp Gln Ile Thr Asp
            420                 425                 430
Ile Trp Ala Tyr Asn Ala Glu Leu Leu Val Leu Leu Glu Asn Gln Lys
        435                 440                 445
Thr Leu Asp Glu His Asp Ala Asn Val Arg Asn Leu His Asp Arg Val
    450                 455                 460
Arg Arg Val Leu Arg Glu Asn Ala Ile Asp Thr Gly Asp Gly Cys Phe
465                 470                 475                 480
Glu Ile Leu His Lys Cys Asp Asn Asn Cys Met Asp Thr Ile Arg Asn
                485                 490                 495
Gly Thr Tyr Asn His Lys Glu Tyr Glu Glu Glu Ser Lys Ile Glu Arg
            500                 505                 510
Gln Lys Val Asn Gly Val Lys Leu Glu Glu Asn Ser Thr Tyr Lys Ile
        515                 520                 525
Leu Ser Ile Tyr Ser Ser Val Ala Ser Ser Leu Val Leu Leu Leu Met
    530                 535                 540
Ile Ile Gly Gly Phe Ile Phe Gly Cys Gln Asn Gly Asn Val Arg Cys
545                 550                 555                 560
Thr Phe Cys Ile

<210> 14
<211> 566
<212> PRT
<213> Influenza A virus

<400> 14
Met Ala Leu Asn Val Ile Ala Thr Leu Thr Leu Ile Ser Val Cys Val
1               5                   10                  15
His Ala Asp Arg Ile Cys Val Gly Tyr Leu Ser Thr Asn Ser Ser Glu
            20                  25                  30
Arg Val Asp Thr Leu Leu Glu Asn Gly Val Pro Val Thr Ser Ser Ile
        35                  40                  45
Asp Leu Ile Glu Thr Asn His Thr Gly Thr Tyr Cys Ser Leu Asn Gly
    50                  55                  60
Val Ser Pro Val His Leu Gly Asp Cys Ser Phe Glu Gly Trp Ile Val
65                  70                  75                  80
Gly Asn Pro Ala Cys Thr Ser Asn Phe Gly Ile Arg Glu Trp Ser Tyr
                85                  90                  95
Leu Ile Glu Asp Pro Ala Ala Pro His Gly Leu Cys Tyr Pro Gly Glu
            100                 105                 110
Leu Asn Asn Asn Gly Glu Leu Arg His Leu Phe Ser Gly Ile Arg Ser
        115                 120                 125
Phe Ser Arg Thr Glu Leu Ile Pro Pro Thr Ser Trp Gly Glu Val Leu
    130                 135                 140
Asp Gly Thr Thr Ser Ala Cys Arg Asp Asn Thr Gly Thr Asn Ser Phe
145                 150                 155                 160
Tyr Arg Asn Leu Val Trp Phe Ile Lys Lys Asn Thr Arg Tyr Pro Val
                165                 170                 175
Ile Ser Lys Thr Tyr Asn Asn Thr Thr Gly Arg Asp Val Leu Val Leu
            180                 185                 190
Trp Gly Ile His His Pro Val Ser Val Asp Glu Thr Lys Thr Leu Tyr
        195                 200                 205
Val Asn Ser Asp Pro Tyr Thr Leu Val Ser Thr Lys Ser Trp Ser Glu
    210                 215                 220
Lys Tyr Lys Leu Glu Thr Gly Val Arg Pro Gly Tyr Asn Gly Gln Arg
225                 230                 235                 240
Ser Trp Met Lys Ile Tyr Trp Ser Leu Ile His Pro Gly Glu Met Ile

245                 250                 255
Thr Phe Glu Ser Asn Gly Gly Phe Leu Ala Pro Arg Tyr Gly Tyr Ile
            260                 265                 270
Ile Glu Glu Tyr Gly Lys Gly Arg Ile Phe Gln Ser Arg Ile Arg Met
        275                 280                 285
Ser Arg Cys Asn Thr Lys Cys Gln Thr Ser Val Gly Gly Ile Asn Thr
    290                 295                 300
Asn Arg Thr Phe Gln Asn Ile Asp Lys Asn Ala Leu Gly Asp Cys Pro
305                 310                 315                 320
Lys Tyr Ile Lys Ser Gly Gln Leu Lys Leu Ala Thr Gly Leu Arg Asn
                325                 330                 335
Val Pro Ala Ile Ser Asn Arg Gly Leu Phe Gly Ala Ile Ala Gly Phe
            340                 345                 350
Ile Glu Gly Gly Trp Pro Gly Leu Ile Asn Gly Trp Tyr Gly Phe Gln
        355                 360                 365
His Gln Asn Glu Gln Gly Thr Gly Ile Ala Ala Asp Lys Glu Ser Thr
    370                 375                 380
Gln Lys Ala Ile Asp Gln Ile Thr Thr Lys Ile Asn Asn Ile Ile Asp
385                 390                 395                 400
Lys Met Asn Gly Asn Tyr Asp Ser Ile Arg Gly Glu Phe Asn Gln Val
                405                 410                 415
Glu Lys Arg Ile Asn Met Leu Ala Asp Arg Ile Asp Asp Ala Val Thr
            420                 425                 430
Asp Ile Trp Ser Tyr Asn Ala Lys Leu Leu Val Leu Leu Glu Asn Asp
        435                 440                 445
Lys Thr Leu Asp Met His Asp Ala Asn Val Lys Asn Leu His Glu Gln
    450                 455                 460
Val Arg Arg Glu Leu Lys Asp Asn Ala Ile Asp Glu Gly Asn Gly Cys
465                 470                 475                 480
Phe Glu Leu Leu His Lys Cys Asn Asp Ser Cys Met Glu Thr Ile Arg
                485                 490                 495
Asn Gly Thr Tyr Asp His Thr Glu Tyr Ala Glu Glu Ser Lys Leu Lys
            500                 505                 510
Arg Gln Glu Ile Asp Gly Ile Lys Leu Lys Ser Glu Asp Asn Val Tyr
        515                 520                 525
Lys Ala Leu Ser Ile Tyr Ser Cys Ile Ala Ser Ser Val Val Leu Val
    530                 535                 540
Gly Leu Ile Leu Ser Phe Ile Met Trp Ala Cys Ser Ser Gly Asn Cys
545                 550                 555                 560
Arg Phe Asn Val Cys Ile
                565

<210> 15
<211> 560
<212> PRT
<213> Influenza A virus

<400> 15
Met Glu Thr Ile Ser Leu Ile Thr Ile Leu Leu Val Val Thr Ala Ser
1               5                   10                  15
Asn Ala Asp Lys Ile Cys Ile Gly His Gln Ser Thr Asn Ser Thr Glu
            20                  25                  30
Thr Val Asp Thr Leu Thr Glu Thr Asn Val Pro Val Thr His Ala Lys
        35                  40                  45
Glu Leu Leu His Thr Glu His Asn Gly Met Leu Cys Ala Thr Ser Leu
    50                  55                  60
Gly His Pro Leu Ile Leu Asp Thr Cys Thr Ile Glu Gly Leu Val Tyr
65                  70                  75                  80
Gly Asn Pro Ser Cys Asp Leu Leu Leu Gly Gly Arg Glu Trp Ser Tyr
                85                  90                  95
Ile Val Glu Arg Ser Ser Ala Val Asn Gly Thr Cys Tyr Pro Gly Asn
            100                 105                 110
Val Glu Asn Leu Glu Glu Leu Arg Thr Leu Phe Ser Ser Ala Ser Ser
        115                 120                 125

Tyr Gln Arg Ile Gln Ile Phe Pro Asp Thr Thr Trp Asn Val Thr Tyr
    130                 135                 140
Thr Gly Thr Ser Arg Ala Cys Ser Gly Ser Phe Tyr Arg Ser Met Arg
145                 150                 155                 160
Trp Leu Thr Gln Lys Ser Gly Phe Tyr Pro Val Gln Asp Ala Gln Tyr
                165                 170                 175
Thr Asn Asn Arg Gly Lys Ser Ile Leu Phe Val Trp Gly Ile His His
            180                 185                 190
Pro Pro Thr Tyr Thr Glu Gln Thr Asn Leu Tyr Ile Arg Asn Asp Thr
        195                 200                 205
Thr Thr Ser Val Thr Thr Glu Asp Leu Asn Arg Thr Phe Lys Pro Val
    210                 215                 220
Ile Gly Pro Arg Pro Leu Val Asn Gly Leu Gln Gly Arg Ile Asp Tyr
225                 230                 235                 240
Tyr Trp Ser Val Leu Lys Pro Gly Gln Thr Leu Arg Val Arg Ser Asn
                245                 250                 255
Gly Asn Leu Ile Ala Pro Trp Tyr Gly His Val Leu Ser Gly Gly Ser
            260                 265                 270
His Gly Arg Ile Leu Lys Thr Asp Leu Lys Gly Gly Asn Cys Val Val
        275                 280                 285
Gln Cys Gln Thr Glu Lys Gly Gly Leu Asn Ser Thr Leu Pro Phe His
    290                 295                 300
Asn Ile Ser Lys Tyr Ala Phe Gly Thr Cys Pro Lys Tyr Val Arg Val
305                 310                 315                 320
Asn Ser Leu Lys Leu Ala Val Gly Leu Arg Asn Val Pro Ala Arg Ser
                325                 330                 335
Ser Arg Gly Leu Phe Gly Ala Ile Ala Gly Phe Ile Glu Gly Gly Trp
            340                 345                 350
Pro Gly Leu Val Ala Gly Trp Tyr Gly Phe Gln His Ser Asn Asp Gln
        355                 360                 365
Gly Val Gly Met Ala Ala Asp Arg Asp Ser Thr Gln Lys Ala Ile Asp
    370                 375                 380
Lys Ile Thr Ser Lys Val Asn Asn Ile Val Asp Lys Met Asn Lys Gln
385                 390                 395                 400
Tyr Glu Ile Ile Asp His Glu Phe Ser Glu Val Glu Thr Arg Leu Asn
                405                 410                 415
Met Ile Asn Asn Lys Ile Asp Asp Gln Ile Gln Asp Val Trp Ala Tyr
            420                 425                 430
Asn Ala Glu Leu Leu Val Leu Leu Glu Asn Gln Lys Thr Leu Asp Glu
        435                 440                 445
His Asp Ala Asn Val Asn Asn Leu Tyr Asn Lys Val Lys Arg Ala Leu
    450                 455                 460
Gly Ser Asn Ala Met Glu Asp Gly Lys Gly Cys Phe Glu Leu Tyr His
465                 470                 475                 480
Lys Cys Asp Asp Gln Cys Met Glu Thr Ile Arg Asn Gly Thr Tyr Asn
                485                 490                 495
Arg Arg Lys Tyr Arg Glu Glu Ser Arg Leu Glu Arg Gln Lys Ile Glu
            500                 505                 510
Gly Val Lys Leu Glu Ser Glu Gly Thr Tyr Lys Ile Leu Thr Ile Tyr
        515                 520                 525
Ser Thr Val Ala Ser Ser Leu Val Leu Ala Met Gly Phe Ala Ala Phe
    530                 535                 540
Leu Phe Trp Ala Met Ser Asn Gly Ser Cys Arg Cys Asn Ile Cys Ile
545                 550                 555                 560


<210> 16
<211> 533
<212> PRT
<213> Influenza A virus

<400> 16
Met Glu Lys Asn Val Thr Val Thr His Ala Gln Asp Ile Leu Glu Lys
1               5                   10                  15
Thr His Asn Gly Lys Leu Cys Asp Leu Asp Gly Val Lys Pro Leu Ile

                    20                  25                  30
Leu Arg Asp Cys Ser Val Ala Gly Trp Leu Leu Gly Asn Pro Met Cys
        35                  40                  45
Asp Glu Phe Leu Asn Val Pro Glu Trp Ser Tyr Ile Val Glu Lys Ile
    50                  55                  60
Asn Pro Ala Asn Asp Leu Cys Tyr Pro Gly Asn Phe Asn Asp Tyr Glu
65                  70                  75                  80
Glu Leu Lys His Leu Leu Ser Arg Ile Asn His Phe Glu Lys Ile Gln
                85                  90                  95
Ile Ile Pro Lys Ser Ser Trp Ser Asp His Glu Ala Ser Ser Gly Val
            100                 105                 110
Ser Ser Ala Cys Pro Tyr Gln Gly Arg Ser Ser Phe Phe Arg Asn Val
        115                 120                 125
Val Trp Leu Ile Lys Lys Asn Asn Ala Tyr Pro Thr Ile Lys Arg Ser
    130                 135                 140
Tyr Asn Asn Thr Asn Gln Glu Asp Leu Leu Val Leu Trp Gly Ile His
145                 150                 155                 160
His Pro Asn Asp Ala Ala Glu Gln Thr Arg Leu Tyr Gln Asn Pro Thr
                165                 170                 175
Thr Tyr Ile Ser Val Gly Thr Ser Thr Leu Asn Gln Arg Leu Val Pro
            180                 185                 190
Lys Ile Ala Thr Arg Ser Lys Val Asn Gly Gln Asn Gly Arg Met Glu
        195                 200                 205
Phe Phe Trp Thr Ile Leu Lys Pro Asn Asp Ala Ile Asn Phe Glu Ser
    210                 215                 220
Asn Gly Asn Phe Ile Ala Pro Glu Asn Ala Tyr Lys Ile Val Lys Lys
225                 230                 235                 240
Gly Asp Ser Thr Ile Met Lys Ser Glu Leu Glu Tyr Gly Asn Cys Asn
                245                 250                 255
Thr Lys Cys Gln Thr Pro Ile Gly Ala Ile Asn Ser Ser Met Pro Phe
            260                 265                 270
His Asn Ile His Pro Leu Thr Ile Gly Glu Cys Pro Lys Tyr Val Lys
        275                 280                 285
Ser Asn Arg Leu Val Leu Ala Thr Gly Leu Arg Asn Ser Pro Gln Gly
    290                 295                 300
Glu Arg Arg Arg Lys Lys Arg Gly Leu Phe Gly Ala Ile Ala Gly Phe
305                 310                 315                 320
Ile Glu Gly Gly Trp Gln Gly Met Val Asp Gly Trp Tyr Gly Tyr His
                325                 330                 335
His Ser Asn Glu Gln Gly Ser Gly Tyr Ala Ala Asp Lys Glu Ser Thr
            340                 345                 350
Gln Lys Ala Ile Asp Gly Val Thr Asn Lys Val Asn Ser Ile Ile Asp
        355                 360                 365
Lys Met Asn Thr Gln Phe Glu Ala Val Gly Arg Glu Phe Asn Asn Leu
    370                 375                 380
Glu Arg Arg Ile Glu Asn Leu Asn Lys Lys Met Glu Asp Gly Phe Leu
385                 390                 395                 400
Asp Val Trp Thr Tyr Asn Ala Glu Leu Leu Val Leu Met Glu Asn Glu
                405                 410                 415
Arg Thr Leu Asp Phe His Asp Ser Asn Val Lys Asn Leu Tyr Asp Lys
            420                 425                 430
Val Arg Leu Gln Leu Arg Asp Asn Ala Lys Glu Leu Gly Asn Gly Cys
        435                 440                 445
Phe Glu Phe Tyr His Arg Cys Asp Asn Glu Cys Met Glu Ser Val Arg
    450                 455                 460
Asn Gly Thr Tyr Asp Tyr Pro Gln Tyr Ser Glu Glu Ala Arg Leu Lys
465                 470                 475                 480
Arg Glu Glu Ile Ser Gly Val Lys Leu Glu Ser Ile Gly Thr Tyr Gln
                485                 490                 495
Ile Leu Ser Ile Tyr Ser Thr Val Ala Ser Ser Leu Ala Leu Ala Ile
            500                 505                 510
Met Val Ala Gly Leu Ser Leu Trp Met Cys Ser Asn Gly Ser Leu Gln
        515                 520                 525
Cys Arg Ile Cys Ile
    530

<210> 17
<211> 533
<212> PRT
<213> Influenza A virus

<400> 17
Met Glu Lys Asn Val Thr Val Thr His Ala Gln Asp Ile Leu Glu Lys
1               5                   10                  15
Thr His Asn Gly Lys Leu Cys Asp Leu Asp Gly Val Lys Pro Leu Ile
            20                  25                  30
Leu Arg Asp Cys Ser Val Ala Gly Trp Leu Leu Gly Asn Pro Met Cys
        35                  40                  45
Asp Glu Phe Leu Asn Val Pro Glu Trp Ser Tyr Ile Val Glu Lys Ile
    50                  55                  60
Asn Pro Ala Asn Asp Leu Cys Tyr Pro Gly Asn Phe Asn Asp Tyr Glu
65                  70                  75                  80
Glu Leu Lys His Leu Leu Ser Arg Ile Asn His Phe Glu Lys Ile Gln
                85                  90                  95
Ile Ile Pro Lys Ser Ser Trp Ser Asp His Glu Ala Ser Ser Gly Val
            100                 105                 110
Ser Ser Ala Cys Pro Tyr Gln Gly Arg Ser Ser Phe Phe Arg Asn Val
        115                 120                 125
Val Trp Leu Ile Lys Lys Asp Asn Ala Tyr Pro Thr Ile Lys Arg Ser
    130                 135                 140
Tyr Asn Asn Thr Asn Gln Glu Asp Leu Leu Val Leu Trp Gly Ile His
145                 150                 155                 160
His Pro Asn Asp Ala Ala Glu Gln Thr Arg Leu Tyr Gln Asn Pro Thr
                165                 170                 175
Thr Tyr Ile Ser Val Gly Thr Ser Thr Leu Asn Gln Arg Leu Val Pro
            180                 185                 190
Lys Ile Ala Thr Arg Ser Lys Val Asn Gly Gln Asn Gly Arg Met Glu
        195                 200                 205
Phe Phe Trp Thr Ile Leu Lys Pro Asn Asp Ala Ile Asn Phe Glu Ser
    210                 215                 220
Asn Gly Asn Phe Ile Ala Pro Glu Asn Ala Tyr Lys Ile Val Lys Lys
225                 230                 235                 240
Gly Asp Ser Thr Ile Met Lys Ser Glu Leu Glu Tyr Gly Asn Cys Asn
                245                 250                 255
Thr Lys Cys Gln Thr Pro Ile Gly Ala Ile Asn Ser Ser Met Pro Phe
            260                 265                 270
His Asn Ile His Pro Leu Thr Ile Gly Glu Cys Pro Lys Tyr Val Lys
        275                 280                 285
Ser Asn Arg Leu Val Leu Ala Thr Gly Leu Arg Asn Ser Pro Gln Gly
    290                 295                 300
Glu Arg Arg Arg Lys Lys Arg Gly Leu Phe Gly Ala Ile Ala Gly Phe
305                 310                 315                 320
Ile Glu Gly Gly Trp Gln Gly Met Val Asp Gly Trp Tyr Gly Tyr His
                325                 330                 335
His Ser Asn Glu Gln Gly Ser Gly Tyr Ala Ala Asp Lys Glu Ser Thr
            340                 345                 350
Gln Lys Ala Ile Asp Gly Val Thr Asn Lys Val Asn Ser Ile Ile Asp
        355                 360                 365
Lys Met Asn Thr Gln Phe Glu Ala Val Gly Arg Glu Phe Asn Asn Leu
    370                 375                 380
Glu Arg Arg Ile Glu Asn Leu Asn Lys Lys Met Glu Asp Gly Phe Leu
385                 390                 395                 400
Asp Val Trp Thr Tyr Asn Ala Glu Leu Leu Val Leu Met Glu Asn Glu
                405                 410                 415
Arg Thr Leu Asp Phe His Asp Ser Asn Val Lys Asn Leu Tyr Asp Lys
            420                 425                 430
Val Arg Leu Gln Leu Arg Asp Asn Ala Lys Glu Leu Gly Asn Gly Cys
        435                 440                 445
Phe Glu Phe Tyr His Arg Cys Asp Asn Glu Cys Met Glu Ser Val Arg

450                 455                 460
Asn Gly Thr Tyr Asp Tyr Pro Gln Tyr Ser Glu Glu Ala Arg Leu Lys
465                 470                 475                 480
Arg Glu Glu Ile Ser Gly Val Lys Leu Glu Ser Ile Gly Thr Tyr Gln
                485                 490                 495
Ile Leu Ser Ile Tyr Ser Thr Val Ala Ser Ser Leu Ala Leu Ala Ile
            500                 505                 510
Met Val Ala Gly Leu Ser Leu Trp Met Cys Ser Asn Gly Ser Leu Gln
        515                 520                 525
Cys Arg Ile Cys Ile
    530

<210> 18
<211> 533
<212> PRT
<213> Influenza A virus

<400> 18
Met Glu Lys Asn Val Thr Val Thr His Ala Gln Asp Ile Leu Glu Lys
1               5                   10                  15
Thr His Asn Gly Lys Leu Cys Asp Leu Asp Gly Val Lys Pro Leu Ile
            20                  25                  30
Leu Arg Asp Cys Ser Val Ala Gly Trp Leu Leu Gly Asn Pro Met Cys
        35                  40                  45
Asp Glu Phe Leu Asn Val Pro Glu Trp Ser Tyr Ile Val Glu Lys Ile
    50                  55                  60
Asn Pro Ala Asn Asp Leu Cys Tyr Pro Gly Asn Phe Asn Asp Tyr Glu
65                  70                  75                  80
Glu Leu Lys His Leu Leu Ser Arg Ile Asn His Phe Glu Lys Ile Gln
                85                  90                  95
Ile Ile Pro Lys Ser Ser Trp Ser Asp His Glu Ala Ser Ser Gly Val
            100                 105                 110
Ser Ser Ala Cys Pro Tyr Gln Gly Arg Ser Ser Phe Phe Arg Asn Val
        115                 120                 125
Val Trp Leu Ile Lys Lys Asp Asn Ala Tyr Pro Thr Ile Lys Arg Ser
    130                 135                 140
Tyr Asn Asn Thr Asn Gln Glu Asp Leu Leu Val Leu Trp Gly Ile His
145                 150                 155                 160
His Pro Asn Asp Ala Ala Glu Gln Thr Arg Leu Tyr Gln Asn Pro Thr
                165                 170                 175
Thr Tyr Ile Ser Val Gly Thr Ser Thr Leu Asn Gln Arg Leu Val Pro
            180                 185                 190
Lys Ile Ala Thr Arg Ser Lys Val Asn Gly Gln Ser Gly Arg Met Glu
        195                 200                 205
Phe Phe Trp Thr Ile Leu Lys Pro Asn Asp Ala Ile Asn Phe Glu Ser
    210                 215                 220
Asn Gly Asn Phe Ile Ala Pro Glu Asn Ala Tyr Lys Ile Val Lys Lys
225                 230                 235                 240
Gly Asp Ser Thr Ile Met Lys Ser Glu Leu Glu Tyr Gly Asn Cys Asn
                245                 250                 255
Thr Lys Cys Gln Thr Pro Ile Gly Ala Ile Asn Ser Ser Met Pro Phe
            260                 265                 270
His Asn Ile His Pro Leu Thr Ile Gly Glu Cys Pro Lys Tyr Val Lys
        275                 280                 285
Ser Asn Arg Leu Val Leu Ala Thr Gly Leu Arg Asn Ser Pro Gln Gly
    290                 295                 300
Glu Arg Arg Arg Lys Lys Arg Gly Leu Phe Gly Ala Ile Ala Gly Phe
305                 310                 315                 320
Ile Glu Gly Gly Trp Gln Gly Met Val Asp Gly Trp Tyr Gly Tyr His
                325                 330                 335
His Ser Asn Glu Gln Gly Ser Gly Tyr Ala Ala Asp Lys Glu Ser Thr
            340                 345                 350
Gln Lys Ala Ile Asp Gly Val Thr Asn Lys Val Asn Ser Ile Ile Asp
        355                 360                 365

Lys Met Asn Thr Gln Phe Glu Ala Val Gly Arg Glu Phe Asn Asn Leu
    370                 375                 380
Glu Arg Arg Ile Glu Asn Leu Asn Lys Lys Met Glu Asp Gly Phe Leu
385                 390                 395                 400
Asp Val Trp Thr Tyr Asn Ala Glu Leu Leu Val Leu Met Glu Asn Glu
                405                 410                 415
Arg Thr Leu Asp Phe His Asp Ser Asn Val Lys Asn Leu Tyr Asp Lys
            420                 425                 430
Val Arg Leu Gln Leu Arg Asp Asn Ala Lys Glu Leu Gly Asn Gly Cys
        435                 440                 445
Phe Glu Phe Tyr His Arg Cys Asp Asn Glu Cys Met Glu Ser Val Arg
    450                 455                 460
Asn Gly Thr Tyr Asp Tyr Pro Gln Tyr Ser Glu Glu Ala Arg Leu Lys
465                 470                 475                 480
Arg Glu Glu Ile Ser Gly Val Lys Leu Glu Ser Ile Gly Thr Tyr Gln
                485                 490                 495
Ile Leu Ser Ile Tyr Ser Thr Val Ala Ser Ser Leu Ala Leu Ala Ile
            500                 505                 510
Met Val Ala Gly Leu Ser Leu Trp Met Cys Ser Asn Gly Ser Leu Gln
        515                 520                 525
Cys Arg Ile Cys Ile
    530

<210> 19
<211> 533
<212> PRT
<213> Influenza A virus

<400> 19
Met Glu Lys Asn Val Thr Val Thr His Ala Gln Asp Ile Leu Glu Lys
 1               5                  10                  15
Thr His Asn Gly Lys Leu Cys Asp Leu Asp Gly Val Lys Pro Leu Ile
            20                  25                  30
Leu Arg Asp Cys Ser Val Ala Gly Trp Leu Leu Gly Asn Pro Met Cys
        35                  40                  45
Asp Glu Phe Leu Asn Val Pro Glu Trp Ser Tyr Ile Val Glu Lys Ile
    50                  55                  60
Asn Pro Ala Asn Asp Leu Cys Tyr Pro Gly Asn Phe Asn Asp Tyr Glu
65                  70                  75                  80
Glu Leu Lys His Leu Leu Ser Arg Ile Asn His Phe Glu Lys Ile Gln
                85                  90                  95
Ile Ile Pro Lys Ser Ser Trp Ser Asp His Glu Ala Ser Ser Gly Val
            100                 105                 110
Ser Ser Ala Cys Pro Tyr Gln Gly Arg Ser Ser Phe Phe Arg Asn Val
        115                 120                 125
Val Trp Leu Ile Lys Lys Asp Asn Ala Tyr Pro Thr Ile Lys Arg Ser
    130                 135                 140
Tyr Asn Asn Thr Asn Gln Glu Asp Leu Leu Val Leu Trp Gly Ile His
145                 150                 155                 160
His Pro Asn Asp Ala Ala Glu Gln Thr Arg Leu Tyr Gln Asn Pro Thr
                165                 170                 175
Thr Tyr Ile Ser Val Gly Thr Ser Thr Leu Asn Gln Arg Leu Val Pro
            180                 185                 190
Lys Ile Ala Thr Arg Ser Lys Val Asn Gly Gln Ser Gly Arg Met Glu
        195                 200                 205
Phe Phe Trp Thr Ile Leu Lys Pro Asn Asp Ala Ile Asn Phe Glu Ser
    210                 215                 220
Asn Gly Asn Phe Ile Ala Pro Glu Asn Ala Tyr Lys Ile Val Lys Lys
225                 230                 235                 240
Gly Asp Ser Thr Ile Met Lys Ser Glu Leu Glu Tyr Gly Asn Cys Asn
                245                 250                 255
Thr Lys Cys Gln Thr Pro Ile Gly Ala Ile Asn Ser Ser Met Pro Phe
            260                 265                 270
His Asn Ile His Pro Leu Thr Ile Gly Glu Cys Pro Lys Tyr Val Lys

275                 280                 285
Ser Asn Arg Leu Val Leu Ala Thr Gly Leu Arg Asn Ser Pro Gln Gly
    290                 295                 300
Glu Arg Arg Arg Lys Lys Arg Gly Leu Phe Gly Ala Ile Ala Gly Phe
305                 310                 315                 320
Ile Glu Gly Gly Trp Gln Gly Met Val Asp Gly Trp Tyr Gly Tyr His
                325                 330                 335
His Ser Asn Glu Gln Gly Ser Gly Tyr Ala Ala Asp Lys Glu Ser Thr
            340                 345                 350
Gln Lys Ala Ile Asp Gly Val Thr Asn Lys Val Asn Ser Ile Ile Asp
        355                 360                 365
Lys Met Asn Thr Gln Phe Glu Ala Val Gly Arg Glu Phe Asn Asn Leu
    370                 375                 380
Glu Arg Arg Ile Glu Asn Leu Asn Lys Lys Met Glu Asp Gly Phe Leu
385                 390                 395                 400
Asp Val Trp Thr Tyr Asn Ala Glu Leu Leu Val Leu Met Glu Asn Glu
                405                 410                 415
Arg Thr Leu Asp Phe His Asp Ser Asn Val Lys Asn Leu Tyr Asp Lys
            420                 425                 430
Val Arg Leu Gln Leu Arg Asp Asn Ala Lys Glu Leu Gly Asn Gly Cys
        435                 440                 445
Phe Glu Phe Tyr His Arg Cys Asp Asn Glu Cys Met Glu Ser Val Arg
    450                 455                 460
Asn Gly Thr Tyr Asp Tyr Pro Gln Tyr Ser Glu Glu Ala Arg Leu Lys
465                 470                 475                 480
Arg Glu Glu Ile Ser Gly Val Lys Leu Glu Ser Ile Gly Thr Tyr Gln
                485                 490                 495
Ile Leu Ser Ile Tyr Ser Thr Val Ala Ser Ser Leu Ala Leu Ala Ile
            500                 505                 510
Met Val Ala Gly Leu Ser Leu Trp Met Cys Ser Asn Gly Ser Leu Gln
        515                 520                 525
Cys Arg Ile Cys Ile
    530

<210> 20
<211> 565
<212> PRT
<213> Influenza A virus

<400> 20
Met Glu Lys Ile Val Leu Leu Phe Ala Ile Val Ser Leu Val Lys Ser
1               5                   10                  15
Asp Gln Ile Cys Ile Gly Tyr His Ala Asn Asn Ser Thr Glu Gln Val
            20                  25                  30
Asp Thr Ile Met Glu Lys Asn Val Thr Val Thr His Ala Gln Asp Ile
        35                  40                  45
Leu Glu Lys Lys His Asn Gly Lys Leu Cys Asp Leu Asp Gly Val Lys
    50                  55                  60
Pro Leu Ile Leu Arg Asp Cys Ser Val Ala Gly Trp Leu Leu Gly Asn
65                  70                  75                  80
Pro Met Cys Asp Glu Phe Ile Asn Val Pro Glu Trp Ser Tyr Ile Val
                85                  90                  95
Glu Lys Ala Asn Pro Val Asn Asp Leu Cys Tyr Pro Gly Asp Phe Asn
            100                 105                 110
Asp Tyr Glu Glu Leu Lys His Leu Leu Ser Arg Ile Asn His Phe Glu
        115                 120                 125
Lys Ile Gln Ile Ile Pro Lys Ser Ser Trp Ser Ser His Glu Ala Ser
    130                 135                 140
Leu Gly Val Ser Ser Ala Cys Pro Tyr Gln Gly Lys Ser Ser Phe Phe
145                 150                 155                 160
Arg Asn Val Val Trp Leu Ile Lys Lys Asn Ser Thr Tyr Pro Thr Ile
                165                 170                 175
Lys Arg Ser Tyr Asn Asn Thr Asn Gln Glu Asp Leu Leu Val Leu Trp
            180                 185                 190

Gly Ile His His Pro Asn Asp Ala Ala Glu Gln Thr Lys Leu Tyr Gln
        195                 200                 205
Asn Pro Thr Thr Tyr Ile Ser Val Gly Thr Ser Thr Leu Asn Gln Arg
    210                 215                 220
Leu Val Pro Arg Ile Ala Thr Arg Ser Lys Val Asn Gly Gln Ser Gly
225                 230                 235                 240
Arg Met Glu Phe Phe Trp Thr Ile Leu Lys Pro Asn Asp Ala Ile Asn
                245                 250                 255
Phe Glu Ser Asn Gly Asn Phe Ile Ala Pro Glu Tyr Ala Tyr Lys Ile
            260                 265                 270
Val Lys Lys Gly Asp Ser Thr Ile Met Lys Ser Glu Leu Glu Tyr Gly
        275                 280                 285
Asn Cys Asn Thr Lys Cys Gln Thr Pro Met Gly Ala Ile Asn Ser Ser
    290                 295                 300
Met Pro Phe His Asn Ile His Pro Leu Thr Ile Gly Glu Cys Pro Lys
305                 310                 315                 320
Tyr Val Lys Ser Asn Arg Leu Val Leu Ala Thr Gly Leu Arg Asn Ser
                325                 330                 335
Pro Gln Arg Glu Arg Arg Arg Lys Lys Arg Gly Leu Phe Gly Ala Ile
            340                 345                 350
Ala Gly Phe Ile Glu Gly Gly Trp Gln Gly Met Val Asp Gly Trp Tyr
        355                 360                 365
Gly Tyr His His Ser Asn Glu Gln Gly Ser Gly Tyr Ala Ala Asp Lys
    370                 375                 380
Glu Ser Thr Gln Lys Ala Ile Asp Gly Val Thr Asn Lys Val Asn Ser
385                 390                 395                 400
Ile Ile Asp Lys Met Asn Thr Gln Phe Glu Ala Val Gly Arg Glu Phe
                405                 410                 415
Asn Asn Leu Glu Arg Arg Ile Glu Asn Leu Asn Lys Lys Met Glu Asp
            420                 425                 430
Gly Phe Leu Asp Val Trp Thr Tyr Asn Ala Glu Leu Leu Val Leu Met
        435                 440                 445
Glu Asn Glu Arg Thr Leu Asp Phe His Asp Ser Asn Val Lys Asn Leu
    450                 455                 460
Tyr Asp Lys Val Arg Leu Gln Leu Arg Asp Asn Ala Lys Glu Leu Gly
465                 470                 475                 480
Asn Gly Cys Phe Glu Phe Tyr His Lys Cys Asp Asn Glu Cys Met Glu
                485                 490                 495
Ser Val Arg Asn Gly Thr Tyr Asp Tyr Pro Gln Tyr Ser Glu Glu Ala
            500                 505                 510
Arg Leu Lys Arg Glu Glu Ile Ser Gly Val Lys Leu Glu Ser Ile Gly
        515                 520                 525
Ile Tyr Gln Ile Leu Ser Ile Tyr Ser Thr Val Ala Ser Ser Leu Ala
    530                 535                 540
Leu Ala Ile Met Val Ala Gly Leu Ser Leu Trp Met Cys Ser Asn Gly
545                 550                 555                 560
Ser Leu Gln Cys Arg
                565

<210> 21
<211> 122
<212> PRT
<213> Homo Sapiens

<400> 21
Gln Val Gln Leu Gln Glu Ser Gly Gly Gly Leu Val Gln Pro Gly Gly
1               5                   10                  15
Ser Leu Arg Leu Ser Cys Ala Ala Ser Gly Phe Pro Phe Ser Ser Tyr
            20                  25                  30
Val Met Ile Trp Val Arg Gln Val Pro Gly Lys Gly Leu Glu Trp Val
        35                  40                  45
Ser Ala Ile Gly Gly Ser Gly Gly Ser Thr Tyr Tyr Ala Asp Ser Val
    50                  55                  60
Lys Gly Arg Phe Thr Ile Ser Arg Asp Asn Ser Lys Asn Thr Leu Tyr

65                  70                  75                  80
Leu Gln Met Asn Ser Leu Arg Ala Asp Asp Thr Ala Val Tyr Tyr Cys
                85                  90                  95
Val Leu Ser Pro Lys Ser Tyr Tyr Asp Asn Ser Gly Ile Tyr Phe Asp
            100                 105                 110
Phe Trp Gly Lys Gly Thr Leu Val Arg Val
        115                 120

<210> 22
<211> 98
<212> PRT
<213> Homo Sapiens

<400> 22
Glu Val Gln Leu Leu Glu Ser Gly Gly Gly Leu Val Gln Pro Gly Gly
1               5                   10                  15
Ser Leu Arg Leu Ser Cys Ala Ala Ser Gly Phe Thr Phe Ser Ser Tyr
            20                  25                  30
Ala Met Ser Trp Val Arg Gln Ala Pro Gly Lys Gly Leu Glu Trp Val
        35                  40                  45
Ser Ala Ile Ser Gly Ser Gly Gly Ser Thr Tyr Tyr Ala Asp Ser Val
    50                  55                  60
Lys Gly Arg Phe Thr Ile Ser Arg Asp Asn Ser Lys Asn Thr Leu Tyr
65                  70                  75                  80
Leu Gln Met Asn Ser Leu Arg Ala Glu Asp Thr Ala Val Tyr Tyr Cys
                85                  90                  95
Ala Lys

<210> 23
<211> 108
<212> PRT
<213> Homo Sapiens

<400> 23
Leu Pro Glu Leu Thr Gln Pro Pro Ser Val Ser Val Ala Pro Gly Lys
1               5                   10                  15
Thr Ala Arg Ile Thr Cys Gly Gly Asn Asn Ile Gly Ser Lys Ser Val
            20                  25                  30
Tyr Trp Tyr Gln Gln Lys Pro Gly Gln Ala Pro Val Leu Val Val Tyr
        35                  40                  45
Glu Asp Ser Asp Arg Pro Ser Gly Leu Pro Glu Arg Phe Ser Gly Ser
    50                  55                  60
Asn Ser Gly Asn Thr Ala Thr Leu Thr Ile Ser Arg Val Glu Ala Gly
65                  70                  75                  80
Asp Glu Ala Asp Tyr Tyr Cys Gln Val Trp Asp Ser Thr Ser Asp His
                85                  90                  95
Trp Val Phe Gly Gly Arg Thr Lys Leu Thr Val Leu
            100                 105

<210> 24
<211> 96
<212> PRT
<213> Homo Sapiens

<400> 24
Ser Tyr Val Leu Thr Gln Pro Pro Ser Val Ser Val Ala Pro Gly Lys
1               5                   10                  15
Thr Ala Arg Ile Thr Cys Gly Gly Asn Asn Ile Gly Ser Lys Ser Val
            20                  25                  30
His Trp Tyr Gln Gln Lys Pro Gly Gln Ala Pro Val Leu Val Ile Tyr
        35                  40                  45

Tyr Asp Ser Asp Arg Pro Ser Gly Ile Pro Glu Arg Phe Ser Gly Ser
    50                  55                  60
Asn Ser Gly Asn Thr Ala Thr Leu Thr Ile Ser Arg Val Glu Ala Gly
65                  70                  75                  80
Asp Glu Ala Asp Tyr Tyr Cys Gln Val Trp Asp Ser Ser Ser Asp His
                85                  90                  95

<210> 25
<211> 111
<212> PRT
<213> Homo Sapiens

<400> 25
Gln Ala Val Leu Thr Gln Pro Pro Ser Ala Ser Gly Thr Pro Gly Gln
 1               5                  10                  15
Arg Val Thr Ile Ser Cys Ser Gly Ser Ser Ser Asn Ile Gly Ser Tyr
            20                  25                  30
Thr Val Asn Trp Tyr Gln Gln Leu Pro Gly Ser Ala Pro Lys Leu Leu
        35                  40                  45
Ile Tyr Ser Asn Asn His Arg Pro Ser Gly Val Pro Asp Arg Phe Ser
    50                  55                  60
Gly Ser Lys Ser Gly Thr Ser Ala Ser Leu Ala Ile Ser Gly Leu Gln
65                  70                  75                  80
Ser Glu Asp Glu Ala Asp Tyr Tyr Cys Ala Ala Trp Ala Asp Arg Gln
                85                  90                  95
Asn Gly Trp Val Phe Gly Gly Gly Thr Lys Leu Thr Val Leu Gly
            100                 105                 110

<210> 26
<211> 98
<212> PRT
<213> Homo Sapiens

<400> 26
Gln Ser Val Leu Thr Gln Pro Pro Ser Ala Ser Gly Thr Pro Gly Gln
 1               5                  10                  15
Arg Val Thr Ile Ser Cys Ser Gly Ser Ser Ser Asn Ile Gly Ser Asn
            20                  25                  30
Thr Val Asn Trp Tyr Gln Gln Leu Pro Gly Thr Ala Pro Lys Leu Leu
        35                  40                  45
Ile Tyr Ser Asn Asn Gln Arg Pro Ser Gly Val Pro Asp Arg Phe Ser
    50                  55                  60
Gly Ser Lys Ser Gly Thr Ser Ala Ser Leu Ala Ile Ser Gly Leu Gln
65                  70                  75                  80
Ser Glu Asp Glu Ala Asp Tyr Tyr Cys Ala Ala Trp Asp Asp Ser Leu
                85                  90                  95
Asn Gly

<210> 27
<211> 122
<212> PRT
<213> Homo Sapiens

<400> 27
Glu Val Gln Leu Val Glu Ser Gly Gly Gly Leu Val Gln Pro Gly Gly
 1               5                  10                  15
Ser Leu Arg Leu Ser Cys Ala Ala Ser Gly Phe Pro Phe Ser Ser Tyr
            20                  25                  30
Val Met Ile Trp Val Arg Gln Val Pro Gly Lys Gly Leu Glu Trp Val
        35                  40                  45
Ser Ala Ile Gly Gly Ser Gly Gly Ser Thr Tyr Tyr Ala Asp Ser Val

50                  55                  60
Lys Gly Arg Phe Thr Ile Ser Arg Asp Asn Ser Lys Asn Thr Leu Tyr
65                  70                  75                  80
Leu Gln Met Asn Ser Leu Arg Ala Asp Asp Thr Ala Val Tyr Tyr Cys
                85                  90                  95
Val Leu Ser Pro Lys Ser Tyr Tyr Asp Asn Ser Gly Ile Tyr Phe Asp
            100                 105                 110
Phe Trp Gly Gln Gly Thr Leu Val Arg Val
        115                 120

<210> 28
<211> 112
<212> PRT
<213> Homo Sapiens

<400> 28
Asn Phe Met Leu Thr Gln Pro His Ser Val Ser Glu Ser Pro Gly Lys
1               5                   10                  15
Thr Ile Thr Ile Ser Cys Thr Ala Ser Ser Gly Ser Ile Ala Ser Asn
            20                  25                  30
Tyr Val Gln Trp Tyr Gln Gln Arg Pro Gly Ser Ala Pro Thr Ile Val
        35                  40                  45
Ile Tyr Glu Asp Asn Gln Arg Pro Ser Gly Val Pro Asp Arg Phe Ser
    50                  55                  60
Gly Ser Ile Asp Thr Ser Ser Asn Ser Ala Ser Leu Thr Ile Ser Gly
65                  70                  75                  80
Leu Lys Thr Glu Asp Glu Ala Asp Tyr Tyr Cys Gln Ser Tyr Asp Asn
                85                  90                  95
Thr Asn His Trp Val Phe Gly Gly Gly Thr Lys Val Thr Val Leu Gly
            100                 105                 110

<210> 29
<211> 98
<212> PRT
<213> Homo Sapiens

<400> 29
Asn Phe Met Leu Thr Gln Pro His Ser Val Ser Glu Ser Pro Gly Lys
1               5                   10                  15
Thr Val Thr Ile Ser Cys Thr Arg Ser Ser Gly Ser Ile Ala Ser Asn
            20                  25                  30
Tyr Val Gln Trp Tyr Gln Gln Arg Pro Gly Ser Ser Pro Thr Thr Val
        35                  40                  45
Ile Tyr Glu Asp Asn Gln Arg Pro Ser Gly Val Pro Asp Arg Phe Ser
    50                  55                  60
Gly Ser Ile Asp Ser Ser Ser Asn Ser Ala Ser Leu Thr Ile Ser Gly
65                  70                  75                  80
Leu Lys Thr Glu Asp Glu Ala Asp Tyr Tyr Cys Gln Ser Tyr Asp Ser
                85                  90                  95
Ser Asn

<210> 30
<211> 124
<212> PRT
<213> Homo Sapiens

<400> 30
Met Ala Glu Val Gln Leu Val Glu Ser Gly Gly Gly Leu Val Gln Pro
1               5                   10                  15
Gly Gly Ser Leu Arg Leu Ser Cys Ala Ala Ser Gly Phe Pro Phe Ser
            20                  25                  30

Ser Tyr Val Met Ile Trp Val Arg Gln Val Pro Gly Lys Gly Leu Glu
        35                  40                  45
Trp Val Ser Ala Ile Gly Gly Ser Gly Gly Ser Thr Tyr Tyr Ala Asp
    50                  55                  60
Ser Val Lys Gly Arg Phe Thr Ile Ser Arg Asp Asn Ser Lys Asn Thr
65                  70                  75                  80
Leu Tyr Leu Gln Met Asn Ser Leu Arg Ala Asp Asp Thr Ala Val Tyr
                85                  90                  95
Tyr Cys Val Leu Ser Pro Lys Ser Tyr Tyr Asp Asn Ser Gly Ile Tyr
            100                 105                 110
Phe Asp Phe Trp Gly Arg Gly Thr Leu Val Arg Val
        115                 120

<210> 31
<211> 112
<212> PRT
<213> Homo Sapiens

<400> 31
Gln Ser Val Leu Thr Gln Pro Pro Ser Val Ser Gly Ala Pro Gly Gln
1               5                   10                  15
Arg Val Thr Ile Ser Cys Thr Gly Ser Ser Ser Asn Thr Gly Ala Gly
            20                  25                  30
Asn His Val His Trp Tyr Gln Gln Val Ala Gly Ala Ala Pro Lys Leu
        35                  40                  45
Leu Ile Ser Asn Asn Asn Asn Arg Pro Ser Gly Val Pro Asp Arg Phe
    50                  55                  60
Ser Ala Ser Lys Ser Gly Thr Ser Ala Ser Leu Asp Ile Thr Gly Leu
65                  70                  75                  80
Gln Ala Glu Asp Glu Ala Asp Tyr Tyr Cys Gln Ser Tyr Asp Asn Ser
                85                  90                  95
Leu Asn Asp Trp Val Phe Gly Gly Gly Thr Lys Leu Thr Val Leu Gly
            100                 105                 110

<210> 32
<211> 99
<212> PRT
<213> Homo Sapiens

<400> 32
Gln Ser Val Leu Thr Gln Pro Pro Ser Val Ser Gly Ala Pro Gly Gln
1               5                   10                  15
Arg Val Thr Ile Ser Cys Thr Gly Ser Ser Ser Asn Ile Gly Ala Gly
            20                  25                  30
Tyr Asp Val His Trp Tyr Gln Gln Leu Pro Gly Thr Ala Pro Lys Leu
        35                  40                  45
Leu Ile Tyr Gly Asn Ser Asn Arg Pro Ser Gly Val Pro Asp Arg Phe
    50                  55                  60
Ser Gly Ser Lys Ser Gly Thr Ser Ala Ser Leu Ala Ile Thr Gly Leu
65                  70                  75                  80
Gln Ala Glu Asp Glu Ala Asp Tyr Tyr Cys Gln Ser Tyr Asp Ser Ser
                85                  90                  95
Leu Ser Gly

<210> 33
<211> 121
<212> PRT
<213> Homo Sapiens

<400> 33
Gln Val Gln Leu Val Gln Ser Gly Gly Gly Leu Val Gln Pro Gly Gly

1               5                   10                  15
Ser Leu Arg Leu Ser Cys Ala Ala Ser Gly Phe Thr Leu Ser Thr Tyr
            20                  25                  30
Gly Met His Trp Val Arg Gln Ala Ala Gly Lys Gly Leu Glu Trp Val
        35                  40                  45
Ala Val Ser Ser Tyr Asp Gly Arg Asn Glu Tyr Tyr Ala Asp Ser Val
    50                  55                  60
Lys Gly Arg Phe Thr Ile Ser Arg Asp Asn Phe Lys Asp Thr Leu Tyr
65                  70                  75                  80
Leu Gln Met Asn Asn Leu Arg Ala Glu Asp Thr Ala Val Tyr Tyr Cys
                85                  90                  95
Ala Lys Glu Val Gly Met Arg Ser Tyr Asp Ser Tyr Gly Met Asp Val
            100                 105                 110
Trp Gly Lys Gly Thr Leu Val Arg Val
        115                 120

<210> 34
<211> 108
<212> PRT
<213> Homo Sapiens

<400> 34
Asp Ile Gln Leu Thr Gln Ser Pro Ser Thr Leu Ser Ala Ser Val Gly
 1               5                   10                  15
Gly Arg Val Thr Ile Thr Cys Arg Ala Ser Gln Ser Ile Gly Pro Trp
            20                  25                  30
Leu Ala Trp Tyr Gln Gln Lys Pro Gly Lys Ala Pro Glu Leu Leu Ile
        35                  40                  45
Tyr Glu Ala Ser Ser Leu Glu Ser Gly Val Pro Ser Arg Phe Ser Gly
    50                  55                  60
Gly Gly Ser Gly Thr Glu Phe Thr Leu Thr Ile Ser Arg Leu Gln Pro
65                  70                  75                  80
Asp Asp Ile Ala Thr Tyr Tyr Cys Gln Gln Tyr Ser Ser Tyr Pro Trp
                85                  90                  95
Thr Phe Gly Gln Gly Thr Lys Leu Glu Ile Lys Arg
            100                 105

<210> 35
<211> 95
<212> PRT
<213> Homo Sapiens

<400> 35
Asp Ile Gln Met Thr Gln Ser Pro Ser Thr Leu Ser Ala Ser Val Gly
 1               5                   10                  15
Asp Arg Val Thr Ile Thr Cys Arg Ala Ser Gln Ser Ile Ser Ser Trp
            20                  25                  30
Leu Ala Trp Tyr Gln Gln Lys Pro Gly Lys Ala Pro Lys Leu Leu Ile
        35                  40                  45
Tyr Asp Ala Ser Ser Leu Glu Ser Gly Val Pro Ser Arg Phe Ser Gly
    50                  55                  60
Ser Gly Ser Gly Thr Glu Phe Thr Leu Thr Ile Ser Ser Leu Gln Pro
65                  70                  75                  80
Asp Asp Phe Ala Thr Tyr Tyr Cys Gln Gln Tyr Asn Ser Tyr Ser
                85                  90                  95

<210> 36
<211> 98
<212> PRT
<213> Homo Sapiens

<400> 36

Gln Val Gln Leu Val Glu Ser Gly Gly Gly Val Val Gln Pro Gly Arg
1               5                   10                  15
Ser Leu Arg Leu Ser Cys Ala Ala Ser Gly Phe Thr Phe Ser Ser Tyr
            20                  25                  30
Gly Met His Trp Val Arg Gln Ala Pro Gly Lys Gly Leu Glu Trp Val
        35                  40                  45
Ala Val Ile Ser Tyr Asp Gly Ser Asn Lys Tyr Tyr Ala Asp Ser Val
    50                  55                  60
Lys Gly Arg Phe Thr Ile Ser Arg Asp Asn Ser Lys Asn Thr Leu Tyr
65                  70                  75                  80
Leu Gln Met Asn Ser Leu Arg Ala Glu Asp Thr Ala Val Tyr Tyr Cys
                85                  90                  95
Ala Lys

<210> 37
<211> 121
<212> PRT
<213> Homo Sapiens

<400> 37
Glu Val Gln Leu Val Glu Ser Gly Gly Gly Val Val Gln Pro Gly Arg
1               5                   10                  15
Ser Leu Arg Leu Ser Cys Ala Ala Ser Gly Phe Thr Phe Ser Ser Tyr
            20                  25                  30
Gly Met His Trp Val Arg Gln Ala Pro Gly Lys Gly Leu Glu Trp Val
        35                  40                  45
Ala His Ile Ser Tyr Asp Gly Thr Glu Thr His Tyr Thr Asp Ser Val
    50                  55                  60
Lys Gly Arg Phe Thr Ile Ser Arg Asp Asn Ser Met Glu Thr Ala Tyr
65                  70                  75                  80
Leu Gln Met Asp Ser Leu Arg Ala Glu Asp Thr Ala Val Tyr Tyr Cys
                85                  90                  95
Ala Lys Asp Val Ser Leu Arg Ala Tyr Asp His Tyr Gly Met Asp Val
            100                 105                 110
Trp Gly Arg Gly Thr Leu Val Arg Val
        115                 120

<210> 38
<211> 109
<212> PRT
<213> Homo Sapiens

<400> 38
Ser Ser Glu Leu Thr Gln Asp Pro Ala Val Ser Val Ala Leu Gly Gln
1               5                   10                  15
Thr Val Arg Ile Thr Cys Gln Gly Glu Ser Leu Arg Asn Tyr Tyr Ala
            20                  25                  30
Asn Trp Tyr Gln Gln Arg Pro Gly Gln Ala Pro Val Leu Val Ile Tyr
        35                  40                  45
Gly Gly Asn Ser Arg Pro Ser Gly Ile Ala Asp Arg Phe Ser Gly Ser
    50                  55                  60
Ser Ser Gly Ile Thr Ala Ser Leu Thr Ile Thr Gly Ala Gln Ala Glu
65                  70                  75                  80
Asp Glu Ala Asp Tyr Tyr Cys Asp Ser Arg Asp Ser Ser Asp Asn His
                85                  90                  95
Arg Val Phe Gly Gly Arg Thr Gln Leu Thr Val Leu Ser
            100                 105

<210> 39
<211> 96
<212> PRT

<213> Homo Sapiens

<400> 39
Ser Ser Glu Leu Thr Gln Asp Pro Ala Val Ser Val Ala Leu Gly Gln
1               5                   10                  15
Thr Val Arg Ile Thr Cys Gln Gly Asp Ser Leu Arg Ser Tyr Tyr Ala
            20                  25                  30
Ser Trp Tyr Gln Gln Lys Pro Gly Gln Ala Pro Val Leu Val Ile Tyr
        35                  40                  45
Gly Lys Asn Asn Arg Pro Ser Gly Ile Pro Asp Arg Phe Ser Gly Ser
    50                  55                  60
Ser Ser Gly Asn Thr Ala Ser Leu Thr Ile Thr Gly Ala Gln Ala Glu
65                  70                  75                  80
Asp Glu Ala Asp Tyr Tyr Cys Asn Ser Arg Asp Ser Ser Gly Asn His
                85                  90                  95


<210> 40
<211> 121
<212> PRT
<213> Homo Sapiens

<400> 40
Gln Val Gln Leu Val Gln Ser Gly Gly Gly Val Val Gln Pro Gly Arg
1               5                   10                  15
Ser Leu Arg Leu Ser Cys Gly Ala Ser Gly Phe Thr Leu Ser Thr Tyr
            20                  25                  30
Gly Met His Trp Val Arg Gln Ala Ala Gly Lys Gly Leu Glu Trp Val
        35                  40                  45
Ala Val Ser Ser Tyr Asp Gly Arg Asn Glu Tyr Tyr Ala Asp Ser Val
    50                  55                  60
Lys Gly Arg Phe Thr Ile Ser Arg Asp Asn Phe Lys Asp Thr Leu Tyr
65                  70                  75                  80
Leu Gln Met Asn Asn Leu Arg Ala Glu Asp Thr Ala Val Tyr Tyr Cys
                85                  90                  95
Ala Lys Glu Val Gly Met Arg Ser Tyr Asp Ser Tyr Gly Met Asp Val
            100                 105                 110
Trp Gly Arg Gly Thr Leu Val Arg Val
        115                 120


<210> 41
<211> 108
<212> PRT
<213> Homo Sapiens

<400> 41
Asp Ile Gln Leu Thr Gln Ser Pro Ser Ser Val Ser Ala Ser Val Gly
1               5                   10                  15
Asp Arg Val Thr Ile Thr Cys Arg Ala Ser Gln Asp Ile Ser Asn Trp
            20                  25                  30
Leu Ala Trp Tyr Gln Gln Lys Pro Gly Glu Ala Pro Lys Val Leu Ile
        35                  40                  45
Phe Gly Ala Ser Ser Leu Gln Ser Gly Val Pro Ser Arg Phe Ser Gly
    50                  55                  60
Ser Gly Ser Gly Thr Glu Phe Thr Leu Thr Ile Ser Ser Leu Gln Ala
65                  70                  75                  80
Glu Asp Phe Ala Thr Tyr Tyr Cys Gln Gln Ser Asn Asn Phe Pro Tyr
                85                  90                  95
Thr Phe Gly Gln Gly Thr Lys Leu Glu Ile Lys Arg
            100                 105


<210> 42
<211> 95

<212> PRT
<213> Homo Sapiens

<400> 42
Asp Ile Gln Met Thr Gln Ser Pro Ser Ser Val Ser Ala Ser Val Gly
1               5                   10                  15
Asp Arg Val Thr Ile Thr Cys Arg Ala Ser Gln Gly Ile Ser Ser Trp
            20                  25                  30
Leu Ala Trp Tyr Gln Gln Lys Pro Gly Lys Ala Pro Lys Leu Leu Ile
        35                  40                  45
Tyr Ala Ala Ser Ser Leu Gln Ser Gly Val Pro Ser Arg Phe Ser Gly
    50                  55                  60
Ser Gly Ser Gly Thr Asp Phe Thr Leu Thr Ile Ser Ser Leu Gln Pro
65                  70                  75                  80
Glu Asp Phe Ala Thr Tyr Tyr Cys Gln Gln Ala Asn Ser Phe Pro
                85                  90                  95


<210> 43
<211> 121
<212> PRT
<213> Homo Sapiens

<400> 43
Glu Val Gln Leu Val Glu Ser Gly Gly Gly Val Val Gln Pro Gly Arg
1               5                   10                  15
Thr Leu Arg Leu Ser Cys Ala Ala Ser Gly Phe Thr Phe Thr Ser Tyr
            20                  25                  30
Gly Met His Trp Val Arg Gln Ala Pro Gly Lys Gly Leu Glu Trp Val
        35                  40                  45
Ala Val Ile Ser Tyr Asp Gly Arg Lys Lys Tyr Tyr Leu Asp Ser Val
    50                  55                  60
Lys Gly Arg Phe Thr Ile Ser Arg Asp Asn Ser Met Glu Thr Ala Tyr
65                  70                  75                  80
Leu Gln Met Asp Ser Leu Arg Pro Glu Asp Thr Ala Val Tyr Tyr Cys
                85                  90                  95
Ala Lys Asp Val Ser Leu Arg Ala Tyr Asp His Tyr Gly Met Asp Val
            100                 105                 110
Trp Gly Gln Gly Thr Leu Val Arg Val
        115                 120


<210> 44
<211> 108
<212> PRT
<213> Homo Sapiens

<400> 44
Asp Ile Gln Met Thr Gln Ser Pro Ser Ser Leu Ser Ala Ser Val Gly
1               5                   10                  15
Asp Arg Val Thr Ile Thr Cys Arg Ala Ser Gln Gly Ile Ser Asn Tyr
            20                  25                  30
Leu Ala Trp Tyr Gln Gln Lys Pro Gly Lys Val Pro Lys Leu Leu Ile
        35                  40                  45
Tyr Ala Ala Ser Thr Leu Gln Ser Gly Val Pro Ser Arg Phe Ser Gly
    50                  55                  60
Ser Gly Ser Gly Thr Asp Phe Thr Leu Thr Ile Ser Ser Leu Gln Pro
65                  70                  75                  80
Glu Asp Val Ala Thr Tyr Phe Cys Gln Gln Ser Tyr Ser Thr Pro Phe
                85                  90                  95
Thr Phe Gly Gly Gly Thr Lys Val Asp Ile Lys Arg
            100                 105


<210> 45

<211> 95
<212> PRT
<213> Homo Sapiens

<400> 45
Asp Ile Gln Met Thr Gln Ser Pro Ser Ser Leu Ser Ala Ser Val Gly
1               5                   10                  15
Asp Arg Val Thr Ile Thr Cys Arg Ala Ser Gln Gly Ile Ser Asn Tyr
            20                  25                  30
Leu Ala Trp Tyr Gln Gln Lys Pro Gly Lys Val Pro Lys Leu Leu Ile
        35                  40                  45
Tyr Ala Ala Ser Thr Leu Gln Ser Gly Val Pro Ser Arg Phe Ser Gly
    50                  55                  60
Ser Gly Ser Gly Thr Asp Phe Thr Leu Thr Ile Ser Ser Leu Gln Pro
65                  70                  75                  80
Glu Asp Val Ala Thr Tyr Tyr Cys Gln Lys Tyr Asn Ser Ala Pro
                85                  90                  95


<210> 46
<211> 270
<212> PRT
<213> Homo Sapiens

<400> 46
Met Ala Gln Val Gln Leu Gln Gln Ser Gly Pro Gly Leu Val Lys Pro
1               5                   10                  15
Ser Glu Thr Leu Ser Leu Thr Cys Ser Val Ser Gly Asp Ser Ile Lys
            20                  25                  30
Ser Arg Arg Tyr Tyr Trp Ala Trp Ile Arg Gln Pro Pro Gly Lys Gly
        35                  40                  45
Met Glu Phe Ile Gly Arg Leu Ser His Asp Gly Ser Thr Tyr Tyr Thr
    50                  55                  60
Pro Ser Leu Lys Ser Arg Leu Thr Ile Ser Pro Asp Thr Ser Lys Asn
65                  70                  75                  80
Gln Phe Ser Leu Lys Leu Ser Ser Val Thr Ala Ala Asp Thr Ala Met
                85                  90                  95
Tyr Tyr Cys Ala Arg Gly Val Tyr Asp Trp Gly Asn Ser Tyr Gln Leu
            100                 105                 110
Asp Val Trp Gly Gln Gly Thr Leu Val Thr Val Ser Ser Gly Gly Gly
        115                 120                 125
Gly Ser Gly Gly Gly Gly Ser Gly Gly Gly Gly Ser Ser Ala Leu Asp
    130                 135                 140
Ile Gln Met Thr Gln Ser Pro Ser Thr Leu Ser Thr Ser Val Gly Asp
145                 150                 155                 160
Arg Val Thr Ile Thr Cys Arg Ala Ser Gln Asn Ile Ser Asn Trp Leu
                165                 170                 175
Ala Trp Tyr Gln Gln Lys Pro Gly Lys Ala Pro Lys Leu Leu Ile Tyr
            180                 185                 190
Lys Ala Ser Ser Leu Glu Ser Gly Val Pro Ser Arg Phe Ser Gly Ser
        195                 200                 205
Gly Ser Gly Thr Glu Phe Thr Leu Thr Ile Ala Ser Leu Gln Pro Asp
    210                 215                 220
Asp Phe Ala Thr Tyr Tyr Cys Gln Gln Tyr Tyr Arg Ser Trp Thr Phe
225                 230                 235                 240
Gly Gln Gly Thr Lys Leu Glu Ile Lys Arg Ala Ala Ala His His His
                245                 250                 255
His His His Gly Glu Gln Lys Leu Ile Ser Glu Glu Asp Leu
            260                 265                 270


<210> 47
<211> 269
<212> PRT
<213> Homo Sapiens

<400> 47
Met Ala Gln Val Gln Leu Val Gln Ser Gly Ala Glu Val Lys Lys Pro
1               5                   10                  15
Gly Glu Ser Leu Lys Ile Ser Cys Lys Ser Ser Gly Tyr Lys Leu Ser
            20                  25                  30
Ser Tyr Trp Ile Ala Trp Val Arg Gln Val Pro Gly Lys Gly Leu Glu
        35                  40                  45
Trp Val Gly Ile Ile Asp Pro Arg Asp Ser Asp Thr Arg Tyr Ser Pro
    50                  55                  60
Ser Phe Glu Gly Gln Val Thr Ile Ser Ala Asp Lys Ser Ile Asn Thr
65                  70                  75                  80
Ala Tyr Leu Gln Trp Ser Ser Leu Lys Thr Ala Asp Thr Ala Met Tyr
                85                  90                  95
Phe Cys Ala Arg Gln Ala Asp Gly Tyr Arg Ser Phe Tyr Gly Met Asp
            100                 105                 110
Val Trp Gly Arg Gly Thr Leu Val Thr Val Ser Ser Gly Gly Gly Gly
        115                 120                 125
Ser Gly Gly Gly Gly Ser Gly Gly Gly Gly Ser Ser Ala Leu Asp Ile
    130                 135                 140
Gln Leu Thr Gln Ser Pro Ser Ser Leu Ser Ala Ser Val Gly Asp Arg
145                 150                 155                 160
Val Thr Ile Thr Cys Arg Ala Ser Gln Thr Ile Asn Asn Tyr Leu Asn
                165                 170                 175
Trp Tyr Gln Gln Lys Pro Gly Lys Ala Pro Lys Leu Leu Ile Tyr Ala
            180                 185                 190
Ala Ser Ser Leu Gln Ser Gly Val Pro Ser Arg Phe Ser Gly Ser Gly
        195                 200                 205
Ser Gly Thr Asp Phe Thr Leu Thr Ile Ser Ser Leu Gln Pro Glu Asp
    210                 215                 220
His Ala Ser Tyr Tyr Cys Gln Gln Ser Tyr Ser Thr Pro Val Thr Phe
225                 230                 235                 240
Gly Gln Gly Thr Arg Leu Glu Ile Lys Arg Ala Ala Ala His His His
                245                 250                 255
His His His Gly Glu Gln Lys Leu Ile Ser Glu Asp Leu
            260                 265

<210> 48
<211> 272
<212> PRT
<213> Homo Sapiens

<400> 48
Met Ala Gln Met Gln Leu Val Gln Ser Gly Ala Glu Val Lys Lys Pro
1               5                   10                  15
Gly Ser Ser Val Lys Ile Ser Cys Lys Ala Ser Gly Gly Thr Phe Asn
            20                  25                  30
Lys Tyr Ile Ile Ser Trp Val Arg Gln Ala Pro Gly Gln Gly Leu Glu
        35                  40                  45
Trp Val Gly Arg Ile Val Pro Ile Thr Gly Ile Thr Asn Tyr Ala Gln
    50                  55                  60
Arg Leu Gln Gly Arg Val Thr Ile Ser Ala Asp Lys Ser Thr Asn Thr
65                  70                  75                  80
Ala Tyr Met Glu Leu Arg Ser Leu Lys Ser Glu Asp Thr Ala Val Tyr
                85                  90                  95
Tyr Cys Ala Arg Asp Gln Gly Asp Leu Trp Pro His Gln Tyr Gln Gly
            100                 105                 110
Thr Asp Val Trp Gly Lys Gly Thr Thr Val Thr Val Ser Ser Gly Gly
        115                 120                 125
Gly Gly Ser Gly Gly Gly Gly Ser Gly Gly Gly Gly Ser Ser Ala Glu
    130                 135                 140
Ile Val Leu Thr Gln Ser Pro Leu Ser Leu Pro Val Thr Pro Gly Glu
145                 150                 155                 160
Pro Ala Ser Ile Ser Cys Arg Ser Ser Gln Ser Leu Leu Tyr Ser Asn

165                 170                 175
Gly Tyr Asn Tyr Leu Asp Trp Tyr Leu Gln Lys Pro Gly Gln Ser Pro
            180                 185                 190
Gln Leu Leu Ile Tyr Leu Gly Ser Asn Arg Ala Ser Gly Val Pro Asp
        195                 200                 205
Arg Phe Ser Gly Ser Gly Ser Gly Thr Asp Phe Thr Leu Lys Ile Ser
    210                 215                 220
Arg Val Glu Ala Glu Asp Val Gly Val Tyr Tyr Cys Met Gln Ala Leu
225                 230                 235                 240
Gln Val Pro His Thr Phe Gly Gln Gly Thr Lys Val Glu Ile Lys Arg
                245                 250                 255
Ala Ala Ala His His His His His His Gly Glu Gln Lys Ile Asp Leu
            260                 265                 270

<210> 49
<211> 269
<212> PRT
<213> Homo Sapiens

<400> 49
Met Ala Gln Val Gln Leu Gln Gln Ser Gly Gly Gly Val Val Gln Pro
 1               5                  10                  15
Gly Arg Ser Leu Arg Leu Ser Cys Thr Thr Ser Gly Phe Ile Phe Lys
            20                  25                  30
Thr Tyr Asp Met His Trp Leu Arg Gln Ala Pro Gly Lys Gly Leu Glu
        35                  40                  45
Trp Val Ala Phe Ile Arg His Asp Gly Arg Asp Ile Lys Tyr Ala Asp
    50                  55                  60
Ser Val Lys Gly Arg Phe Thr Ile Ser Arg Asp Asp Ser Lys Asp Thr
65                  70                  75                  80
Leu Tyr Leu Gln Met Asp Ser Leu Arg Ala Glu Asp Thr Ala Val Tyr
                85                  90                  95
Phe Cys Ala Arg Asn Arg Phe Thr Gly Tyr Asn Tyr Phe Glu His Trp
            100                 105                 110
Gly Gln Gly Thr Leu Val Thr Val Ser Ser Gly Gly Gly Gly Ser Gly
        115                 120                 125
Gly Gly Gly Ser Gly Gly Gly Gly Ser Ser Ala Leu Glu Ile Val Met
    130                 135                 140
Thr Gln Ser Pro Gly Thr Leu Ser Leu Ser Pro Gly Glu Arg Ala Thr
145                 150                 155                 160
Leu Ser Cys Arg Ala Ser Gln Ser Val Ser Ser Arg Tyr Leu Ala Trp
                165                 170                 175
Tyr Gln Gln Lys Pro Gly Gln Ala Pro Arg Leu Leu Ile Tyr Gly Val
            180                 185                 190
Ser Ser Arg Ala Thr Gly Ile Pro Asp Arg Phe Ser Gly Ser Gly Ser
        195                 200                 205
Gly Thr Asp Phe Thr Leu Thr Ile Ser Arg Leu Glu Pro Glu Asp Phe
    210                 215                 220
Ala Val Tyr Tyr Cys Gln Gln Tyr Ala Ala Ser Pro Glu Thr Phe Gly
225                 230                 235                 240
Gln Gly Thr Lys Val Glu Ile Lys Arg Ala Ala Ala His His His His
                245                 250                 255
His His Gly Glu Gln Lys Leu Ile Ser Glu Glu Asp Leu
            260                 265

<210> 50
<211> 268
<212> PRT
<213> Homo Sapiens

<400> 50
Met Ala Glu Val Gln Leu Val Glu Ser Gly Gly Gly Leu Ala Gln Pro
 1               5                  10                  15

Gly Gly Ser Leu Arg Leu Ser Cys Ser Ala Ser Gly Phe Ser Phe Lys
            20                  25                  30
Thr Tyr Asp Met His Trp Val Arg Gln Ala Pro Gly Lys Gly Pro Glu
        35                  40                  45
Trp Ile Ser Lys Ile Asp Tyr Gly Asn Arg Thr Thr Asp Tyr Ala Asp
    50                  55                  60
Ser Val Lys Gly Arg Phe Thr Ile Ser Arg Asp Asn Ala Lys Asn Ser
65                  70                  75                  80
Leu Tyr Leu Gln Met Ser Ser Leu Arg Ala Glu Asp Thr Ala Val Tyr
                85                  90                  95
Tyr Cys Ala Arg Thr Arg Phe Ser Gly Tyr Asp Tyr Phe Glu Asp Trp
            100                 105                 110
Gly Lys Gly Thr Leu Val Thr Val Ser Ser Gly Gly Gly Gly Ser Gly
        115                 120                 125
Gly Gly Gly Ser Gly Gly Gly Gly Ser Ser Ala Leu Glu Ile Val Leu
    130                 135                 140
Thr Gln Ser Pro Gly Thr Leu Ser Leu Ser Pro Gly Glu Ser Ala Thr
145                 150                 155                 160
Leu Ser Cys Arg Pro Ser Gln Ser Val Ser Ser Arg Asp Leu Ala Trp
                165                 170                 175
Tyr Gln Gln Lys Pro Gly Gln Ala Pro Arg Leu Leu Ile Tyr Gly Ala
            180                 185                 190
Ser Ser Arg Ala Thr Gly Ile Pro Asp Arg Phe Ser Gly Ser Gly Ser
        195                 200                 205
Gly Thr Glu Phe Thr Leu Thr Ile Thr Arg Leu Glu Pro Glu Asp Phe
    210                 215                 220
Ala Val Tyr Tyr Cys Gln Gln Tyr Gly Arg Ser Pro Leu Thr Phe Gly
225                 230                 235                 240
Gly Gly Thr Lys Val Glu Ile Lys Arg Ala Ala Ala His His His His
                245                 250                 255
His His Gly Glu Gln Lys Leu Ile Ser Glu Asp Leu
            260                 265

<210> 51
<211> 265
<212> PRT
<213> Homo Sapiens

<400> 51
Met Ala Glu Val Gln Leu Val Glu Ser Gly Gly Gly Val Val Gln Pro
1               5                   10                  15
Gly Arg Ser Leu Arg Leu Ser Cys Ala Ala Ser Gly Phe Ser Phe Ser
            20                  25                  30
Thr Tyr Asp Met His Trp Val Arg Arg Ala Pro Gly Lys Gly Leu Glu
        35                  40                  45
Trp Val Ala His Ile Arg Phe Asp Gly Ser Lys Thr Ser Tyr Ala Asp
    50                  55                  60
Ser Val Lys Gly Arg Phe Thr Ile Ser Arg Asp Asn Thr Lys Asn Thr
65                  70                  75                  80
Leu Phe Leu Gln Met Asn Ser Leu Arg Gly Glu Asp Thr Ala Ile Tyr
                85                  90                  95
Tyr Cys Ala Arg Val Arg Phe Ser Gly Tyr Asp Tyr Phe Glu Asn Trp
            100                 105                 110
Gly Lys Gly Thr Leu Val Thr Val Ser Ser Gly Gly Gly Gly Ser Gly
        115                 120                 125
Gly Gly Gly Ser Gly Gly Gly Gly Ser Val His Leu Lys Leu Thr Gln
    130                 135                 140
Ser Pro Ala Thr Leu Ser Val Ser Pro Gly Glu Ser Ala Thr Leu Ser
145                 150                 155                 160
Cys Arg Ala Ser Gln Ser Val Arg Ser Tyr Leu Ala Trp Tyr Gln Gln
                165                 170                 175
Lys Pro Gly Gln Ala Pro Arg Leu Leu Ile Tyr Gly Ala Ser Ser Arg
            180                 185                 190
Ala Thr Gly Ile Pro Asp Arg Phe Ser Gly Ser Gly Ser Gly Thr Asp

195                 200                 205
Phe Thr Leu Thr Ile Ser Arg Leu Glu Pro Glu Asp Phe Ala Val Tyr
    210                 215                 220
Tyr Cys Gln Gln Tyr Gly Ser Ser Pro Val Thr Phe Gly Gln Gly Thr
225                 230                 235                 240
Lys Leu Glu Ile Lys Arg Ala Ala Ala His His His His His His Gly
                245                 250                 255
Glu Gln Lys Leu Ile Ser Glu Asp Leu
            260                 265

<210> 52
<211> 267
<212> PRT
<213> Homo Sapiens

<400> 52
Met Ala Glu Val Gln Leu Val Glu Ser Gly Gly Gly Val Val Gln Ser
1               5                   10                  15
Gly Gly Ser Leu Arg Leu Ser Cys Ala Ala Ser Gly Phe Thr Phe Ser
            20                  25                  30
Gly Tyr Asp Met His Trp Val Arg Gln Ala Pro Gly Lys Gly Leu Glu
        35                  40                  45
Trp Val Ala Gly Leu Arg Tyr Asp Gly Thr Lys Arg Glu Tyr Ala Asp
    50                  55                  60
Ser Val Arg Gly Arg Phe Thr Ile Ser Arg Asp Asn Thr Lys Asn Thr
65                  70                  75                  80
Leu Tyr Leu Gln Met Asp Ser Leu Arg Gly Glu Asp Thr Ala Val Tyr
                85                  90                  95
Phe Cys Ala Arg Val Arg Phe Ser Gly Tyr Asn Tyr Phe Glu Asn Trp
            100                 105                 110
Gly Lys Gly Thr Thr Val Thr Val Ser Ser Gly Gly Gly Gly Ser Gly
        115                 120                 125
Gly Gly Gly Ser Gly Gly Gly Gly Ser Ser Ala Leu Glu Ile Val Met
    130                 135                 140
Thr Gln Ser Pro Gly Thr Leu Ser Leu Ser Pro Gly Glu Arg Ala Thr
145                 150                 155                 160
Leu Ser Cys Arg Ala Ser Gln Ser Val Ser Ser Arg Tyr Leu Ala Trp
                165                 170                 175
Tyr Gln Gln Lys Pro Gly Gln Ala Pro Arg Leu Leu Ile Tyr Gly Val
            180                 185                 190
Ser Ser Arg Ala Thr Gly Ile Pro Asp Arg Phe Ser Gly Ser Gly Ser
        195                 200                 205
Gly Thr Asp Phe Thr Leu Thr Ile Ser Arg Leu Glu Pro Glu Asp Phe
    210                 215                 220
Ala Val Tyr Tyr Cys Gln Gln Tyr Ala Ala Ser Pro Glu Thr Phe Gly
225                 230                 235                 240
Gln Gly Thr Lys Val Glu Ile Lys Arg Ala Ala Ala His His His His
                245                 250                 255
His His Gly Glu Gln Lys Leu Ile Ser Asn Leu
            260                 265

<210> 53
<211> 269
<212> PRT
<213> Homo Sapiens

<400> 53
Met Ala Glu Val Gln Leu Val Glu Ser Gly Gly Gly Leu Val Lys Pro
1               5                   10                  15
Gly Gly Ser Leu Arg Leu Ser Cys Ala Ala Ser Gly Phe Ser Phe Ser
            20                  25                  30
Thr Tyr Asp Met His Trp Val Arg Gln Ala Pro Gly Lys Gly Leu Glu
        35                  40                  45

Trp Val Ala Gly Leu Arg Tyr Asp Gly Ser Lys Lys Tyr Tyr Ala Asp
    50                  55                  60
Ser Val Lys Gly Arg Phe Thr Ile Ser Arg Asp Asn Thr Lys Asn Thr
65                  70                  75                  80
Leu Tyr Leu Gln Met Asp Ser Leu Arg Gly Glu Asp Thr Ala Val Tyr
                85                  90                  95
Tyr Cys Ala Arg Val Arg Phe Ser Gly Tyr Glu Tyr Phe Glu Asn Trp
            100                 105                 110
Gly Gln Gly Thr Leu Val Thr Val Ser Ser Gly Gly Gly Gly Ser Gly
        115                 120                 125
Gly Gly Gly Ser Gly Gly Gly Gly Ser Ser Ala Leu Asp Val Val Met
    130                 135                 140
Thr Gln Ser Pro Ser Thr Leu Ser Ala Ser Val Gly Asp Arg Val Thr
145                 150                 155                 160
Ile Thr Cys Arg Ala Ser Gln Ser Val Ser Thr Trp Leu Ala Trp Tyr
                165                 170                 175
Gln Gln Lys Pro Gly Lys Ala Pro Lys Leu Leu Ile Tyr Gln Ala Ser
            180                 185                 190
Asn Leu Glu Ser Gly Val Pro Ser Arg Phe Ser Gly Ser Gly Ser Asp
        195                 200                 205
Thr Glu Phe Thr Leu Thr Ile Asn Asn Leu Gln Pro Ala Asp Phe Ala
    210                 215                 220
Thr Tyr Tyr Cys Gln Gln Tyr Asn Thr Tyr Ser Ser Ala Thr Phe Gly
225                 230                 235                 240
Gln Gly Thr Lys Val Glu Ile Lys Arg Ala Ala Ala His His His His
                245                 250                 255
His His Gly Glu Gln Lys Leu Ile Ser Glu Glu Asp Leu
            260                 265


<210> 54
<211> 269
<212> PRT
<213> Homo Sapiens

<400> 54
Met Ala Glu Val Gln Leu Val Glu Ser Gly Gly Gly Val Val Gln Pro
 1               5                  10                  15
Gly Arg Ser Leu Arg Leu Ser Cys Ala Ala Ser Gly Phe Ser Phe Ser
            20                  25                  30
Thr Tyr Asp Met His Trp Val Arg Gln Ala Pro Gly Lys Gly Leu Glu
        35                  40                  45
Trp Val Ala His Ile Arg Phe Asp Gly Ser Lys Thr Ser Tyr Ala Asp
    50                  55                  60
Ser Val Lys Gly Arg Phe Thr Ile Ser Arg Asp Asn Thr Lys Asn Thr
65                  70                  75                  80
Leu Phe Leu Gln Met Asn Ser Leu Arg Gly Glu Asp Thr Ala Ile Tyr
                85                  90                  95
Tyr Cys Ala Arg Val Arg Phe Ser Gly Tyr Asp Tyr Phe Glu Asn Trp
            100                 105                 110
Gly Gln Gly Thr Leu Val Thr Val Ser Ser Gly Gly Gly Gly Ser Gly
        115                 120                 125
Gly Gly Gly Ser Gly Gly Gly Gly Ser Ser Ala Leu Glu Ile Val Leu
    130                 135                 140
Thr Gln Ser Pro Gly Thr Leu Ser Leu Ser Pro Gly Glu Arg Ala Thr
145                 150                 155                 160
Leu Ser Cys Arg Ala Ser Gln Ser Val Ser Ser Ser Tyr Leu Ala Trp
                165                 170                 175
Tyr Gln Gln Lys Pro Gly Gln Ala Pro Arg Leu Leu Ile Tyr Gly Ala
            180                 185                 190
Ser Ser Arg Ala Thr Gly Ile Pro Asp Arg Phe Ser Gly Ser Gly Ser
        195                 200                 205
Gly Thr Asp Phe Thr Leu Thr Ile Ser Arg Leu Glu Pro Glu Asp Phe
    210                 215                 220
Ala Val Tyr Tyr Cys Gln Gln Tyr Gly Ser Ser Pro Pro Tyr Thr Phe

225                 230                 235                 240
Gly Gln Gly Thr Lys Leu Glu Ile Lys Arg Ala Ala Ala His His His
                245                 250                 255
His His His Gly Glu Gln Lys Leu Ile Ser Glu Asp Leu
            260                 265

<210> 55
<211> 267
<212> PRT
<213> Homo Sapiens

<400> 55
Met Ala Glu Val Gln Leu Val Glu Ser Gly Gly Asn Val Val Gln Pro
 1               5                  10                  15
Gly Arg Ser Leu Arg Leu Ser Cys Ala Ala Ser Gly Leu Ser Phe Ser
            20                  25                  30
Thr Tyr Asp Met His Trp Val Arg Gln Ala Pro Gly Lys Gly Leu Glu
        35                  40                  45
Trp Val Ala Gly Val Arg Tyr Asp Gly Ser Lys Lys Tyr Tyr Thr Glu
    50                  55                  60
Ser Val Arg Gly Arg Phe Thr Ile Ser Arg Asp Asn Thr Lys Asn Thr
65                  70                  75                  80
Leu Phe Leu Gln Met Asp Ser Leu Arg Gly Asp Asp Thr Ala Val Tyr
                85                  90                  95
Tyr Cys Ala Arg Val Arg Phe Ser Gly Tyr Asp Tyr Phe Glu Asn Trp
            100                 105                 110
Gly Lys Gly Thr Leu Val Thr Val Ser Ser Gly Gly Gly Gly Ser Gly
        115                 120                 125
Gly Gly Gly Ser Gly Gly Gly Gly Ser Ser Ala Leu Glu Ile Val Met
    130                 135                 140
Thr Gln Ser Pro Ala Thr Leu Ser Val Ser Pro Gly Glu Arg Ala Thr
145                 150                 155                 160
Leu Ser Cys Arg Ala Ser Gln Ser Val Ser Ser Asn Leu Ala Trp Tyr
                165                 170                 175
Gln Gln Lys Pro Gly Gln Ala Pro Arg Leu Leu Ile Tyr Gly Ala Ser
            180                 185                 190
Thr Arg Ala Thr Gly Ile Pro Ala Arg Phe Ser Gly Ser Gly Ser Gly
        195                 200                 205
Thr Glu Phe Thr Leu Thr Ile Ser Gly Leu Gln Ser Glu Asp Phe Ala
    210                 215                 220
Val Tyr Tyr Cys Gln Gln Tyr Asp Asn Trp Pro Leu Thr Phe Gly Gly
225                 230                 235                 240
Gly Thr Lys Val Glu Ile Lys Arg Ala Ala Ala His His His His His
                245                 250                 255
His Gly Glu Gln Lys Leu Ile Ser Glu Asp Leu
            260                 265

<210> 56
<211> 268
<212> PRT
<213> Homo Sapiens

<400> 56
Met Ala Glu Val Gln Leu Val Glu Ser Gly Gly Gly Val Val Gln Pro
 1               5                  10                  15
Gly Arg Ser Leu Arg Leu Ser Cys Ala Ala Ser Gly Phe Ser Phe Ser
            20                  25                  30
Thr Tyr Asp Met His Trp Val Arg Gln Ala Pro Gly Lys Gly Leu Glu
        35                  40                  45
Trp Val Ala Gly Leu Arg Tyr Asp Gly Ser Lys Lys Tyr Tyr Ala Asp
    50                  55                  60
Ser Val Lys Gly Arg Phe Thr Ile Ser Arg Asp Asn Thr Lys Asn Thr
65                  70                  75                  80

Leu Tyr Leu Gln Met Asp Ser Leu Arg Gly Glu Asp Thr Ala Val Tyr
                85                  90                  95
Tyr Cys Ala Arg Val Arg Phe Ser Gly Tyr Glu Tyr Phe Glu Asn Trp
            100                 105                 110
Gly Gln Gly Thr Leu Val Thr Val Ser Ser Gly Gly Gly Gly Ser Gly
        115                 120                 125
Gly Gly Gly Ser Gly Gly Gly Gly Ser Ser Ala Leu Glu Ile Val Met
    130                 135                 140
Thr Gln Ser Pro Gly Thr Leu Ser Leu Ser Pro Gly Glu Arg Ala Thr
145                 150                 155                 160
Leu Ser Cys Arg Ala Ser Gln Ser Val Ser Ser Ser Tyr Leu Ala Trp
                165                 170                 175
Tyr Gln His Lys Pro Gly Gln Ala Pro Arg Leu Leu Ile Tyr Gly Ala
            180                 185                 190
Ser Asn Arg Ala Thr Gly Ile Pro Asp Arg Phe Ser Gly Ser Gly Ser
        195                 200                 205
Gly Thr Asp Phe Thr Leu Thr Ile Ser Arg Leu Glu Pro Glu Asp Phe
    210                 215                 220
Ala Val Tyr Tyr Cys Gln Gln Tyr Gly Ser Ser Pro Phe Thr Phe Gly
225                 230                 235                 240
Gly Gly Thr Lys Val Glu Ile Lys Arg Ala Ala Ala His His His His
                245                 250                 255
His His Gly Glu Gln Lys Leu Ile Ser Glu Asp Leu
            260                 265

<210> 57
<211> 268
<212> PRT
<213> Homo Sapiens

<400> 57
Met Ala Glu Val Gln Leu Val Glu Ser Gly Gly Gly Val Val Gln Pro
1               5                   10                  15
Gly Arg Ser Leu Arg Leu Ser Cys Ala Ala Ser Gly Phe Ser Phe Ser
            20                  25                  30
Thr Tyr Asp Met His Trp Val Arg Gln Ala Pro Gly Lys Gly Leu Glu
        35                  40                  45
Trp Val Ala Gly Leu Arg Tyr Asp Gly Ser Lys Lys Tyr Tyr Ala Asp
    50                  55                  60
Ser Val Lys Gly Arg Phe Thr Ile Ser Arg Asp Asn Thr Lys Asn Thr
65                  70                  75                  80
Leu Tyr Leu Gln Met Asp Ser Leu Arg Gly Glu Asp Thr Ala Val Tyr
                85                  90                  95
Tyr Cys Ala Arg Val Arg Phe Ser Gly Tyr Glu Tyr Phe Glu Asn Trp
            100                 105                 110
Gly Arg Gly Thr Leu Val Thr Val Ser Ser Gly Gly Gly Gly Ser Gly
        115                 120                 125
Gly Gly Gly Ser Gly Gly Gly Gly Ser Ser Ala Leu Glu Ile Val Leu
    130                 135                 140
Thr Gln Ser Pro Gly Thr Leu Ser Leu Ser Pro Gly Glu Arg Val Thr
145                 150                 155                 160
Leu Ser Cys Arg Ala Ser Gln Ser Val Ser Ser Thr Tyr Leu Ala Trp
                165                 170                 175
Tyr Gln Gln Lys Pro Gly Gln Ala Pro Arg Leu Leu Ile Tyr Gly Ala
            180                 185                 190
Ser Ser Arg Ala Thr Gly Ile Pro Asp Arg Phe Ser Gly Ser Gly Ser
        195                 200                 205
Gly Thr Asp Phe Thr Leu Thr Val Thr Arg Leu Glu Pro Glu Asp Phe
    210                 215                 220
Ala Val Tyr Tyr Cys Gln Gln Tyr Ala Ser Ser Pro Leu Thr Phe Gly
225                 230                 235                 240
Gly Gly Thr Lys Val Glu Ile Lys Arg Ala Ala Ala His His His His
                245                 250                 255
His His Gly Glu Gln Lys Leu Ile Ser Glu Asp Leu

260                 265


<210> 58
<211> 269
<212> PRT
<213> Homo Sapiens

<400> 58
Met Ala Glu Val Gln Leu Val Glu Thr Gly Gly Gly Val Val Gln Pro
1               5                   10                  15
Gly Arg Ser Leu Arg Leu Ser Cys Ala Ala Ser Gly Leu Ser Phe Ser
            20                  25                  30
Thr Tyr Asp Met His Trp Val Arg Gln Ala Pro Gly Lys Gly Leu Glu
        35                  40                  45
Trp Val Ala Gly Ile Arg Tyr Asp Gly Ser Lys Lys Tyr Tyr Ala Asp
    50                  55                  60
Ser Val Lys Gly Arg Phe Thr Ile Ser Arg Asp Asn Thr Arg Asn Thr
65                  70                  75                  80
Leu Tyr Leu Gln Met Asp Ser Leu Arg Gly Glu Asp Thr Ala Ile Tyr
                85                  90                  95
Tyr Cys Ala Arg Val Arg Phe Ser Gly Tyr Glu Tyr Phe Glu Asn Trp
            100                 105                 110
Gly Arg Gly Thr Leu Val Thr Val Ser Ser Gly Gly Gly Gly Ser Gly
        115                 120                 125
Gly Gly Gly Ser Gly Gly Gly Gly Ser Ser Ala Leu Glu Ile Val Met
    130                 135                 140
Thr Gln Ser Pro Ser Thr Leu Ser Leu Ser Pro Gly Glu Arg Ala Thr
145                 150                 155                 160
Leu Ser Cys Arg Ala Ser Gln Ser Val Ser Ser Ser Tyr Leu Ala Trp
                165                 170                 175
Tyr Gln Gln Lys Pro Gly Gln Ala Pro Arg Leu Leu Ile Tyr Gly Ala
            180                 185                 190
Ser Ser Arg Ala Thr Gly Ile Pro Asp Arg Phe Ser Gly Ser Gly Ser
        195                 200                 205
Gly Thr Asp Phe Thr Leu Thr Ile Ser Arg Leu Glu Pro Glu Asp Phe
    210                 215                 220
Ala Val Tyr Tyr Cys Gln Gln Tyr Gly Ser Ser Ser Tyr Thr Phe Gly
225                 230                 235                 240
Gln Gly Thr Lys Leu Glu Ile Lys Arg Ala Ala Ala His His His His
                245                 250                 255
His His Gly Glu Gln Lys Leu Ile Ser Glu Glu Asp Leu
            260                 265


<210> 59
<211> 269
<212> PRT
<213> Homo Sapiens

<400> 59
Met Ala Glu Val Gln Leu Val Glu Ser Gly Gly Gly Leu Val Asn Pro
1               5                   10                  15
Gly Gly Ser Leu Arg Leu Ser Cys Ala Ala Ser Gly Phe Ser Phe Ser
            20                  25                  30
Thr Tyr Asp Met His Trp Val Arg Gln Ala Pro Gly Lys Gly Leu Glu
        35                  40                  45
Trp Val Ala His Ile Arg Phe Asp Gly Ser Lys Thr Ser Tyr Ala Asp
    50                  55                  60
Ser Val Lys Gly Arg Phe Thr Ile Ser Arg Asp Asn Thr Lys Asn Thr
65                  70                  75                  80
Leu Phe Leu Gln Met Asn Ser Leu Arg Gly Glu Asp Thr Ala Ile Tyr
                85                  90                  95
Tyr Cys Ala Arg Val Arg Phe Ser Gly Tyr Asp Tyr Phe Glu Asn Trp
            100                 105                 110

Gly Lys Gly Thr Leu Val Thr Val Ser Ser Gly Gly Gly Gly Ser Gly
        115                 120                 125
Gly Gly Gly Ser Gly Gly Gly Gly Ser Ser Ala Leu Glu Ile Val Met
    130                 135                 140
Thr Gln Ser Pro Gly Thr Leu Ser Leu Ser Pro Gly Glu Arg Ala Thr
145                 150                 155                 160
Leu Ser Cys Arg Ala Ser Gln Ser Val Ser Ser Ser Tyr Leu Ala Trp
                165                 170                 175
Tyr Gln Gln Lys Pro Gly Gln Ala Pro Arg Leu Leu Ile Tyr Gly Ala
            180                 185                 190
Ser Ser Arg Ala Thr Gly Ile Pro Asp Arg Phe Ser Gly Ser Gly Ser
        195                 200                 205
Gly Thr Asp Phe Thr Leu Thr Ile Ser Arg Leu Glu Pro Glu Asp Phe
    210                 215                 220
Ala Val Tyr Tyr Cys Gln Gln Tyr Gly Ser Ser Pro Gly Thr Phe Gly
225                 230                 235                 240
Gln Gly Thr Lys Val Glu Ile Lys Arg Ala Ala Ala His His His His
                245                 250                 255
His His Gly Glu Gln Lys Leu Ile Ser Glu Glu Asp Leu
            260                 265

<210> 60
<211> 269
<212> PRT
<213> Homo Sapiens

<400> 60
Leu Ala Glu Val Gln Leu Val Glu Ser Gly Gly Gly Val Val Gln Pro
1               5                   10                  15
Gly Arg Ser Leu Arg Leu Ser Cys Ala Ala Ser Gly Phe Ser Phe Ser
            20                  25                  30
Thr Tyr Asp Met His Trp Val Arg Gln Ala Pro Gly Lys Gly Leu Glu
        35                  40                  45
Trp Val Ala His Ile Arg Phe Asp Gly Ser Lys Thr Ser Tyr Ala Asp
    50                  55                  60
Ser Val Lys Gly Arg Phe Thr Ile Ser Arg Asp Asn Thr Lys Asn Thr
65                  70                  75                  80
Leu Phe Leu Gln Met Asn Ser Leu Arg Ala Glu Asp Thr Ala Ile Tyr
                85                  90                  95
Tyr Cys Ala Arg Val Arg Phe Ser Gly Tyr Asp Tyr Phe Glu Asn Trp
            100                 105                 110
Gly Lys Gly Thr Leu Val Thr Val Ser Ser Gly Gly Gly Gly Ser Gly
        115                 120                 125
Gly Gly Gly Ser Gly Gly Gly Gly Ser Ser Ala Leu Glu Ile Val Leu
    130                 135                 140
Thr Gln Ser Pro Gly Thr Leu Ser Leu Ser Pro Gly Glu Arg Ala Thr
145                 150                 155                 160
Leu Ser Cys Arg Ala Ser Gln Ser Val Ser Ser Ser Tyr Leu Ala Trp
                165                 170                 175
Tyr Gln Gln Lys Pro Gly Gln Ala Pro Arg Leu Leu Ile Tyr Gly Ala
            180                 185                 190
Ser Ser Arg Ala Thr Gly Ile Pro Asp Arg Phe Ser Gly Ser Gly Ser
        195                 200                 205
Gly Thr Asp Phe Thr Leu Thr Ile Ser Arg Leu Glu Pro Glu Asp Phe
    210                 215                 220
Ala Val Tyr Tyr Cys Gln Gln Tyr Gly Ser Ser Leu Ala Leu Thr Phe
225                 230                 235                 240
Gly Gly Gly Thr Lys Val Glu Ile Lys Arg Ala Ala Ala His His His
                245                 250                 255
His His His Gly Glu Gln Lys Leu Ile Ser Glu Asp Leu
            260                 265

<210> 61

<211> 268
<212> PRT
<213> Homo Sapiens

<400> 61
Met Ala Gln Val Gln Leu Val Glu Ser Gly Gly Gly Val Val Gln Pro
1               5                   10                  15
Gly Arg Ser Leu Arg Leu Ser Cys Ala Ala Ser Gly Phe Ser Phe Ser
            20                  25                  30
Thr Tyr Asp Met His Trp Val Arg Gln Ala Pro Gly Lys Gly Leu Glu
        35                  40                  45
Trp Val Ala His Ile Arg Phe Asp Gly Ser Lys Thr Ser Tyr Ala Asp
    50                  55                  60
Ser Val Lys Gly Arg Phe Thr Ile Ser Arg Asp Asn Thr Lys Asn Thr
65                  70                  75                  80
Leu Phe Leu Gln Met Asn Ser Leu Arg Gly Glu Asp Thr Ala Ile Tyr
                85                  90                  95
Tyr Cys Ala Arg Val Arg Phe Ser Gly Tyr Asp Tyr Phe Glu Asn Trp
            100                 105                 110
Gly Lys Gly Thr Leu Val Thr Val Ser Ser Gly Gly Gly Gly Ser Gly
        115                 120                 125
Gly Gly Gly Ser Gly Gly Gly Gly Ser Ser Ala Leu Asp Ile Gln Leu
    130                 135                 140
Thr Gln Ser Pro Ser Ser Val Ser Ala Ser Val Gly Asp Arg Val Thr
145                 150                 155                 160
Ile Thr Cys Arg Ala Ser Gln Gly Ile Ser Ser Gly Leu Ala Trp Tyr
                165                 170                 175
Gln Gln Asn Pro Gly Lys Ala Pro Asn Leu Leu Ile Tyr Ala Ala Ser
            180                 185                 190
Asn Leu Gln Ser Gly Val Pro Ser Arg Phe Ser Gly Ser Gly Ser Gly
        195                 200                 205
Thr Asp Phe Thr Leu Thr Ile Ser Ser Leu Gln Pro Glu Asp Phe Ala
    210                 215                 220
Thr Tyr Tyr Cys Gln Gln Thr Asn Ser Phe Pro Leu Thr Phe Gly Gly
225                 230                 235                 240
Gly Thr Lys Val Glu Ile Lys Arg Ala Ala Ala His His His His His
                245                 250                 255
His Gly Glu Gln Lys Leu Ile Ser Glu Glu Asp Leu
            260                 265


<210> 62
<211> 268
<212> PRT
<213> Homo Sapiens

<400> 62
Met Ala Gln Val Gln Leu Val Glu Ser Gly Gly Gly Val Val Gln Pro
1               5                   10                  15
Gly Arg Ser Leu Arg Leu Ser Cys Ala Ala Ser Gly Phe Ser Phe Ser
            20                  25                  30
Thr Tyr Asp Met His Trp Val Arg Gln Ala Pro Gly Lys Gly Leu Glu
        35                  40                  45
Trp Val Ala His Ile Arg Phe Asp Gly Ser Lys Thr Ser Tyr Ala Asp
    50                  55                  60
Ser Val Lys Gly Arg Phe Thr Ile Ser Arg Asp Asn Thr Lys Asn Thr
65                  70                  75                  80
Leu Phe Leu Gln Met Asn Ser Leu Arg Gly Glu Asp Thr Ala Ile Tyr
                85                  90                  95
Tyr Cys Ala Arg Val Arg Phe Ser Gly Tyr Asp Tyr Phe Glu Asn Trp
            100                 105                 110
Gly Arg Gly Thr Leu Val Thr Val Ser Ser Gly Gly Gly Gly Ser Gly
        115                 120                 125
Gly Gly Gly Ser Gly Gly Gly Gly Ser Ser Ala Leu Glu Ile Val Met
    130                 135                 140

Thr Gln Ser Pro Gly Thr Leu Ser Leu Ser Pro Gly Glu Arg Ala Thr
145                 150                 155                 160
Leu Ser Cys Arg Ala Ser Gln Ser Val Ser Ser Arg Tyr Leu Ala Trp
                165                 170                 175
Tyr Gln Gln Lys Pro Gly Gln Ala Pro Arg Leu Leu Ile Tyr Gly Val
            180                 185                 190
Ser Ser Arg Ala Thr Gly Ile Pro Asp Arg Phe Ser Gly Ser Gly Ser
        195                 200                 205
Gly Thr Asp Phe Thr Leu Thr Ile Ser Arg Leu Glu Pro Glu Asp Phe
    210                 215                 220
Ala Val Tyr Tyr Cys Gln Gln Tyr Ala Ala Ser Pro Glu Thr Phe Gly
225                 230                 235                 240
Gln Gly Thr Lys Val Glu Ile Lys Arg Ala Ala Ala His His His His
                245                 250                 255
His His Gly Glu Gln Lys Leu Ile Ser Glu Asp Leu
            260                 265

<210> 63
<211> 268
<212> PRT
<213> Homo Sapiens

<400> 63
Met Ala Glu Val Gln Leu Val Glu Ser Gly Gly Gly Val Val Gln Pro
 1               5                  10                  15
Gly Arg Ser Leu Arg Leu Ser Cys Ala Ala Ser Gly Leu Ser Phe Ser
            20                  25                  30
Thr Tyr Asp Met His Trp Val Arg Gln Ala Pro Gly Lys Gly Leu Glu
        35                  40                  45
Trp Val Ala Gly Ile Arg Tyr Asp Gly Ser Lys Lys Tyr Tyr Ala Asp
    50                  55                  60
Ser Val Lys Gly Arg Phe Thr Ile Ser Arg Asp Asn Thr Arg Asn Thr
65                  70                  75                  80
Leu Tyr Leu Gln Met Asp Ser Leu Arg Gly Glu Asp Thr Ala Ile Tyr
                85                  90                  95
Tyr Cys Ala Arg Val Arg Phe Ser Gly Tyr Glu Tyr Phe Glu Asn Trp
            100                 105                 110
Gly Lys Gly Thr Met Val Thr Val Ser Ser Gly Gly Gly Gly Ser Gly
        115                 120                 125
Gly Gly Gly Ser Gly Gly Gly Gly Ser Ser Ala Leu Glu Ile Val Met
    130                 135                 140
Thr Gln Ser Pro Gly Thr Leu Ser Leu Ser Pro Gly Glu Arg Ala Thr
145                 150                 155                 160
Leu Ser Cys Arg Ala Ser Gln Ser Val Ser Ser Arg Tyr Leu Ala Trp
                165                 170                 175
Tyr Gln Gln Lys Pro Gly Gln Ala Pro Arg Leu Leu Ile Tyr Gly Val
            180                 185                 190
Ser Ser Arg Ala Thr Gly Ile Pro Asp Arg Phe Ser Gly Ser Gly Ser
        195                 200                 205
Gly Thr Asp Phe Thr Leu Thr Ile Ser Arg Leu Glu Pro Glu Asp Phe
    210                 215                 220
Ala Val Tyr Tyr Cys Gln Gln Tyr Ala Ala Ser Pro Glu Thr Phe Gly
225                 230                 235                 240
Gln Gly Thr Lys Val Glu Ile Lys Arg Ala Ala Ala His His His His
                245                 250                 255
His His Gly Glu Gln Lys Leu Ile Ser Glu Asp Leu
            260                 265

<210> 64
<211> 268
<212> PRT
<213> Homo Sapiens

<400> 64
Met Ala Gln Val Gln Leu Val Glu Ser Gly Gly Gly Leu Val Gln Pro
1               5                   10                  15
Gly Gly Ser Leu Arg Leu Ser Cys Ala Ala Ser Gly Leu Ser Phe Pro
            20                  25                  30
Thr Tyr Asp Met His Trp Val Arg Gln Ala Pro Gly Lys Gly Leu Glu
        35                  40                  45
Trp Val Ala His Ile Arg Phe Asp Gly Thr Lys Thr Ser Tyr Gly Asp
    50                  55                  60
Ala Val Arg Gly Arg Phe Thr Ile Ser Arg Asp Asn Thr Lys Asn Thr
65                  70                  75                  80
Leu Tyr Leu Gln Met Asn Arg Leu Arg Gly Glu Asp Thr Ala Ile Tyr
                85                  90                  95
Tyr Cys Ala Arg Val Arg Phe Ser Gly Tyr Asp Tyr Phe Glu Asn Trp
            100                 105                 110
Gly Arg Gly Thr Thr Val Thr Val Ser Ser Gly Gly Gly Gly Ser Gly
        115                 120                 125
Gly Gly Gly Ser Gly Gly Gly Gly Ser Ser Ala Leu Glu Ile Val Met
    130                 135                 140
Thr Gln Ser Pro Ala Thr Leu Ser Val Ser Pro Gly Glu Arg Ala Thr
145                 150                 155                 160
Leu Ser Cys Arg Ala Ser Gln Ser Val Ser Ser Asn Leu Ala Trp Tyr
                165                 170                 175
Gln Gln Lys Pro Gly Gln Ala Pro Arg Leu Leu Ile Tyr Gly Ala Ser
            180                 185                 190
Thr Arg Ala Thr Gly Ile Pro Ala Arg Phe Ser Gly Ser Gly Ser Gly
        195                 200                 205
Thr Glu Phe Thr Leu Thr Ile Ser Gly Leu Gln Ser Glu Asp Phe Ala
    210                 215                 220
Val Tyr Tyr Cys Gln Gln Tyr Asp Asn Trp Pro Leu Thr Phe Gly Gly
225                 230                 235                 240
Gly Thr Lys Val Glu Ile Lys Arg Ala Ala Ala His His His His His
                245                 250                 255
His Gly Glu Gln Lys Leu Ile Ser Glu Glu Asp Leu
            260                 265

<210> 65
<211> 270
<212> PRT
<213> Homo Sapiens

<400> 65
Met Ala Glu Val Gln Leu Val Glu Thr Gly Gly Gly Val Val Gln Pro
1               5                   10                  15
Gly Arg Ser Leu Arg Leu Ser Cys Ala Ala Ser Gly Phe Ser Phe Ser
            20                  25                  30
Thr Tyr Asp Met His Trp Val Arg Gln Ala Pro Gly Lys Gly Leu Glu
        35                  40                  45
Trp Val Ala Gly Leu Arg Tyr Asp Gly Ser Lys Lys Tyr Tyr Ala Asp
    50                  55                  60
Ser Val Lys Gly Arg Phe Thr Ile Ser Arg Asp Asn Thr Lys Asn Thr
65                  70                  75                  80
Leu Tyr Leu Gln Met Asp Ser Leu Arg Gly Glu Asp Thr Ala Val Tyr
                85                  90                  95
Tyr Cys Ala Arg Val Arg Phe Ser Gly Tyr Glu Tyr Phe Glu Asn Trp
            100                 105                 110
Gly Lys Gly Thr Leu Val Thr Val Ser Ser Gly Gly Gly Gly Ser Gly
        115                 120                 125
Gly Gly Gly Ser Gly Gly Gly Gly Ser Ser Ala Gln Ser Ala Leu Thr
    130                 135                 140
Gln Pro Arg Ser Val Ser Gly Ser Pro Gly Gln Ser Val Thr Ile Ser
145                 150                 155                 160
Cys Thr Gly Thr Ser Ser Asp Val Gly Gly Tyr Asn Tyr Val Ser Trp
                165                 170                 175

Tyr Gln Gln His Pro Gly Lys Ala Pro Lys Leu Met Ile Tyr Asp Val
            180                 185                 190
Ser Asn Arg Pro Ser Gly Val Ser Asn Arg Phe Ser Gly Ser Lys Ser
        195                 200                 205
Gly Asn Thr Ala Ser Leu Thr Ile Ser Gly Leu Gln Ala Glu Asp Glu
    210                 215                 220
Ala Asp Tyr Tyr Cys Ser Ser Tyr Thr Ser Ser Ser Thr Leu Val Ile
225                 230                 235                 240
Phe Gly Gly Arg Thr Lys Leu Thr Val Leu Gly Ala Ala Ala His His
                245                 250                 255
His His His His Gly Glu Gln Lys Leu Ile Ser Glu Asn Cys
            260                 265                 270

<210> 66
<211> 276
<212> PRT
<213> Homo Sapiens

<400> 66
Met Ala Glu Val Gln Leu Val Gln Ser Gly Ala Glu Val Lys Lys Pro
1               5                   10                  15
Gly Ser Ser Val Lys Val Ser Cys Lys Ala Ser Gly Val Thr Leu Ser
            20                  25                  30
Ile Tyr Ser Met Asn Trp Val Arg Gln Ala Pro Gly Gln Gly Leu Glu
        35                  40                  45
Trp Met Gly Arg Ile Ile Pro Ile Thr Gly Val Pro Asn Tyr Ser Gln
    50                  55                  60
Asn Phe Gln Gly Arg Val Thr Ile Thr Ala Asp Lys Ser Thr Ser Thr
65                  70                  75                  80
Thr Tyr Met Glu Leu Ser Ser Leu Arg Ser Glu Asp Thr Ala Val Tyr
                85                  90                  95
Tyr Cys Ala Leu Ser Gly Ala Gly Tyr Asn Tyr Tyr Gly Met Asp Val
            100                 105                 110
Trp Gly Gln Gly Thr Leu Val Thr Val Ser Ser Gly Gly Gly Gly Ser
        115                 120                 125
Gly Gly Gly Gly Ser Gly Gly Gly Ala Leu Glu Ile Val Leu Thr Gln
    130                 135                 140
Ser Pro Leu Ser Leu Pro Val Thr Pro Gly Glu Pro Ala Ser Ile Ser
145                 150                 155                 160
Cys Arg Ser Ser Gln Ser Leu Leu His Ser Asn Gly Tyr Asn Tyr Leu
                165                 170                 175
Asp Trp Tyr Leu Gln Lys Pro Gly Gln Ser Pro Gln Leu Leu Ile Tyr
            180                 185                 190
Leu Gly Ser Asn Arg Ala Ser Gly Val Pro Asp Arg Phe Ser Gly Ser
        195                 200                 205
Gly Ser Gly Thr Asp Phe Thr Leu Lys Ile Ser Arg Val Glu Ala Glu
    210                 215                 220
Asp Val Gly Val Tyr Tyr Cys Met Gln Ala Leu Gln Thr Pro Leu Thr
225                 230                 235                 240
Phe Gly Gly Gly Thr Lys Val Glu Ile Lys Arg Ala Ala Ala His His
                245                 250                 255
His His His His Gly Glu Gln Lys Leu Ile Ser Glu Asn Cys Lys Leu
            260                 265                 270
Leu Lys Val Val
        275

<210> 67
<211> 272
<212> PRT
<213> Homo Sapiens

<400> 67
Met Ala Glu Val Gln Leu Val Gln Ser Gly Ala Glu Val Lys Lys Pro

1               5                   10                  15
Gly Ser Ser Val Lys Val Ser Cys Lys Ala Ser Gly Val Thr Leu Ser
            20                  25                  30
Ile Tyr Ser Met Asn Trp Val Arg Gln Ala Pro Gly Gln Gly Leu Glu
        35                  40                  45
Trp Met Gly Arg Ile Ile Pro Ile Thr Gly Val Pro Asn Tyr Ser Gln
    50                  55                  60
Asn Phe Gln Gly Arg Val Thr Ile Thr Ala Asp Lys Ser Thr Ser Thr
65                  70                  75                  80
Thr Tyr Met Glu Leu Ser Ser Leu Arg Ser Glu Asp Thr Ala Val Tyr
                85                  90                  95
Tyr Cys Ala Leu Ser Gly Ala Gly Tyr Asn Tyr Tyr Gly Met Asp Val
            100                 105                 110
Trp Gly Lys Gly Thr Leu Val Thr Val Ser Ser Gly Gly Gly Gly Ser
        115                 120                 125
Gly Gly Gly Gly Ser Gly Gly Gly Gly Ser Ser Ala Leu Glu Ile Val
    130                 135                 140
Leu Thr Gln Ser Pro Leu Ser Leu Pro Val Thr Pro Gly Glu Pro Ala
145                 150                 155                 160
Ser Ile Ser Cys Arg Ser Ser Gln Ser Leu Leu His Ser Asn Gly Tyr
                165                 170                 175
Asn Tyr Leu Asp Trp Tyr Leu Gln Lys Pro Gly Gln Ser Pro His Leu
            180                 185                 190
Leu Ile Tyr Leu Gly Ser Asn Arg Ala Ser Gly Val Pro Asp Arg Phe
        195                 200                 205
Ser Gly Ser Gly Ser Gly Thr Asp Phe Thr Leu Lys Ile Ser Arg Val
    210                 215                 220
Glu Ala Glu Asp Val Gly Val Tyr Tyr Cys Met Gln Ala Leu Gln Thr
225                 230                 235                 240
Pro Arg Thr Phe Gly Pro Gly Thr Lys Val Glu Ile Lys Arg Ala Ala
                245                 250                 255
Ala His His His His His His Gly Glu Gln Lys Leu Ile Ser Asp Leu
            260                 265                 270

<210> 68
<211> 270
<212> PRT
<213> Homo Sapiens

<400> 68
Met Ala Glu Val Gln Leu Val Glu Ser Gly Gly Gly Leu Val Gln Pro
1               5                   10                  15
Gly Arg Ser Leu Arg Leu Ser Cys Gly Ala Ser Gly Phe Thr Leu Ser
            20                  25                  30
Thr Tyr Gly Met His Trp Val Arg Gln Ala Ala Gly Lys Gly Leu Glu
        35                  40                  45
Trp Val Ala Val Ser Ser Tyr Asp Gly Arg Asn Glu Tyr Tyr Ala Asp
    50                  55                  60
Ser Val Lys Gly Arg Phe Thr Ile Ser Arg Asp Asn Phe Lys Asp Thr
65                  70                  75                  80
Leu Tyr Leu Gln Met Asn Asn Leu Arg Ala Glu Asp Thr Ala Val Tyr
                85                  90                  95
Tyr Cys Ala Lys Glu Val Gly Met Arg Ser Tyr Asp Ser Tyr Gly Met
            100                 105                 110
Asp Val Trp Gly Gln Gly Thr Met Val Thr Val Ser Ser Gly Gly Gly
        115                 120                 125
Gly Ser Gly Gly Gly Gly Ser Gly Gly Gly Gly Ser Ser Ala Leu Asp
    130                 135                 140
Ile Gln Met Thr Gln Ser Pro Ser Ser Leu Ser Ala Ser Val Gly Asp
145                 150                 155                 160
Arg Val Thr Ile Thr Cys Arg Ala Ser Gln Ser Ile Ser Ser Tyr Leu
                165                 170                 175
Asn Trp Tyr Gln Gln Lys Pro Gly Lys Ala Pro Lys Leu Leu Ile Tyr
            180                 185                 190

Ala Ala Ser Ser Leu Gln Ser Gly Val Pro Ser Arg Phe Ser Gly Ser
        195                 200                 205
Gly Ser Gly Thr Asp Phe Thr Leu Thr Ile Ser Ser Leu Gln Pro Glu
    210                 215                 220
Asp Phe Ala Thr Tyr Tyr Cys Gln Gln Ser Tyr Ser Thr Pro Tyr Thr
225                 230                 235                 240
Phe Gly Gln Gly Thr Lys Val Glu Ile Lys Arg Ala Ala Ala His His
                245                 250                 255
His His His His Gly Glu Gln Lys Leu Ile Ser Glu Asp Leu
            260                 265                 270

<210> 69
<211> 264
<212> PRT
<213> Homo Sapiens

<400> 69
Met Ala Gln Val Gln Leu Gln Gln Ser Gly Gly Gly Leu Val Gln Pro
 1               5                  10                  15
Gly Gly Ser Leu Arg Leu Ser Cys Thr Ala Ser Gly Phe Thr Phe Ser
            20                  25                  30
Asn Tyr His Met Asn Trp Val Arg Gln Ala Pro Gly Lys Gly Leu Glu
        35                  40                  45
Trp Val Ser His Ile Ser Ser Ser Ser Arg Thr Ile Lys Tyr Ala Asp
    50                  55                  60
Ser Val Lys Gly Arg Phe Thr Ile Ser Arg Asp Asn Ala Lys Asn Ser
65                  70                  75                  80
Leu Tyr Leu Gln Met Asn Ser Leu Arg Ala Glu Asp Thr Ala Val Tyr
                85                  90                  95
Tyr Cys Ala Arg Ala Gly Ser Gly Tyr Ser Ser Gly Pro Thr Asp Tyr
            100                 105                 110
Trp Gly Lys Gly Thr Met Val Thr Val Ser Ser Gly Gly Gly Gly Ser
        115                 120                 125
Gly Gly Gly Gly Ser Gly Gly Gly Gly Ser Ser Ala Gln Ser Val Leu
    130                 135                 140
Thr Gln Leu Pro Ser Val Ser Gly Ala Pro Gly Gln Arg Val Thr Ile
145                 150                 155                 160
Ser Cys Thr Gly Ser Ser Ser Asn Ile Gly Ala Gly Tyr Asp Val His
                165                 170                 175
Trp Tyr Gln Gln Leu Pro Gly Thr Ala Pro Lys Leu Leu Ile Tyr Gly
            180                 185                 190
Asn Ser Asn Arg Pro Ser Gly Val Pro Asp Arg Phe Ser Gly Ser Lys
        195                 200                 205
Ser Gly Thr Ser Ala Ser Leu Ala Ile Thr Gly Leu Gln Ala Asp Ala
    210                 215                 220
Asp Tyr Tyr Cys Gln Ser Tyr Asp Thr Asn Leu Arg Ala Tyr Val Phe
225                 230                 235                 240
Gly Thr Gly Thr Lys Leu Thr Val Leu Ala Ala Ala His His His His
                245                 250                 255
His His Gly Lys Gln Asn Ser Gln
            260

<210> 70
<211> 275
<212> PRT
<213> Homo Sapiens

<400> 70
Met Ala Glu Val Gln Leu Val Glu Ser Gly Gly Gly Val Val Gln Pro
 1               5                  10                  15
Gly Arg Ser Leu Arg Leu Ser Cys Thr Ala Ser Gly Phe Ser Ser Ser
            20                  25                  30
Val Tyr Asp Met His Trp Val Arg Gln Ala Pro Gly Gln Gly Leu Glu

35                  40                  45
Trp Val Ala Leu Ile Ser His Asp Gly Asn His Lys His Tyr Ala Asp
    50                  55                  60
Ser Val Lys Gly Arg Phe Thr Ile Ser Arg Asp Asn Ser Lys Asn Ala
65                  70                  75                  80
Leu Tyr Leu Gln Met Asp Ser Leu Arg Gly Glu Asp Thr Ala Val Tyr
                85                  90                  95
Tyr Cys Ala Arg Asp Arg Phe Gly Arg Ser Gly Ile Lys Leu Lys Val
            100                 105                 110
Thr Tyr Leu Asp Tyr Trp Gly Glu Gly Thr Thr Val Thr Val Ser Ser
        115                 120                 125
Gly Gly Gly Gly Ser Gly Gly Gly Gly Ser Gly Gly Gly Gly Ser Ser
    130                 135                 140
Ala Leu Glu Ile Val Leu Thr Gln Ser Pro Ala Ser Leu Ser Leu Ser
145                 150                 155                 160
Pro Gly Glu Arg Ala Thr Leu Ser Cys Arg Ala Ser Glu Ser Ile His
                165                 170                 175
Arg Tyr Leu Ala Trp Tyr Gln Gln Lys Pro Gly Gln Ala Pro Arg Leu
            180                 185                 190
Leu Ile Tyr Asp Thr Ser Asn Arg Ala Thr Gly Ile Pro Ala Arg Phe
        195                 200                 205
Ser Gly Ser Gly Ser Gly Thr Asp Phe Thr Leu Thr Ile Ser Ser Leu
    210                 215                 220
Glu Pro Glu Asp Phe Ala Val Tyr Tyr Cys Gln Gln Tyr Asn Ser Trp
225                 230                 235                 240
Pro Pro Ile Thr Phe Gly Gln Gly Thr Arg Leu Glu Ile Lys Arg Ala
                245                 250                 255
Ala Ala His His His His His His Gly Glu Gln Lys Leu Ile Ser Glu
            260                 265                 270
Glu Asp Leu
        275

<210> 71
<211> 268
<212> PRT
<213> Homo Sapiens

<400> 71
Met Ala Gln Val Gln Leu Val Glu Ser Gly Gly Gly Val Val Gln Pro
 1               5                  10                  15
Gly Lys Ser Leu Arg Leu Ser Cys Ala Ala Ser Gly Phe Asp Phe Ser
            20                  25                  30
Gly Tyr Gly Met His Trp Val Arg Gln Ala Pro Gly Lys Gly Leu Glu
        35                  40                  45
Trp Val Ala Phe Ile Ser Tyr Asp Ala Ser Asn Gln Tyr Tyr Ala Asp
    50                  55                  60
Ser Val Lys Gly Arg Phe Thr Val Ser Arg Asp Asn Ser Lys Asn Thr
65                  70                  75                  80
Val Ser Leu Gln Met Ser Ser Leu Lys Thr Asp Asp Thr Ala Val Tyr
                85                  90                  95
Tyr Cys Ala Lys Asp Phe Ser Trp Ser Gly Ser Ile Asp Ser Trp Gly
            100                 105                 110
Gln Gly Thr Leu Val Thr Val Ser Ser Gly Gly Gly Gly Ser Gly Gly
        115                 120                 125
Gly Gly Ser Gly Gly Gly Gly Ser Ser Ala Leu Asp Val Val Met Thr
    130                 135                 140
Gln Ser Pro Gly Thr Leu Ser Leu Ser Pro Gly Glu Arg Ala Thr Leu
145                 150                 155                 160
Ser Cys Arg Ala Ser Gln Ser Val Ser Ser Ser Tyr Leu Ala Trp Tyr
                165                 170                 175
Gln Gln Lys Pro Gly Gln Ala Pro Arg Leu Leu Ile Tyr Gly Ala Ser
            180                 185                 190
Ser Arg Ala Thr Gly Ile Pro Asp Arg Phe Ser Gly Ser Gly Ser Gly
        195                 200                 205

Thr Asp Phe Thr Leu Thr Ile Ser Arg Leu Glu Pro Glu Asp Phe Ala
    210                 215                 220
Val Tyr Tyr Cys Gln Gln Tyr Gly Ser Ser Leu Ser Leu Thr Phe Gly
225                 230                 235                 240
Gly Gly Thr Lys Val Glu Ile Lys Arg Ala Ala Ala His His His His
                245                 250                 255
His His Gly Glu Gln Lys Leu Ile Ser Glu Glu Leu
            260                 265

<210> 72
<211> 273
<212> PRT
<213> Homo Sapiens

<400> 72
Met Ala Glu Val Gln Leu Val Glu Ser Gly Gly Gly Val Val Gln Pro
 1               5                  10                  15
Gly Arg Ser Leu Arg Leu Ser Cys Thr Ala Ser Gly Phe Ser Ser Ser
            20                  25                  30
Val Tyr Asp Met His Trp Val Arg Gln Ala Pro Gly Gln Gly Leu Glu
        35                  40                  45
Trp Val Ala Leu Ile Ser His Asp Gly Asn His Lys His Tyr Ala Asp
    50                  55                  60
Ser Val Lys Gly Arg Phe Thr Ile Ser Arg Asp Asn Ser Lys Asn Ala
65                  70                  75                  80
Leu Tyr Leu Gln Met Asp Ser Leu Arg Gly Glu Asp Thr Ala Val Tyr
                85                  90                  95
Tyr Cys Ala Arg Asp Arg Phe Gly Arg Ser Gly Ile Lys Leu Lys Val
            100                 105                 110
Thr Tyr Leu Asp Tyr Trp Gly Lys Gly Thr Leu Val Thr Val Ser Ser
        115                 120                 125
Gly Gly Gly Gly Ser Gly Gly Gly Gly Ser Gly Gly Gly Gly Ser Ser
    130                 135                 140
Ala Leu Asp Ile Gln Met Thr Gln Ser Pro Ser Ser Leu Ser Ala Ser
145                 150                 155                 160
Val Gly Asp Arg Val Thr Ile Thr Cys Arg Ala Ser Gln Asn Ile Ser
                165                 170                 175
Ser Phe Leu Asn Trp Tyr Gln Gln Lys Pro Gly Lys Ala Pro Lys Leu
            180                 185                 190
Leu Ile Tyr Ala Thr Ser Arg Leu Gln Ser Gly Val Pro Ser Arg Phe
        195                 200                 205
Ser Gly Ser Gly Ser Gly Thr Asp Phe Thr Leu Thr Ile Ser Ser Leu
    210                 215                 220
Gln Pro Glu Asp Phe Ala Thr Tyr Tyr Cys Gln Gln Ser Tyr Asn Thr
225                 230                 235                 240
Pro Leu Thr Phe Gly Gly Gly Thr Lys Val Asp Ile Lys Arg Ala Ala
                245                 250                 255
Ala His His His His His His Gly Glu Gln Lys Leu Ile Ser Glu Asp
            260                 265                 270
Leu

<210> 73
<211> 273
<212> PRT
<213> Homo Sapiens

<400> 73
Met Ala Gln Val Gln Leu Val Glu Ser Gly Gly Gly Val Val Gln Pro
 1               5                  10                  15
Gly Arg Ser Leu Arg Leu Ser Cys Ala Ala Ser Gly Phe Thr Ser Ser
            20                  25                  30
Pro Tyr Asp Met His Trp Val Arg Gln Ala Pro Gly Gln Gly Leu Glu

        35                  40                  45
Trp Val Ala Leu Ile Ser His Asp Gly Ser Tyr Lys His Tyr Thr Asp
    50                  55                  60
Ser Val Lys Gly Arg Phe Thr Ile Ser Arg Asp Asn Ser Lys Asn Ala
65                  70                  75                  80
Leu Tyr Leu Gln Met Asn Ser Leu Arg Ala Glu Asp Thr Ala Val Tyr
                85                  90                  95
Tyr Cys Ala Arg Asp Arg Phe Gly Arg Ser Gly Ile Lys Leu Lys Val
            100                 105                 110
Thr Tyr Leu Asp Tyr Trp Gly Lys Gly Thr Leu Val Thr Val Ser Ser
        115                 120                 125
Gly Gly Gly Gly Ser Gly Gly Gly Gly Ser Gly Gly Gly Gly Ser Ser
    130                 135                 140
Ala Leu Glu Ile Val Leu Thr Gln Ser Pro Gly Thr Leu Ser Leu Ser
145                 150                 155                 160
Pro Gly Glu Arg Ala Thr Leu Ser Cys Arg Ala Ser Glu Ser Val Asp
                165                 170                 175
Asn Thr Phe Leu Ala Trp Tyr Gln Gln Lys Pro Gly Gln Ala Pro Arg
            180                 185                 190
Leu Leu Ile Tyr Gly Ala Ser Ser Arg Ala Thr Gly Ile Pro Asp Arg
        195                 200                 205
Phe Ser Gly Gly Gly Ser Gly Thr Asp Phe Thr Leu Thr Ile Ser Arg
    210                 215                 220
Leu Glu Pro Glu Asp Ser Ala Val Tyr Tyr Cys Gln Gln Tyr Gly Asn
225                 230                 235                 240
Ser Leu Asn Phe Gly Gly Gly Thr Lys Leu Glu Ile Lys Arg Ala Ala
                245                 250                 255
Ala His His His His His His Gly Glu Gln Lys Leu Ile Ser Glu Asp
            260                 265                 270
Leu

<210> 74
<211> 273
<212> PRT
<213> Homo Sapiens

<400> 74
Met Ala Glu Val Gln Leu Val Glu Ser Gly Gly Gly Val Val Gln Pro
1               5                   10                  15
Gly Arg Ser Leu Arg Leu Ser Cys Thr Ala Ser Gly Phe Ser Ser Ser
            20                  25                  30
Val Tyr Asp Met His Trp Val Arg Gln Ala Pro Gly Gln Gly Leu Glu
        35                  40                  45
Trp Val Ala Leu Ile Ser His Asp Gly Asn His Lys His Tyr Ala Asp
    50                  55                  60
Ser Val Lys Gly Arg Phe Thr Ile Ser Arg Asp Asn Ser Lys Asn Ala
65                  70                  75                  80
Leu Tyr Leu Gln Met Asp Ser Leu Arg Gly Glu Asp Thr Ala Val Tyr
                85                  90                  95
Tyr Cys Ala Arg Asp Arg Phe Gly Arg Ser Gly Ile Lys Leu Lys Val
            100                 105                 110
Thr Tyr Leu Asp Tyr Trp Gly Arg Gly Thr Leu Val Thr Val Ser Ser
        115                 120                 125
Gly Gly Gly Gly Ser Gly Gly Gly Gly Ser Gly Gly Gly Gly Ser Ser
    130                 135                 140
Ala Leu Asp Ile Gln Leu Thr Gln Ser Pro Ser Thr Leu Ser Ala Ser
145                 150                 155                 160
Val Gly Asp Arg Val Thr Ile Thr Cys Arg Ala Ser Gln Ser Ile Thr
                165                 170                 175
Asn Leu Leu Ala Trp Tyr Gln Gln Lys Pro Gly Lys Ala Pro Asn Leu
            180                 185                 190
Leu Ile Tyr Lys Thr Ser Thr Leu Gln Ser Gly Val Pro Ser Arg Phe
        195                 200                 205

Ser Gly Ser Gly Ser Gly Thr Glu Phe Thr Leu Thr Ile Ser Ser Leu
    210                 215                 220
Gln Pro Asp Asp Phe Ala Thr Tyr Tyr Cys Gln Gln Tyr His Arg Phe
225                 230                 235                 240
Ser Tyr Ser Phe Gly Gln Gly Thr Lys Val Glu Ile Lys Arg Ala Ala
                245                 250                 255
Ala His His His His His His Gly Glu Gln Lys Leu Ile Ser Glu Asn
            260                 265                 270
Leu

<210> 75
<211> 273
<212> PRT
<213> Homo Sapiens

<400> 75
Met Ala Glu Val Gln Leu Val Glu Ser Gly Gly Gly Val Val Gln Pro
 1               5                  10                  15
Gly Arg Ser Leu Arg Leu Ser Cys Ala Ala Ser Gly Phe Ser Ser Ser
            20                  25                  30
Val Tyr Asp Met His Trp Val Arg Gln Ala Pro Gly Gln Gly Leu Glu
        35                  40                  45
Trp Val Ala Leu Ile Ser His Asp Gly Asn His Lys His Tyr Thr Asp
    50                  55                  60
Ser Val Lys Gly Arg Phe Thr Ile Ser Arg Asp Asn Ser Lys Asn Ala
65                  70                  75                  80
Leu Tyr Leu Gln Met Asn Ser Leu Arg Gly Glu Asp Thr Ala Val Tyr
                85                  90                  95
Tyr Cys Ala Arg Asp Arg Phe Gly Arg Ser Gly Met Lys Leu Lys Val
            100                 105                 110
Thr Tyr Leu Asp Tyr Trp Gly Lys Gly Thr Leu Val Thr Val Ser Ser
        115                 120                 125
Gly Gly Gly Gly Ser Gly Gly Gly Gly Ser Gly Gly Gly Gly Ser Ser
    130                 135                 140
Ala Leu Asp Ile Gln Met Thr Gln Ser Pro Ser Ser Leu Ser Ala Ser
145                 150                 155                 160
Val Gly Asp Arg Val Thr Leu Thr Cys Arg Ala Ser Gln Ser Ile Asn
                165                 170                 175
Ala Tyr Leu Asn Trp Tyr Gln Gln Lys Pro Gly Lys Ala Pro Lys Leu
            180                 185                 190
Leu Ile Tyr Thr Ala Ser Ser Leu Gln Ser Gly Val Pro Ser Arg Phe
        195                 200                 205
Ser Gly Ser Gly Ser Gly Thr Asp Phe Thr Leu Thr Ile Ser Arg Leu
    210                 215                 220
Gln Pro Glu Asp Phe Ala Thr Tyr Tyr Cys Gln Gln Thr Tyr Ser Ser
225                 230                 235                 240
Pro Leu Thr Phe Gly Gly Gly Thr Lys Val Glu Ile Lys Arg Ala Ala
                245                 250                 255
Ala His His His His His His Gly Glu Gln Lys Leu Ile Ser Glu Asp
            260                 265                 270
Leu

<210> 76
<211> 276
<212> PRT
<213> Homo Sapiens

<400> 76
Met Ala Glu Val Gln Leu Val Glu Thr Gly Gly Gly Val Val Gln Pro
 1               5                  10                  15
Gly Arg Ser Leu Arg Leu Ser Cys Thr Ala Ser Gly Phe Ser Ser Ser

                    20                  25                  30
Val Tyr Asp Met His Trp Val Arg Gln Ala Pro Gly Gln Gly Leu Glu
        35                  40                  45
Trp Val Ala Leu Ile Ser His Asp Gly Asn His Lys His Tyr Ala Asp
    50                  55                  60
Ser Val Lys Gly Arg Phe Thr Ile Ser Arg Asp Asn Ser Lys Asn Ala
65                  70                  75                  80
Leu Tyr Leu Gln Met Asp Ser Leu Arg Gly Glu Asp Thr Ala Val Tyr
                85                  90                  95
Tyr Cys Ala Arg Asp Arg Phe Gly Arg Ser Gly Ile Lys Leu Lys Val
            100                 105                 110
Thr Tyr Leu Asp Tyr Trp Gly Arg Gly Thr Thr Val Thr Val Ser Ser
        115                 120                 125
Gly Gly Gly Gly Ser Gly Gly Gly Gly Ser Gly Gly Gly Gly Ser Ser
    130                 135                 140
Ala Gln Ser Val Leu Thr Gln Pro Pro Ser Val Ser Ala Ala Pro Gly
145                 150                 155                 160
Gln Lys Val Thr Ile Ser Cys Ser Gly Ser Ser Ser Asn Ile Gly Lys
                165                 170                 175
Asn Tyr Val Ser Trp Tyr Gln Gln Val Pro Gly Thr Ala Pro Lys Leu
            180                 185                 190
Leu Ile Tyr Asp Asn Asn Lys Arg Pro Ser Gly Ile Pro Asp Arg Phe
        195                 200                 205
Ser Gly Ser Lys Ser Gly Thr Ser Ala Thr Leu Gly Ile Thr Gly Leu
    210                 215                 220
Gln Thr Gly Asp Glu Ala Asp Tyr His Cys Gly Thr Trp Asp Ser Ser
225                 230                 235                 240
Leu His Ser Gly Leu Phe Gly Gly Gly Thr Lys Leu Thr Val Leu Gly
                245                 250                 255
Ala Ala Ala His His His His His His Gly Glu Gln Lys Leu Ile Ser
            260                 265                 270
Glu Glu Asp Leu
        275

<210> 77
<211> 268
<212> PRT
<213> Homo Sapiens

<400> 77
Met Ala Glu Val Gln Leu Val Glu Ser Gly Gly Asp Leu Val Arg Pro
 1               5                  10                  15
Gly Gly Ser Leu Arg Leu Ser Cys Thr Ile Ser Gly Val Thr Phe Asn
            20                  25                  30
Gln Tyr Ala Ile Thr Trp Val Arg Gln Ala Pro Gly Lys Gly Leu Gln
        35                  40                  45
Trp Leu Ser Thr Ile Ala Gly Thr Gly Thr Lys Thr Phe Tyr Ala Asp
    50                  55                  60
Ser Val Lys Gly Arg Phe Thr Met Ser Arg Asp Ser Ser Gly Asn Thr
65                  70                  75                  80
Leu Tyr Leu Gln Met Asn Ser Leu Arg Asp Glu Asp Thr Ala Val Tyr
                85                  90                  95
Tyr Cys Ala Lys Ser Leu Ser Met Arg Tyr Phe Leu Asp Leu Trp Gly
            100                 105                 110
Arg Gly Thr Leu Val Thr Val Ser Ser Gly Gly Gly Gly Ser Gly Gly
        115                 120                 125
Gly Gly Ser Gly Gly Gly Gly Ser Ser Ala His Val Ile Leu Thr Gln
    130                 135                 140
Pro Pro Ser Ala Ser Gly Thr Pro Gly Gln Arg Val Ile Ile Ser Cys
145                 150                 155                 160
Ser Gly Ser Ser Ser Asn Ile Gly Ser Asn Thr Val Asn Trp Tyr Gln
                165                 170                 175
Gln Leu Pro Gly Thr Ala Pro Lys Leu Leu Ile Tyr Asn Asn Asn Gln
            180                 185                 190

Arg Pro Ser Gly Val Pro Asp Arg Phe Ser Gly Ser Lys Ser Gly Thr
        195                 200                 205
Ser Ala Ser Leu Ala Ile Ser Gly Leu Gln Ser Glu Asp Glu Ala Asp
    210                 215                 220
Tyr Tyr Cys Ala Ala Trp Asp Asp Ser Leu Asn Gly Trp Val Phe Gly
225                 230                 235                 240
Gly Gly Thr Lys Val Thr Val Leu Ala Ala Ala His His His His His
                245                 250                 255
His Gly Glu Gln Lys Leu Ile Ser Glu Glu Asp Leu
            260                 265

<210> 78
<211> 271
<212> PRT
<213> Homo Sapiens

<400> 78
Met Ala Gln Val Gln Leu Gln Glu Ser Gly Gly Asp Leu Val Lys Pro
 1               5                  10                  15
Gly Gly Ser Leu Arg Leu Ser Cys Val Ala Ser Gly Leu Thr Phe Asn
            20                  25                  30
Ser Tyr Gly Met Ser Trp Val Arg Gln Ala Pro Gly Lys Gly Leu Glu
        35                  40                  45
Trp Val Ser Asp Ile Ser Ala Ser Gly Phe Asn Thr Tyr Tyr Val Asp
    50                  55                  60
Ser Leu Lys Gly Arg Phe Thr Ile Ser Arg Asp Asn Ser Arg Asn Thr
65                  70                  75                  80
Leu Phe Leu Gln Met Asn Asn Leu Arg Asp Glu Asp Thr Ala Ile Tyr
                85                  90                  95
Tyr Cys Ala Lys Asn Gly Gly Asp Tyr Met Gly Ala Tyr Ile Asp Asn
            100                 105                 110
Trp Gly Lys Gly Thr Leu Val Thr Val Ser Ser Gly Gly Gly Gly Ser
        115                 120                 125
Gly Gly Gly Gly Ser Gly Gly Gly Gly Ser Ser Ala Gln Ser Val Leu
    130                 135                 140
Thr Gln Pro Pro Ser Val Ser Ala Ala Pro Gly Gln Lys Val Thr Ile
145                 150                 155                 160
Ser Cys Ser Gly Ser Ser Ser Asn Ile Gly Asn Asn Tyr Val Ser Trp
                165                 170                 175
Tyr Gln Gln Leu Pro Gly Thr Ala Pro Lys Leu Leu Ile Tyr Asp Asn
            180                 185                 190
Asn Lys Arg Pro Ser Gly Ile Pro Asp Arg Phe Ser Gly Ser Lys Ser
        195                 200                 205
Gly Thr Ser Ala Thr Leu Gly Ile Thr Gly Leu Gln Thr Gly Asp Glu
    210                 215                 220
Ala Asp Tyr Tyr Cys Gly Thr Trp Asp Ser Ser Leu Ser Ala Gly Val
225                 230                 235                 240
Phe Gly Gly Gly Thr Gln Leu Thr Val Leu Gly Ala Ala Ala His His
                245                 250                 255
His His His His Gly Glu Gln Lys Leu Ile Ser Glu Glu Asp Leu
            260                 265                 270

<210> 79
<211> 269
<212> PRT
<213> Homo Sapiens

<400> 79
Met Ala Gln Val Gln Leu Val Gln Ser Gly Ala Glu Val Lys Lys Pro
 1               5                  10                  15
Gly Ser Ser Val Lys Val Ser Cys Arg Ala Ser Gly Gly Thr Phe Arg
            20                  25                  30
Ser Tyr Ser Phe Asn Trp Leu Arg Gln Ala Pro Gly Gln Gly Leu Glu

        35                  40                  45
Trp Met Gly Arg Ile Ile Pro Val Val Gly Val Leu Asp Tyr Ala Pro
    50                  55                  60
Lys Phe Gln Ala Arg Val Thr Phe Thr Val Asp Thr Ser Thr Ser Val
65                  70                  75                  80
Gly Tyr Met Asp Leu Asn Gly Leu Thr Pro Glu Asp Thr Ala Val Tyr
                85                  90                  95
Tyr Cys Ala Gly Gly Asp His Val Val Lys Ala Ala Leu Ala Tyr Trp
            100                 105                 110
Gly Gly Gly Thr Thr Val Thr Val Ser Ser Gly Gly Gly Gly Ser Gly
        115                 120                 125
Gly Gly Gly Ser Gly Gly Gly Gly Ser Ser Ala Gln Ser Ala Leu Thr
    130                 135                 140
Gln Pro Ala Ser Glu Ser Gly Ser Pro Gly Gln Ser Ile Thr Ile Ser
145                 150                 155                 160
Cys Thr Gly Thr Ser Thr Asp Val Gly Ala Arg Asn Ser Val Ser Trp
                165                 170                 175
Tyr Gln Gln His Pro Gly Lys Ala Pro Lys Leu Ile Leu Tyr Asp Val
            180                 185                 190
Ser His Arg Pro Ser Gly Val Ser Asn Arg Phe Ser Gly Ser Lys Ser
        195                 200                 205
Gly Asn Thr Ala Ser Leu Thr Ile Ser Gly Leu Gln Ala Glu Asp Glu
    210                 215                 220
Gly Asp Phe Tyr Cys Ser Ser Tyr Thr Thr Ser Asn Asn Leu Val Phe
225                 230                 235                 240
Gly Gly Gly Thr Lys Leu Thr Val Leu Gly Ala Ala Ala His His His
                245                 250                 255
His His His Gly Glu Gln Lys Leu Ile Ser Glu Asp Leu
            260                 265

<210> 80
<211> 269
<212> PRT
<213> Homo Sapiens

<400> 80
Met Ala Gln Val Gln Leu Gln Gln Ser Gly Pro Gly Leu Val Lys Pro
1               5                   10                  15
Ser Glu Thr Leu Ser Leu Thr Cys Thr Leu Ser Gly Gly Ser Met Glu
            20                  25                  30
Ser His Tyr Trp Ser Trp Ile Arg Gln Pro Pro Gly Lys Gly Leu Glu
        35                  40                  45
Trp Met Gly Arg Val Ser Tyr Ile Gly Ile Ser Asn Tyr Asn Pro Tyr
    50                  55                  60
Leu Lys Asn Arg Val Thr Ile Ser Gln Asp Lys Ser Lys Asn Gln Leu
65                  70                  75                  80
Ser Leu Arg Leu Asn Ser Val Thr Ala Ala Asp Thr Ala Val Tyr Tyr
                85                  90                  95
Cys Ala Arg His Arg Leu Arg Ser Asp Gln Ala Phe Asp Leu Trp Gly
            100                 105                 110
Lys Gly Thr Leu Val Thr Val Ser Ser Gly Gly Gly Gly Ser Gly Gly
        115                 120                 125
Gly Gly Ser Gly Gly Gly Gly Ser Ser Ala Gln Ser Val Leu Thr Gln
    130                 135                 140
Pro Pro Ser Val Ser Gly Ala Pro Gly Gln Arg Val Thr Ile Ser Cys
145                 150                 155                 160
Thr Gly Ser Ser Ser Asn Ile Gly Ala Gly Tyr Asp Val His Trp Tyr
                165                 170                 175
Gln Gln Leu Pro Gly Thr Ala Pro Lys Leu Leu Ile Tyr Gly Asn Ser
            180                 185                 190
Asn Arg Pro Ser Gly Val Pro Asp Arg Phe Ser Gly Ser Lys Ser Gly
        195                 200                 205
Thr Ser Ala Ser Leu Ala Ile Thr Gly Leu Gln Ala Glu Asp Glu Ala
    210                 215                 220

Asp Tyr Tyr Cys Gln Ser Tyr Asp Ser Ser Leu Ser Gly Ser Val Phe
225                 230                 235                 240
Gly Gly Gly Thr Gln Leu Thr Val Leu Ser Ala Ala Ala His His His
                245                 250                 255
His His His Gly Glu Gln Lys Leu Ile Ser Glu Asp Leu
            260                 265

<210> 81
<211> 269
<212> PRT
<213> Homo Sapiens

<400> 81
Met Ala Glu Val Gln Leu Val Glu Ser Gly Gly Gly Val Val Gln Pro
 1               5                  10                  15
Gly Arg Ser Leu Arg Leu Ser Cys Thr Ile Ser Gly Val Thr Phe Asn
            20                  25                  30
Gln Tyr Ala Ile Thr Trp Val Arg Gln Ala Pro Gly Lys Gly Leu Gln
        35                  40                  45
Trp Leu Ser Thr Ile Ala Gly Thr Gly Thr Lys Thr Phe Tyr Ala Asp
    50                  55                  60
Ser Val Lys Gly Arg Phe Thr Met Ser Arg Asp Ser Ser Gly Asn Thr
65                  70                  75                  80
Leu Tyr Leu Gln Met Asn Ser Leu Arg Asp Glu Asp Thr Ala Val Tyr
                85                  90                  95
Tyr Cys Ala Lys Ser Leu Ser Met Arg Tyr Phe Leu Asp Leu Trp Gly
            100                 105                 110
Arg Gly Thr Leu Val Thr Val Ser Ser Gly Gly Gly Gly Ser Gly Gly
        115                 120                 125
Gly Gly Ser Gly Gly Gly Gly Ser Ser Ala Leu Pro Glu Leu Thr Gln
    130                 135                 140
Pro Pro Ser Ala Ser Gly Thr Pro Gly Gln Arg Val Thr Ile Ser Cys
145                 150                 155                 160
Ser Gly Ser Ser Ser Asn Ile Gly Ser Asn Thr Val Asn Trp Tyr Gln
                165                 170                 175
Gln Leu Pro Gly Thr Ala Pro Lys Leu Leu Ile Tyr Ser Asn Asn Gln
            180                 185                 190
Arg Pro Ser Gly Val Pro Asp Arg Phe Ser Gly Ser Lys Ser Gly Thr
        195                 200                 205
Ser Ala Ser Leu Ala Ile Ser Gly Leu Gln Ser Glu Asp Glu Ala Asp
    210                 215                 220
Tyr Tyr Cys Ala Ala Trp Asp Asp Ser Leu Asn Gly Leu Val Phe Gly
225                 230                 235                 240
Gly Gly Thr Lys Leu Thr Val Leu Gly Ala Ala Ala His His His His
                245                 250                 255
His His Gly Glu Gln Lys Leu Ile Ser Glu Glu Asp Leu
            260                 265

<210> 82
<211> 268
<212> PRT
<213> Homo Sapiens

<400> 82
Met Ala Glu Val Gln Leu Val Glu Thr Gly Gly Asp Leu Val Arg Pro
 1               5                  10                  15
Gly Gly Ser Leu Arg Leu Ser Cys Thr Ile Ser Gly Val Thr Phe Asn
            20                  25                  30
Gln Tyr Ala Ile Thr Trp Val Arg Gln Ala Pro Gly Lys Gly Leu Gln
        35                  40                  45
Trp Leu Ser Thr Ile Ala Gly Thr Gly Thr Lys Thr Phe Tyr Ala Asp
    50                  55                  60
Ser Val Lys Gly Arg Phe Thr Met Ser Arg Asp Ser Ser Gly Asn Thr

65                  70                  75                  80
Leu Tyr Leu Gln Met Asn Ser Leu Arg Asp Glu Asp Thr Ala Val Tyr
                85                  90                  95
Tyr Cys Ala Lys Ser Leu Ser Met Arg Tyr Phe Leu Asp Leu Trp Gly
            100                 105                 110
Gln Gly Thr Met Val Thr Val Ser Ser Gly Gly Gly Gly Ser Gly Gly
        115                 120                 125
Gly Gly Ser Gly Gly Gly Gly Ser Ser Ala Gln Ser Val Leu Thr Gln
    130                 135                 140
Pro Pro Ser Ala Ser Gly Thr Pro Gly Gln Arg Val Thr Ile Ser Cys
145                 150                 155                 160
Ser Gly Ser Ser Ser Asn Ile Gly Ser Asn Thr Val Asn Trp Tyr Gln
                165                 170                 175
Gln Leu Pro Gly Thr Ala Pro Lys Leu Leu Ile Tyr Ser Asn Asn Gln
            180                 185                 190
Arg Pro Ser Gly Val Pro Asp Arg Phe Ser Gly Ser Lys Ser Gly Thr
        195                 200                 205
Ser Ala Ser Leu Ala Ile Ser Gly Leu Gln Ser Glu Asp Glu Ala Asp
    210                 215                 220
Tyr Tyr Cys Ala Ala Trp Asp Asp Ser Leu Asn Gly Trp Val Phe Gly
225                 230                 235                 240
Gly Gly Thr Lys Leu Thr Val Leu Ala Ala Ala His His His His His
                245                 250                 255
His Gly Glu Gln Lys Leu Ile Ser Glu Glu Asp Leu
            260                 265

<210> 83
<211> 270
<212> PRT
<213> Homo Sapiens

<400> 83
Met Ala Gln Val Gln Leu Gln Glu Ser Gly Pro Gly Leu Val Lys Pro
1               5                   10                  15
Ser Glu Thr Leu Ser Leu Thr Cys Ser Val Ser Gly Asp Ala Ile Ser
            20                  25                  30
Asn Gly Tyr Phe Trp Gly Trp Ile Arg Gln Pro Pro Gly Gly Gly Leu
        35                  40                  45
Glu Trp Ile Gly Ser Ile Ser His Arg Gly Ser Thr Tyr Tyr Asn Pro
    50                  55                  60
Ser Leu Lys Ser Arg Val Ser Ile Ser Val Asp Thr Ser Lys Asn Gln
65                  70                  75                  80
Phe Ser Leu Ser Leu Thr Ser Val Thr Ala Ala Asp Thr Ala Val Phe
                85                  90                  95
Tyr Cys Ala Arg Ser Asn Gly Asp Tyr Asp Thr Phe Thr Ala Tyr Tyr
            100                 105                 110
Trp Gly Arg Gly Thr Met Val Thr Val Ser Ser Gly Gly Gly Gly Ser
        115                 120                 125
Gly Gly Gly Gly Ser Gly Gly Gly Gly Ser Ser Ala Gln Ala Val Leu
    130                 135                 140
Thr Gln Pro Pro Ser Val Ser Gly Ala Pro Gly Gln Arg Val Thr Ile
145                 150                 155                 160
Ser Cys Thr Gly Ser Ser Ser Asn Ile Gly Ala Gly Tyr Asp Val His
                165                 170                 175
Trp Tyr Gln Gln Leu Pro Gly Thr Ala Pro Lys Leu Leu Ile Tyr Gly
            180                 185                 190
Asn Ser Asn Arg Pro Ser Gly Val Pro Asp Arg Phe Ser Gly Ser Lys
        195                 200                 205
Ser Gly Thr Ser Ala Ser Leu Ala Ile Thr Gly Leu Gln Ala Glu Asp
    210                 215                 220
Glu Ala Asp Tyr Tyr Cys Gln Ser Tyr Asp Ser Ser Leu Ser Gly Ser
225                 230                 235                 240
Val Phe Gly Gly Gly Thr Lys Val Thr Val Leu Ala Ala Ala His His
                245                 250                 255

His His His His Gly Glu Gln Lys Leu Ile Ser Glu Asp Leu
            260                 265                 270

<210> 84
<211> 269
<212> PRT
<213> Homo Sapiens

<400> 84
Met Ala Gln Val Gln Leu Gln Glu Ser Gly Pro Gly Leu Leu Lys Pro
 1               5                  10                  15
Ser Gln Thr Leu Ser Leu Thr Cys Thr Val Ser Gly Asp Ser Ile Ser
            20                  25                  30
Ser Gly Asp His Tyr Trp Asn Trp Ile Arg Gln Pro Ala Gly Lys Gly
        35                  40                  45
Leu Glu Trp Ile Gly Arg Leu Tyr Thr Asn Gly Ile Thr Asp Tyr Asn
    50                  55                  60
Pro Ser Leu Arg Ser Arg Val Ile Ile Ser Ala Asp Thr Ser Lys Asn
65                  70                  75                  80
Gln Phe Thr Leu Lys Leu Ser Ala Val Thr Ala Ala Asp Thr Ala Val
                85                  90                  95
Tyr Tyr Cys Ala Arg Asp Val Trp Glu Pro Gly Thr Phe Glu His Trp
            100                 105                 110
Gly Lys Gly Thr Met Val Thr Val Ser Ser Gly Gly Gly Gly Ser Gly
        115                 120                 125
Gly Gly Gly Ser Gly Gly Gly Gly Ser Ser Ala Leu Ser Ser Glu Leu
    130                 135                 140
Thr Gln Asp Pro Ala Val Ser Val Ala Leu Gly Gln Thr Val Arg Ile
145                 150                 155                 160
Thr Cys Gln Gly Asp Ser Leu Arg Ser Tyr Tyr Ala Ser Trp Tyr Gln
                165                 170                 175
Gln Lys Pro Gly Gln Ala Pro Ile Leu Val Ile Tyr Gly Lys Asn Asn
            180                 185                 190
Arg Pro Ser Gly Ile Pro Asp Arg Phe Ser Gly Ser Ser Ser Gly Asn
        195                 200                 205
Thr Ala Ser Leu Thr Ile Thr Gly Ala Gln Ala Glu Asp Glu Ala Asp
    210                 215                 220
Tyr Tyr Cys Asn Ser Arg Asp Ser Asn Gly Asp Val Leu Ser Val Phe
225                 230                 235                 240
Gly Gly Gly Thr Lys Leu Thr Val Leu Ala Ala Ala His His His His
                245                 250                 255
His His Gly Glu Gln Lys Leu Ile Ser Glu Glu Asp Leu
            260                 265

## Description

### Field of the Invention

The present invention concerns methods and means for identifying, producing, and engineering neutralizing antibodies against influenza A viruses, and to the neutralizing antibodies produced. In particular, the invention concerns neutralizing antibodies against various influenza A virus subtypes, including neutralizing antibodies against two or more of H1, H2, H3, H5, H7 and H9, such as, for example all of H1, H2, H3, and H5 subtypes, and methods and means for making such antibodies. More specifically, the invention concerns antibodies capable of neutralizing more than one, preferably all, isolates of an influenza A virus subtype.

### Background of the Invention

The flu is a contagious respiratory illness caused by influenza viruses. It causes mild to severe illness, and at times can lead to death. Annually, in the United States, influenza is contracted by 5-20% of the population, hospitalizing about 200,000, and causing the deaths of about 36,000.

Influenza viruses spread in respiratory droplets caused by coughing and sneezing, which are usually transmitted from person to person. Immunity to influenza surface antigens, particularly hemagglutinin, reduces the likelihood of infection and severity of disease if infection occurs. Although influenza vaccines are available, because an antibody against one influenza virus type or subtype confers limited or no protection against another type or subtype of influenza, it is necessary to incorporate one or more new strains in each year's influenza vaccine.

Influenza viruses are segmented negative-strand RNA viruses and belong to the *Orthomyxoviridae* family. Influenza A virus consists of 9 structural proteins and codes additionally for one nonstructural NS1 protein with regulatory functions. The non-structural NS1 protein is synthesized in large quantities during the reproduction cycle and is localized in the cytosol and nucleus of the infected cells. The segmented nature of the viral genome allows the mechanism of genetic reassortment (exchange of genome segments) to take place during mixed infection of a cell with different viral strains. The influenza A virus is further classified into various subtypes depending on the different hemagglutinin (HA) and neuraminidase (NA) viral proteins displayed on their surface.

Influenza A virus subtypes are identified by two viral surface glycoproteins, hemagglutinin (HA or H) and neuraminidase (NA or N). Each influenza virus subtype is identified by its combination of H and N proteins. There are 16 known HA subtypes and 9 known NA subtypes. Influenza type A viruses can infect people, birds, pigs, horses, and other animals, but wild birds are the natural hosts for these viruses. Only some influenza A subtypes (i.e., H1N1, H1N2, and H3N2) are currently in circulation among people, but all combinations of the 16 H and 9 NA subtypes have been identified in avian species, especially in wild waterfowl and shorebirds. In addition, there is increasing evidence that H5 and H7 influenza viruses can also cause human illness.

The HA of influenza A virus comprises two structurally distinct regions, namely, a globular head region and a stem region. The globular head region contains a receptor binding site which is responsible for virus attachment to a target cell and participates in the hemagglutination activity of HA. The stem region contains a fusion peptide which is necessary for membrane fusion between the viral envelope and an endosomal membrane of the cell and thus relates to fusion activity (Wiley et al., Ann. Rev. Biochem., 56:365-394 (1987)).

A pandemic is a global disease outbreak. An influenza pandemic occurs when a new influenza A virus: (1) emerges for which there is little or no immunity in the human population, (2) begins to cause serious illness, and then (3) spreads easily person-to-person worldwide. During the 20^{th} century there have been three such influenza pandemics. First, in 1918, the "Spanish Flu" influenza pandemic caused at least 500,000 deaths in the United States and up to 40 million deaths worldwide. This pandemic was caused by influenza A H1N1 subtype. Second, in 1957, the "Asian Flu" influenza pandemic, caused by the influenza A H2N2 subtype, resulted in at least 70,000 deaths in the United States and 1-2 million deaths worldwide. Most recently in 1968 the "Hong Kong Flu" influenza pandemic, caused by the influenza A H3N2 subtype, resulted in about 34,000 U.S. deaths and 700,000 deaths worldwide.

In 1997, the first influenza A H5N1 cases were reported in Hong Kong. This was the first time that this avian type virus directly infected humans, but a pandemic did not result because human to human transmission was not observed.

Lu et al., Resp. Res. 7:43 (2006) (doi: 10.1186/1465-992-7-43) report the preparation of anti-H51 IgGs from horses vaccinated with inactivated H5N1 virus, and of H5N1-specifc F(ab')₂ fragments, which were described to protect BALB/c mice infected with H5N1 virus.

Hanson et al., Resp. Res. 7:126 (doi: 10.1186/1465-9921-7-126) describe the use of a chimeric monoclonal antibody specific for influenza A H5 virus hemagglutinin for passive immunization of mice.

In view of the severity of the respiratory illness caused by certain influenza A viruses, and the threat of a potential pandemic, there is a great need for effective preventative and treatment methods. The present invention addresses this need by providing influenza A neutralizing antibodies against various H subtypes of the virus, including, without limitation, the H1, and H3 subtypes, and the H5 subtype of the influenza A virus. The invention further provides antibodies capable of neutralizing more than one, and preferably all, isolates (strains) of a given subtype of the influenza A virus, including, without limitation, isolates obtained from various human and non-human species and isolates from victims and/or survivors of various influenza epidemics and/or pandemics.

Such neutralizing antibodies can be used for the prevention and/or treatment influenza virus infection, including passive immunization of infected or at risk populations in cases of epidemics or pandemics.

### Summary of the Invention

In one aspect, the present invention concerns a neutralizing antibody neutralizing more than one isolate of an influenza A virus subtype or more than one subtype of the influenza A virus.

In one embodiment, the antibody neutralizes substantially all isolates of an influenza A virus subtype, such as one or more of the H5, H7 and H9 subtypes.

In another embodiment, the antibody neutralizes more than one isolate of a particular influenza A virus subtype, such as one or more of the H5, H7 and H9 subtypes.

In yet another embodiment, the antibody neutralizes more than one subtype and more than one isolates of at least one subtype of the influenza A virus.

In a further embodiment, at least one of the subtypes and/or isolates neutralized by the antibodies herein has the ability to infect humans.

In another embodiment, at least one of the isolates is from a bird, including, for example, wild-fowls and chicken.

In a particular embodiment, the antibodies herein neutralize the H5N1 subtype of the influenza A virus. Preferably, the antibodies neutralize more than one isolate, or, even more preferably, substantially all isolates of this influenza A virus subtype.

In another embodiment, the antibodies herein neutralize the H5N1 subtype and at least one additional subtype selected from the group consisting of H1N1, H1N2, and H3N2 subtypes.

In additional embodiments, the antibodies herein neutralize more than one isolate, preferably substantially all isolates of the additional subtype(s).

In another embodiment, the neutralizing antibodies of the present invention bind the H5 protein. Preferably, the antibodies bind more than one variants of the H5 protein, or, even more preferably, substantially all variants of the H5 protein.

In other embodiments, the antibodies herein bind to the H5 protein and to at least one additional H protein, such as an H1, H2 and/or H3 protein.

In a different aspect, the invention concerns compositions comprising the neutralizing antibodies described herein.

In a further aspect, the invention concerns a method for identifying an antibody capable of neutralizing more than one isolate of a single influenza A virus subtype or multiple influenza A virus subtypes. This method comprises identifying antibodies in an antibody library that react with both a first and a second isolate of the influenza A virus subtype or with a first and a second subtype of the influenza A virus, and subjecting the antibodies identified to successive alternating rounds of selection, based on their ability to bind the first and second isolates, or the first and second subtypes, respectively.

In an embodiment, antibodies that react with both a first and a second influenza A virus subtype isolate have been identified by at least two rounds of separate enrichment of antibodies reacting with the first isolate and the second isolate, respectively, and recombining the antibodies identified.

In another embodiment, the antibody that can react with both the first and the second influenza A subtype isolate is subjected to mutagenesis prior to being subjected to successive alternating rounds of selection, based on its ability to bind the first and second isolate, respectively. If desired, the antibodies capable of binding the first and the second isolate are additionally selected based on their ability to bind more than one influenza A subtype.

The application of such enrichment techniques can be similarly applied to antibodies in general, regardless of the target to which they bind. Such general enrichment/selection methods are specifically included as part of the invention.

In a further aspect, the invention concerns a collection of sequences shared by the neutralizing antibodies of the present invention.

In a still further aspect, the invention concerns a method for treating an influenza A infection in a subject comprising of administering to the subject an effective amount of a neutralizing antibody or antibody composition herein.

In another aspect, the invention concerns a method for preventing influenza A infection comprising of administering to a subject at risk of developing influenza A infection an effective amount of a neutralizing antibody of the present invention.

In a different aspect, the invention concerns a method for producing a diverse multifunctional antibody collection, comprising: (a) aligning CDR sequences of at least two functionally different antibodies, (b) identifying amino acid residues conserved between the CDR sequences aligned, and (c) performing mutagenesis of multiple non-conserved amino acid residues in at least one of the CDR sequences aligned, using degenerate oligonucleotide probes encoding at least the amino acid residues present in the functionally different antibodies at the non-conserved positions mutagenized to produce multiple variants of the aligned CDR sequences, and, if desired, repeating steps (b) and (c) with one or more of the variants until the antibody collection reaches a desired degree of diversity and/or size.

In a particular embodiment, the CDR sequences aligned have the same lengths.

In another embodiment, the conserved amino acid residues are retained in at least two of the CDR sequences aligned.

In a further aspect, the invention concerns an antibody collection comprising a plurality of neutralizing antibodies which differ from each other in at least one property.

The invention further concerns a method for uniquely identifying nucleic acids in a collection comprising labeling the nucleic acids with a unique barcode linked to or incorporated in the sequences of the nucleic acid present in such collection.

### Brief Description of the Drawings

Figure 1 shows the amino acid sequences of 15 known hemagglutinin (H) protein subtypes.

Figure 2 illustrates a typical panning enrichment scheme for increasing the reactive strength towards two different targets, A and B. Each round of enrichment increases the reactive strength of the pool towards the individual target(s).

Figure 3 illustrates a strategy for the selection of clones cross-reactive with targets A and B, in which each successive round reinforces the reactive strength of the resulting pool towards both targets.

Figure 4 illustrates a strategy for increasing the reactive strengths towards two different targets (targets A and B), by recombining parallel discovery pools to generate/increase cross-reactivity. Each round of selection of the recombined antibody library increases the reactive strength of the resulting pool towards both targets.

Figure 5 illustrates a strategy for increasing cross-reactivity to a target B while maintaining reactivity to a target A. First, a clone reactive with target A is selected, then a mutagenic library of the clones reactive with target A is prepared, and selection is performed as shown, yielding one or more antibody clones that show strong reactivity with both target A and target B.

Figure 6 illustrates a representative mutagenesis method for generating a diverse multifunctional antibody collection by the "destinational mutagenesis" method.

Figure 7 shows the H5 hemagglutinin (HA) serology results for blood samples obtained from six human survivors of a Turkish H5N1 bird flu outbreak. The data demonstrate the presence of antibodies to the HA antigen.

Figure 8 shows serology results obtained with serum samples of twelve local donors, tested on H5 antigen (A/Vietnam/1203/2004) and H1N1 (A/New Caledonia/20/99) and H3N2 (A/Panama/2007/99) viruses.

Figure 9 illustrates the unique barcoding approach used in the construction of antibody phage libraries.

Figure 10 shows the results of a scFv ELISA test of five distinct clones obtained from pooled libraries of Turkish bird flu survivors on H5 protein and H5N1 virus.

Figure 11 shows sequence alignments comparing the sequences of H5 hemagglutinin proteins from reported Turkish isolates and one Vietnamese isolate downloaded from the Los Alamos National Laboratory sequence database.

Figures 12 and 13 show heavy chain variable region sequences of unique clones identified in pooled antibody libraries of Turkish donors, along with the corresponding light chain and germline origin sequences. The sequences shown in Figure 12 (3-23 heavy chain clones) originate from a pooled library of all heavy and light chains of all Turkish donors after three rounds of panning. The sequences shown in Figure 13 (3-30 heavy chain clones) originate from a pooled library of all heavy and light chains of all Turkish donors after two rounds of panning.

Figures 14A-D show additional unique H5N1-specific antibody heavy chain variable region sequences identified from antibody libraries of individual Turkish donors, after four rounds of panning.

Figures 15 and 16 illustrate the use of destinational mutagenesis to create diverse antibody heavy and light chain libraries using the antibody heavy (Figure 15) and light chain (Figure 16) sequences identified by analysis of sera and bone marrow of Turkish bird flu survivors.

Figures 17 and 18 show ELISA results confirming cross-reactivity of certain Fab fragments obtained from an H5N1 Vietnam virus scFv antibody with Turkish and Indonesian variants of the HA protein.

### Detailed Description

### A. Definitions

Unless defined otherwise, technical and scientific terms used herein have the same meaning as commonly understood by one of ordinary skill in the art to which this invention belongs. Singleton et al., Dictionary of Microbiology and Molecular Biology 2nd ed., J. Wiley & Sons (New York, NY 1994), provides one skilled in the art with a general guide to many of the terms used in the present application.

One skilled in the art will recognize many methods and materials similar or equivalent to those described herein, which could be used in the practice of the present invention. Indeed, the present invention is in no way limited to the methods and materials described. For purposes of the present invention, the following terms are defined below.

The terms "influenza A subtype" or "influenza A virus subtype" are used interchangeably, and refer to influenza A virus variants that are characterized by various combinations of the hemagglutinin (H) and neuraminidase (N) viral surface proteins, and thus are labeled by a combination of an H number and an N number, such as, for example, H1N1 and H3N2. The terms specifically include all strains (including extinct strains) within each subtype, which usually result from mutations and show different pathogenic profiles. Such strains will also be referred to as various "isolates" of a viral subtype, including all past, present and future isolates. Accordingly, in this context, the terms "strain" and "isolate" are used interchangeably.

The term "influenza" is used to refer to a contagious disease caused by an influenza virus.

In the context of the present invention, the term "antibody" (Ab) is used in the broadest sense and includes polypeptides which exhibit binding specificity to a specific antigen as well as immunoglobulins and other antibody-like molecules which lack antigen specificity. Polypeptides of the latter kind are, for example, produced at low levels by the lymph system and, at increased levels, by myelomas. In the present application, the term "antibody" specifically covers, without limitation, monoclonal antibodies, polyclonal antibodies, and antibody fragments.

"Native antibodies" are usually heterotetrameric glycoproteins of about 150,000 daltons, composed of two identical light (L) chains and two identical heavy (H) chains. Each light chain is linked to a heavy chain by covalent disulfide bond(s), while the number of disulfide linkages varies between the heavy chains of different immunoglobulin isotypes. Each heavy and light chain also has regularly spaced intrachain disulfide bridges. Each heavy chain has, at one end, a variable domain (V_{H}) followed by a number of constant domains. Each light chain has a variable domain at one end (V_{L}) and a constant domain at its other end; the constant domain of the light chain is aligned with the first constant domain of the heavy chain, and the light chain variable domain is aligned with the variable domain of the heavy chain. Particular amino acid residues are believed to form an interface between the light- and heavy-chain variable domains, Chothia et al., J. Mol. Biol, 186:651 (1985); Novotny and Haber, Proc. Natl. Acad. Sci. U.S.A. 82:4592 (1985).

The term "variable" with reference to antibody chains is used to refer to portions of the antibody chains which differ extensively in sequence among antibodies and participate in the binding and specificity of each particular antibody for its particular antigen. Such variability is concentrated in three segments called hypervariable regions both in the light chain and the heavy chain variable domains. The more highly conserved portions of variable domains are called the framework region (FR). The variable domains of native heavy and light chains each comprise four FRs (FR1, FR2, FR3 and FR4, respectively), largely adopting a β-sheet configuration, connected by three hypervariable regions, which form loops connecting, and in some cases forming part of, the β-sheet structure. The hypervariable regions in each chain are held together in close proximity by the FRs and, with the hypervariable regions from the other chain, contribute to the formation of the antigen-binding site of antibodies (see Kabat et al., Sequences of Proteins of Immunological Interest, 5th Ed. Public Health Service, National Institutes of Health, Bethesda, Md. (1991), pages 647-669). The constant domains are not involved directly in binding an antibody to an antigen, but exhibit various effector functions, such as participation of the antibody in antibody-dependent cellular toxicity.

The term "hypervariable region" when used herein refers to the amino acid residues of an antibody which are responsible for antigen-binding. The hypervariable region comprises amino acid residues from a "complementarity determining region" or "CDR" (*i.e*., residues 30-36 (L1), 46-55 (L2) and 86-96 (L3) in the light chain variable domain and 30-35 (H1), 47-58 (H2) and 93-101 (H3) in the heavy chain variable domain; MacCallum et al,. J Mol Biol. 1996. "Framework" or "FR" residues are those variable domain residues other than the hypervariable region residues as herein defined.

Depending on the amino acid sequence of the constant domain of their heavy chains, antibodies can be assigned to different classes. There are five major classes of antibodies IgA, IgD, IgE, IgG, and IgM, and several of these may be further divided into subclasses (isotypes), *e.g*., IgG1, IgG2, IgG3, IgG4, IgA, and IgA2.

The heavy-chain constant domains that correspond to the different classes of immunoglobulins are called α, δ, ε, γ, and µ, respectively.

The "light chains" of antibodies from any vertebrate species can be assigned to one of two clearly distinct types, called kappa (κ) and lambda (λ), based on the amino acid sequences of their constant domains.

"Antibody fragments" comprise a portion of a full length antibody, generally the antigen binding or variable domain thereof. Examples of antibody fragments include, but are not limited to, Fab, Fab', F(ab')₂, and Fv fragments, linear antibodies, single-chain antibody molecules, diabodies, and multispecific antibodies formed from antibody fragments.

The term "monoclonal antibody" is used to refer to an antibody molecule synthesized by a single clone of B cells. The modifier "monoclonal" indicates the character of the antibody as being obtained from a substantially homogeneous population of antibodies, and is not to be construed as requiring production of the antibody by any particular method. Thus, monoclonal antibodies may be made by the hybridoma method first described by Kohler and Milstein, Nature 256:495 (1975); Eur. J. Immunol. 6:511 (1976), by recombinant DNA techniques, or may also be isolated from phage antibody libraries.

The term "polyclonal antibody" is used to refer to a population of antibody molecules synthesized by a population of B cells.

"Single-chain Fv" or "sFv" antibody fragments comprise the V_{H} and V_{L} domains of antibody, wherein these domains are present in a single polypeptide chain. Generally, the Fv polypeptide further comprises a polypeptide linker between the V_{H} and V_{L} domains which enables the sFv to form the desired structure for antigen binding. For a review of sFv see Plückthun in The Pharmacology of Monoclonal Antibodies, vol. 113, Rosenburg and Moore eds. Springer-Verlag, New York, pp. 269-315 (1994). Single-chain antibodies are disclosed, for example in WO 88/06630 and WO 92/01047.

The term "diabody" refers to small antibody fragments with two antigen-binding sites, which fragments comprise a heavy chain variable domain (V_{H}) connected to a light chain variable domain (V_{L}) in the same polypeptide chain (V_{H}-V_{L}). By using a linker that is too short to allow pairing between the two domains on the same chain, the domains are forced to pair with the complementary domains of another chain and create two antigen-binding sites. Diabodies are described more fully in, for example, EP 404,097; WO 93111161; and Hollinger et al., Proc. Natl. Acad Sci. USA 90:6444-6448 (1993).

The term "bispecific antibody" refers to an antibody that shows specificities to two different types of antigens. The term as used herein specifically includes, without limitation, antibodies which show binding specificity for a target antigen and to another target that facilitates delivery to a particular tissue. Similarly, multi-specific antibodies have two or more binding specificities.

The expression "linear antibody" is used to refer to comprising a pair of tandem Fd segments (V_{H}-C_{H}1-V_{H}-C_{H}1) which form a pair of antigen binding regions. Linear antibodies can be bispecific or monospecific and are described, for example, by Zapata et al., Protein Eng. 8(10):1057-1062 (1995).

The term "neutralizing antibody" is used herein in the broadest sense and refers to any antibody that inhibits an influenza virus from replicatively infecting a target cell, regardless of the mechanism by which neutralization is achieved. Thus, for example, neutralization can be achieved by inhibiting the attachment or adhesion of the virus to the cell surface, *e.g*., by engineering an antibody that binds directly to, or close by, the site responsible for the attachment or adhesion of the virus. Neutralization can also be achieved by an antibody directed to the virion surface, which results in the aggregation of virions. Neutralization can further occur by inhibition of the fusion of viral and cellular membranes following attachment of the virus to the target cell, by inhibition of endocytosis, inhibition of progeny virus from the infected cell, and the like. The neutralizing antibodies of the present invention are not limited by the mechanism by which neutralization is achieved.

The term "antibody repertoire" is used herein in the broadest sense and refers to a collection of antibodies or antibody fragments which can be used to screen for a particular property, such as binding ability, binding specificity, ability of gastrointestinal transport, stability, affinity, and the like. The term specifically includes antibody libraries, including all forms of combinatorial libraries, such as, for example, antibody phage display libraries, including, without limitation, single-chain Fv (scFv) and Fab antibody phage display libraries from any source, including naïve, synthetic and semi-synthetic libraries.

A "phage display library" is a protein expression library that expresses a collection of cloned protein sequences as fusions with a phage coat protein. Thus, the phrase "phage display library" refers herein to a collection of phage (*e.g*., filamentous phage) wherein the phage express an external (typically heterologous) protein. The external protein is free to interact with (bind to) other moieties with which the phage are contacted. Each phage displaying an external protein is a "member" of the phage display library.

An "antibody phage display library" refers to a phage display library that displays antibodies or antibody fragments. The antibody library includes the population of phage or a collection of vectors encoding such a population of phage, or cell(s) harboring such a collection of phage or vectors. The library can be monovalent, displaying on average one single-chain antibody or antibody fragment per phage particle, or multi-valent, displaying, on average, two or more antibodies or antibody fragments per viral particle. The term "antibody fragment" includes, without limitation, single-chain Fv (scFv) fragments and Fab fragments. Preferred antibody libraries comprise on average more than 10⁶, or more than 10⁷, or more than 10⁸, or more than 10⁹ different members.

The term "filamentous phage" refers to a viral particle capable of displaying a heterogenous polypeptide on its surface, and includes, without limitation, f1, fd, Pf1, and M13. The filamentous phage may contain a selectable marker such as tetracycline (*e.g*., "fd-tet"). Various filamentous phage display systems are well known to those of skill in the art (see, *e.g.,* Zacher et al., Gene 9:127-140 (1980), Smith et al., Science 228:1315-1317 (1985); and Parmley and Smith, Gene 73:305-318 (1988)).

The term "panning" is used to refer to the multiple rounds of screening process in identification and isolation of phages carrying compounds, such as antibodies, with high affinity and specificity to a target.

The term "non-human animal" as used herein includes, but is not limited to, mammals such as, for example, non-human primates, rodents (*e.g*., mice and rats), and non-rodent animals, such as, for example, rabbits, pigs, sheep, goats, cows, pigs, horses and donkeys. It also includes birds (*e.g*., chickens, turkeys, ducks, geese and the like). The term "non-primate animal" as used herein refers to mammals other than primates, including but not limited to the mammals specifically listed above.

The phrase "functionally different antibodies," and grammatical variants thereof, are used to refer to antibodies that differ from each other in at least one property, including, without limitation, binding specificity, binding affinity, and any immunological or biological function, such as, for example, ability to neutralize a target, extent or quality of biological activity, etc.

The phrase "conserved amino acid residues" is used to refer to amino acid residues that are identical between two or more amino acid sequences aligned with each other.

### B. General Techniques

Techniques for performing the methods of the present invention are well known in the art and described in standard laboratory textbooks, including, for example, Ausubel et al., Current Protocols of Molecular Biology, John Wiley and Sons (1997); Molecular Cloning: A Laboratory Manual, Third Edition, J. Sambrook and D. W. Russell, eds., Cold Spring Harbor, New York, USA, Cold Spring Harbor Laboratory Press, 2001; Antibody Phage Display: Methods and Protocols, P.M. O'Brian and R. Aitken, eds., Humana Press, In: Methods in Molecular Biology, Vol. 178; Phage Display: A Laboratory Manual, C.F. Barbas III et al. eds., Cold Spring Harbor, New York, USA, Cold Spring Harbor Laboratory Press, 2001; and Antibodies, G. Subramanian, ed., Kluwer Academic, 2004. Mutagenesis can, for example, be performed using site-directed mutagenesis (Kunkel et al., Proc. Natl. Acad. Sci USA 82:488-492 (1985)).

In the following description, the invention is illustrated with reference to certain types of antibody libraries, but the invention is not limited to the use of any particular type of antibody library. Recombinant monoclonal antibody libraries can be based on immune fragments or naïve fragments. Antibodies from immune antibody libraries are typically constructed with V_{H} and V_{L} gene pools that are cloned from source B cells into an appropriate vector for expression to produce a random combinatorial library, which can subsequently be selected for and/or screened. Other types of libraries may be comprised of antibody fragments from a source of genes that is not explicitly biased for clones that bind to an antigen. Thus, naive antibody libraries derive from natural, unimmunized, rearranged V genes. Synthetic antibody libraries are constructed entirely by *in vitro* methods, introducing areas of complete or tailored degeneracy into the CDRs of one or more V genes. Semi-synthetic libraries combine natural and synthetic diversity, and are often created to increase natural diversity while maintaining a desired level of functional diversity. Thus, such libraries can, for example, be created by shuffling natural CDR regions (Soderlind et al., Nat. Biotechnol. 18:852-856 (2000)), or by combining naturally rearranged CDR sequences from human B cells with synthetic CDR1 and CDR2 diversity (Hoet et al., Nat. Biotechnol. 23:455-38 (2005)). The present invention encompasses the use of naïve, synthetic and semi-synthetic antibody libraries, or any combination thereof.

Similarly, the methods of the present invention are not limited by any particular technology used for the display of antibodies. Although the invention is illustrated with reference to phage display, antibodies of the present invention can also be identified by other display and enrichment technologies, such as, for example, ribosome or mRNA display (Mattheakis et al., Proc. NatL Acad. Sci. USA 91:9022-9026 (1994); Hanes and Pluckthun, Proc. Natl. Acad. Sci. USA 94:4937-4942 (1997)), microbial cell display, such as bacterial display (Georgiou et al., Nature Biotech. 15:29-34 (1997)), or yeast cell display (Kieke et al., Protein Eng. 10:1303-1310 (1997)), display on mammalian cells, spore display, viral display, such as retroviral display (Urban et al., Nucleic Acids Res. 33:e35 (2005), display based on protein-DNA linkage (Odegrip et al., Proc. Acad. Natl. Sci. USA 101:2806-2810 (2004); Reiersen et al., Nucleic Acids Res. 33:e10 (2005)), and microbead display (Sepp et al., FEBS Lett. 532:455-458 (2002)).

In ribosome display, the antibody and the encoding mRNA are linked by the ribosome, which at the end of translating the mRNA is made to stop without releasing the polypeptide. Selection is based on the ternary complex as a whole.

In a mRNA display library, a covalent bond between an antibody and the encoding mRNA is established via puromycin, used as an adaptor molecule (Wilson et al., Proc. Natl. Acad. Sci. USA 98:3750-3755 (2001)). For use of this technique to display antibodies, see, *e.g*., Lipovsek and Pluckthun, J. Immunol. Methods. 290:51-67 (2004).

Microbial cell display techniques include surface display on a yeast, such as *Saccharomyces cerevisiae* (Boder and Wittrup, Nat. Biotechnol. 15:553-557 (1997)). Thus, for example, antibodies can be displayed on the surface of *S. cerevisiae* via fusion to the α-agglutinin yeast adhesion receptor, which is located on the yeast cell wall. This method provides the possibility of selecting repertoires by flow cytometry. By staining the cells by fluorescently labeled antigen and an anti-epitope tag reagent, the yeast cells can be sorted according to the level of antigen binding and antibody expression on the cell surface. Yeast display platforms can also be combined with phage (see, *e.g*., Van den Beucken et al., FEBS Lett. 546:288-294 (2003)).

For a review of techniques for selecting and screening antibody libraries see, *e.g*., Hoogenboom, Nature Biotechnol. 23(9):1105-1116 (2005).

### C. Detailed Description of Preferred Embodiments

The present invention concerns the selection, production and use of monoclonal antibodies neutralizing more than one strain (isolate) of an influenza A subtype, including isolates of extinct strains, as well as neutralizing antibodies to more than one influenza A subtype, including subtypes characterized by the presence of an H5 hemagglutinin. In a particular embodiment, the invention concerns the selection, production and use of monoclonal antibodies neutralizing more than one influenza A subtypes and/or more than one isolate, or more than two isolates, or more than three isolates, or more than four isolates, or more than five isolates, etc., most preferably all isolates of one or more subtypes.

The virions of influenza A virus contain 8 segments of linear negative-sense single stranded RNA. The total genome length is 13600 nucleotides, and the eight segments are 2350 nucleotides; 2350 nucleotides; of 2250 nucleotides; 1780 nucleotides; 1575 nucleotides; 1420 nucleotides; 1050 nucleotides; and 900 nucleotides, respectively, in length. Host specificity and attenuation of influenza A virus have been attributed to viral hemagglutinin (H, HA), nucleoprotein (NP), matrix (M), and non-structural (NS) genes individually or in combinations of viral genes (see, *e.g.,* Rogers et al., Virology 127:361-373 (1983); Scholtissek et al., Virology 147:287-294 (1985); Snyder et al., J. Clin. Microbiol. 24:467-469 (1986); Tian et al., J. Virol. 53:771-775 (1985); Treanor et al., Virology 171:1-9 (1989).

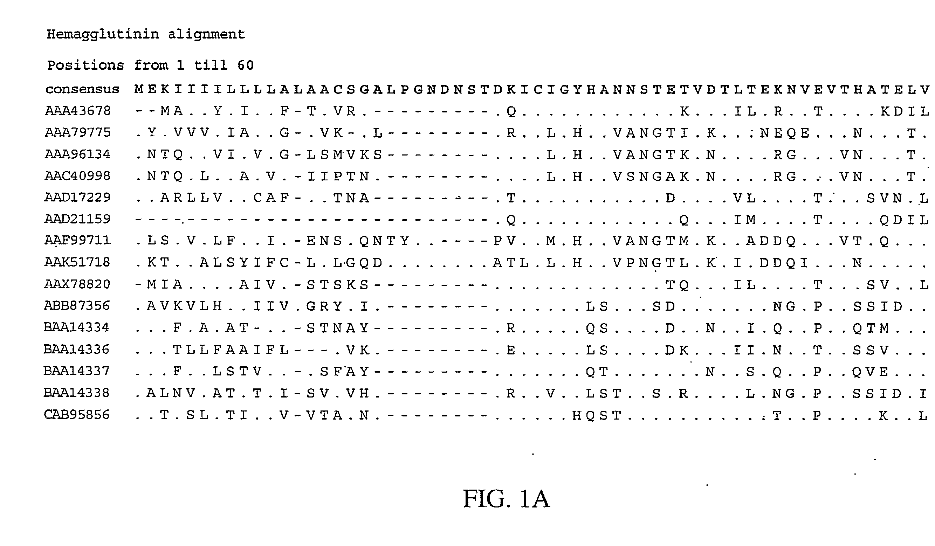

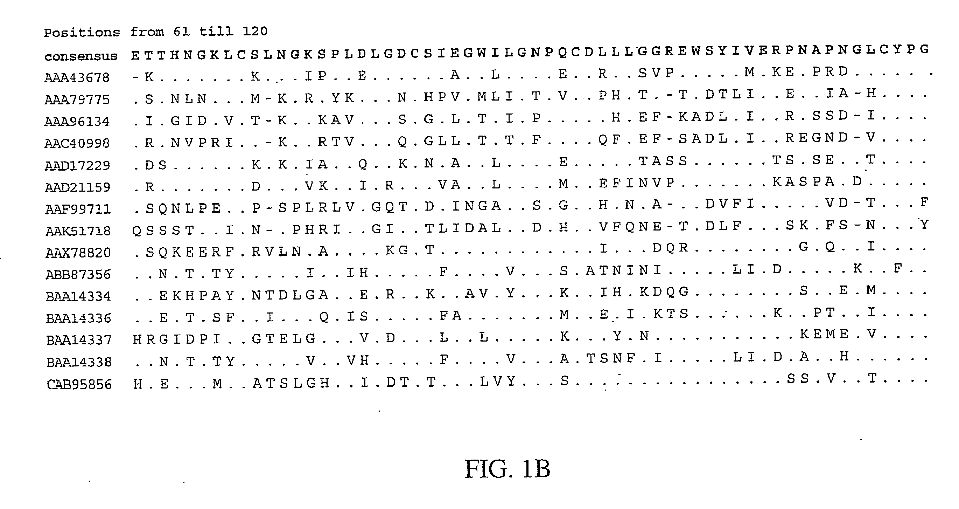

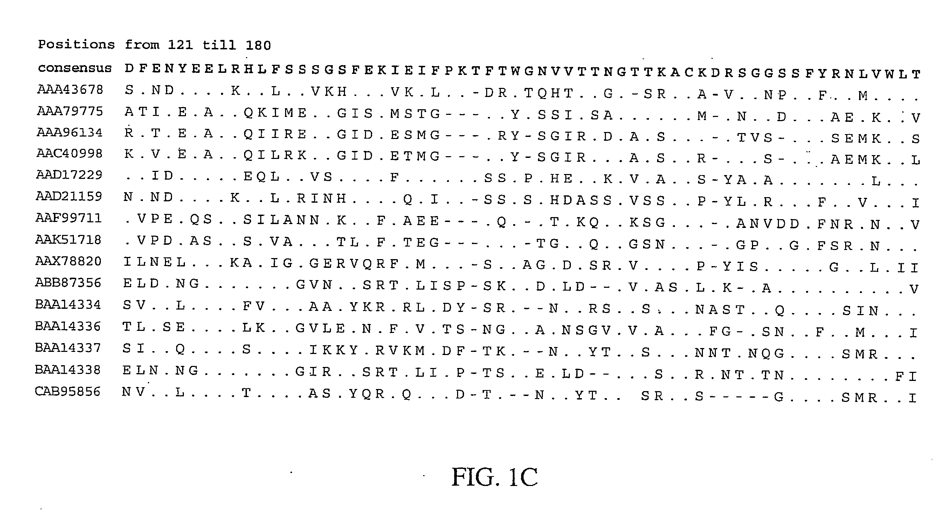

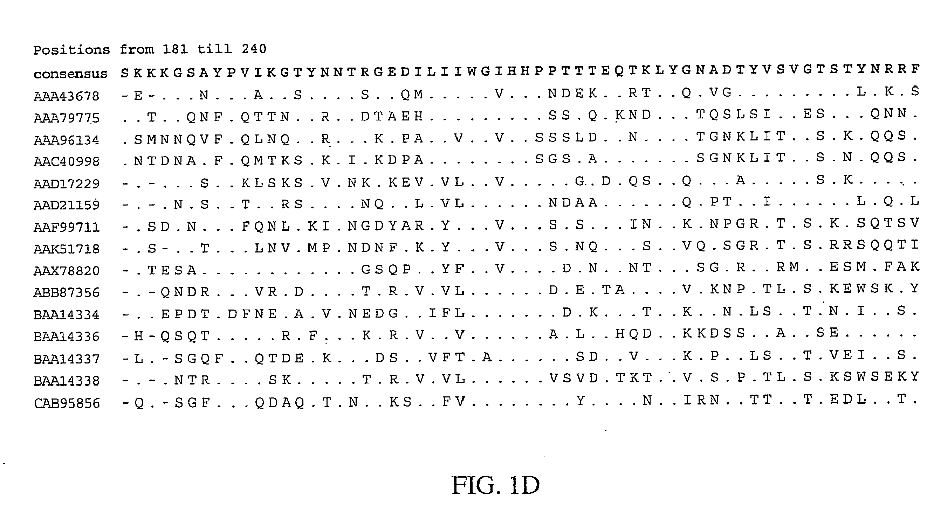

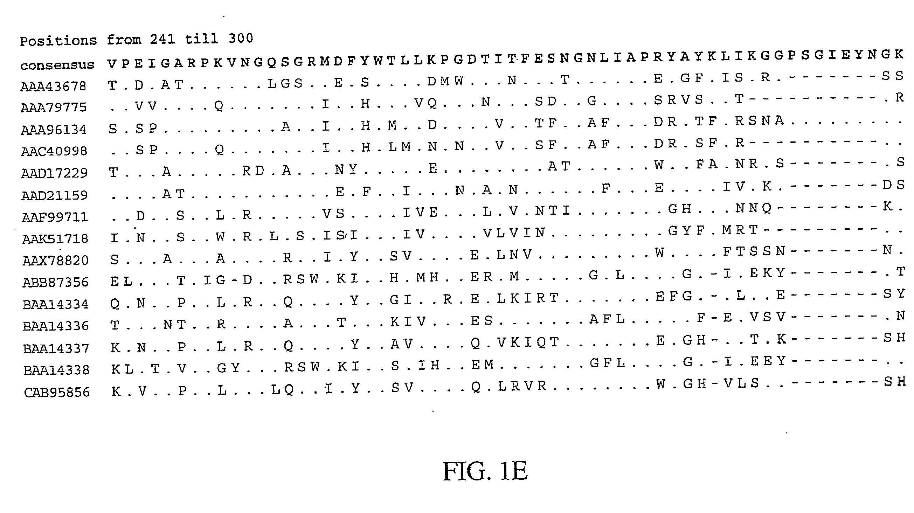

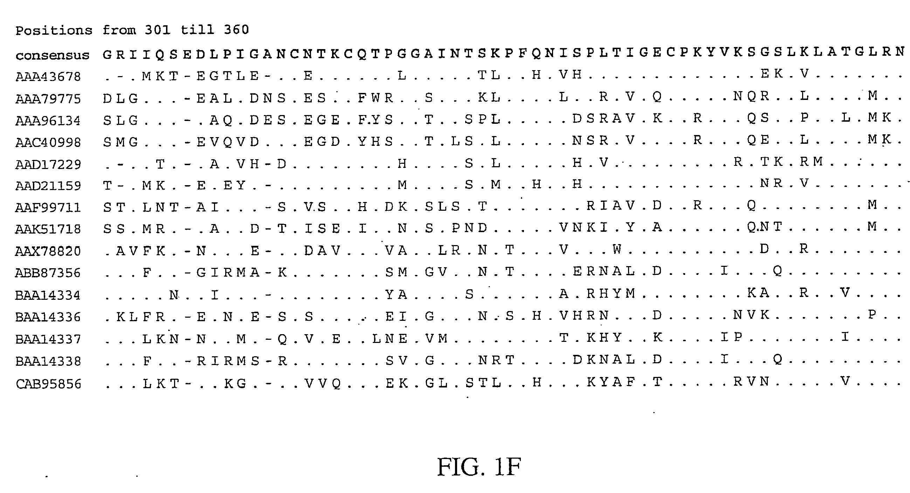

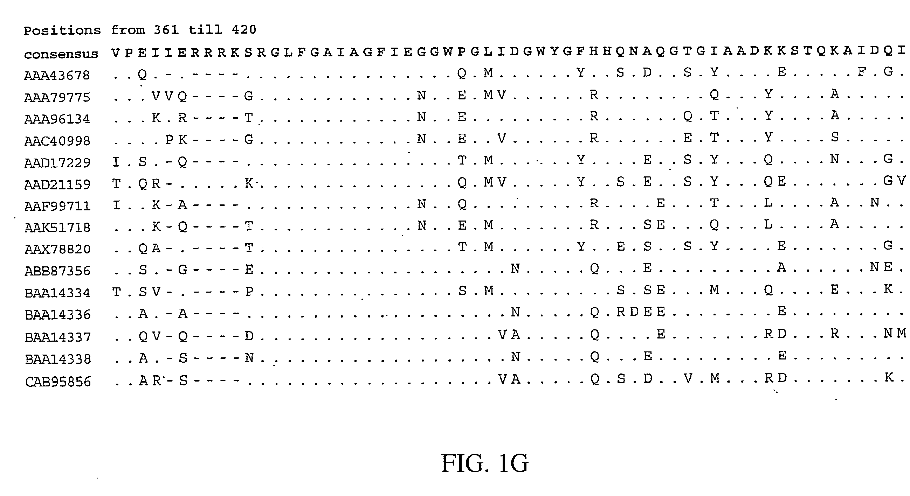

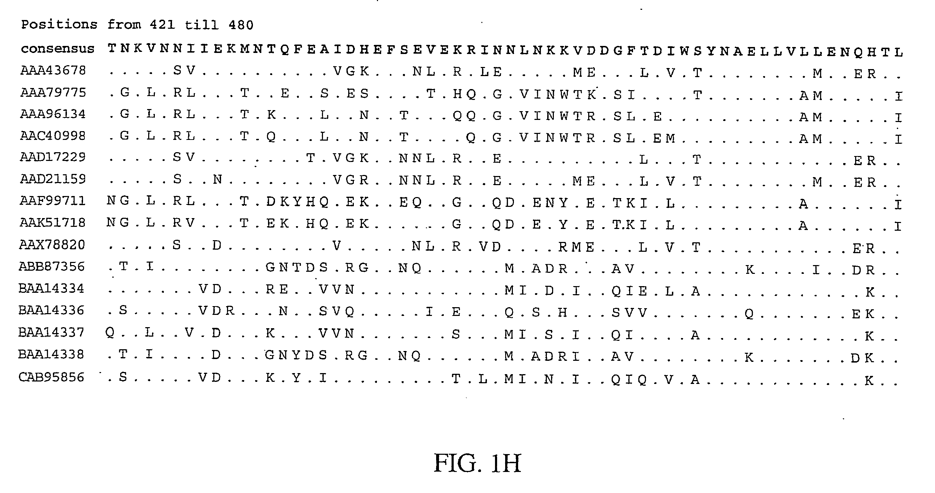

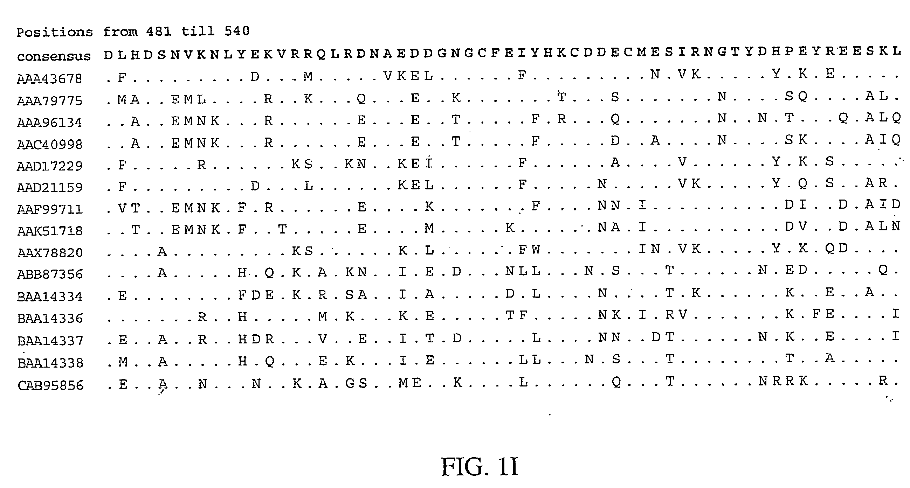

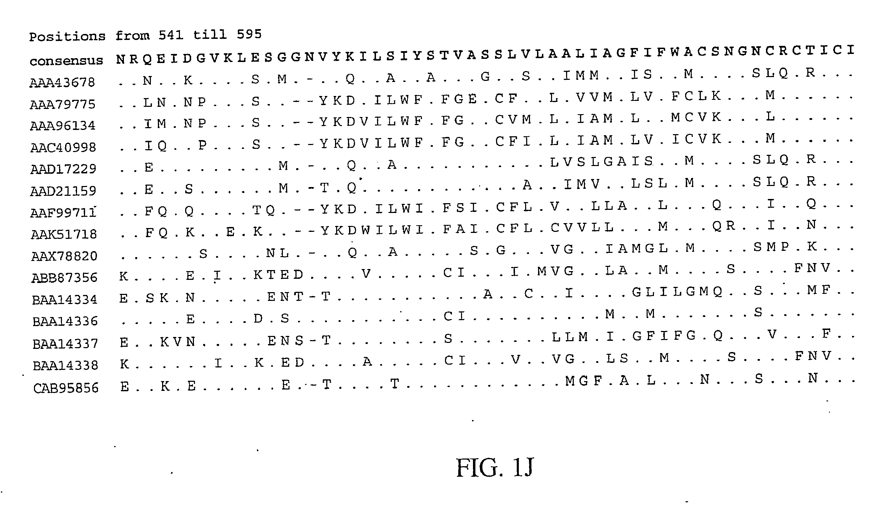

Nucleotide and amino acid sequences of influenza A viruses and their surface proteins, including hemagglutinins and neuraminidase proteins, are available from GenBank and other sequence databases, such as, for example, the Influenza Sequence Database maintained by the Theoretical Biology and Biophysics Group of Los Alamos National Laboratory. The amino acid sequences of 15 known H subtypes of the influenza A virus hemagglutinin (H1- H15) are shown in Figure 1 (SEQ ID NOS: 1-15). An additional influenza A virus hemagglutinin subtype (HI6) was isolated recently from black-headed gulls in Sweden, and reported by Fouchier et al., J. Virol. 79(5):2814-22 (2005). A large variety of strains of each H subtype are also known. For example, the sequence of the HA protein designated H5 A/Hong Kong/156/97 in Figure 1 was determined from an influenza A H5N1 virus isolated from a human in Hong Kong in May 1997, and is shown in comparison with sequences of several additional strains obtained from other related H5N1 isolates in Suarez et al., J. Virol. 72:6678-6688 (1998).

The structure of the catalytic and antigenic sites of influenza virus neuraminidase have been published by Colman et al., Nature 303:41-4 (1983), and neuraminidase sequences are available from GenBank and other sequence databases.

It has been known that virus-specific antibodies resulting from the immune response of infected individuals typically neutralize the virus via interaction with the viral hemagglutinin (Ada et al., Curr. Top. Microbiol. ImmunoL 128:1-54 (1986); Couch et al., Annu. Rev. Micobiol. 37:529-549 (1983)). The three-dimensional structures of influenza virus hemagglutinins and crystal structures of complexes between influenza virus hemagglutinins and neutralizing antibodies have also been determined and published, see, *e.g*., Wilson et al., Nature 289:366-73 (1981); Ruigrok et al., J. Gen. Virol. 69 (Pt 11):2785-95 (1988); Wrigley et al., Virology 131(2):308-14 (1983); Daniels et al., EMBO J. 6:1459-1465 (1987); and Bizebard et al., Nature 376:92-94 (2002).

According to the present invention, antibodies with the desired properties are identified from one or more antibody libraries, which can come from a variety of sources and can be of different types.

### Comprehensive human influenza antibody libraries

Comprehensive human influenza antibody libraries can be created from antibodies obtained from convalescent patients of various prior influenza, seasonal outbreaks epidemics, and pandemics, including the 1968 Hong Kong flu (H3N2), the 1957 Asian flu (H2N2), the 1918 Spanish flu (H1N1), and the 2004/2005 Avian flu (H5N1). In order to prepare such libraries, blood or bone marrow samples are collected from individuals known or suspected to have been infected with an influenza virus. Peripheral blood samples, especially from geographically distant sources, may need to be stabilized prior to transportation and use. Kits for this purpose are well known and commercially available, such as, for example, BD Vacutainer^{®} CPT™ cell preparation tubes can be used for centrifugal purification of lymphocytes, and guanidium, Trizol, or RNAlater used to stabilize the samples. Upon receipt of the stabilized lymphocytes or whole bone marrow, RT-PCR is performed to rescue heavy and light chain repertoires, using immunoglobulin oligo primers known in the art. The PCR repertoire products are combined with linker oligos to generate scFv libraries to clone directly in frame with m13 pIII protein, following procedures known in the art.

In a typical protocol, antibodies in the human sera can be detected by well known serological assays, including, for example, by the well-known hemagglutinin inhibition (HAI) assay (Kendal, A. P., M. S. Pereira, and J. J. Skehel. 1982. Concepts and procedures for laboratory-based influenza surveillance. U.S. Department of Health and Human Services, Public Health Service, Centers for Disease Control, Atlanta, Georgia), or the microneutralization assay (Harmon et al., J. Clin. Microbiol. 26:333-337 (1988)). This detection step might not be necessary if the serum sample has already been confirmed to contain influenza neutralizing antibodies. Lymphocytes from whole blood or those present in bone marrow are next processed by methods known in the art. Whole RNA is extracted by Tri BD reagent (Sigma) from fresh or RNAlater stabilized tissue. Subsequently, the isolated donor total RNA is further purified to mRNA using Oligotex purification (Qiagen). Next first strand cDNA synthesis, is generated by using random nonamer oligonucleotides and or oligo (dT)₁₈ primers according to the protocol of AccuScript reverse transcriptase (Stratagene). Briefly, 100 ng mRNA, 0.5 mM dNTPs and 300 ng random nonamers and or 500 ng oligo (dT)₁₈ primers in Accuscript RT buffer (Stratagene) are incubated at 65 °C for 5 min, followed by rapid cooling to 4 °C. Then, 100 mM DTT, Accuscript RT, and RNAse Block are added to each reaction and incubated at 42 °C for 1h, and the reverse transcriptase is inactivated by heating at 70 °C for 15 minutes. The cDNA obtained can be used as a template for RT-PCR amplification of the antibody heavy and light chain V genes, which can then be cloned into a vector, or, if phage display library is intended, into a phagemid vector. This procedure generates a repertoire of antibody heavy and light chain variable region clones (V_{H} and V_{L} libraries), which can be kept separate or combined for screening purposes.

Immunoglobulin repertoires from peripheral lymphocytes of survivors of earlier epidemics and pandemics, such as the 1918 Spanish Flu, can be retrieved, stabilized, and rescued in a manner similar to that described above. For additional H1 and H3 libraries repertoires can be recovered from properly timed vaccinated locally-sourced donors. As an additional option commercially available bone marrow total RNA or mRNA can be purchased from commercial sources to produce libraries suitable for H 1 and H3, and depending upon the background of donor also suitable for H2 antibody screening.

### Universal Antibody Library (UAL) - Synthetic Human-like Repertoire

In the methods of the present invention, the synthetic human antibody repertoire can be represented by a universal antibody library, which can be made by methods known in the art or obtained from commercial sources. Thus, for example, universal immunoglobulin libraries, including subsets of such libraries, are described in U.S. Patent Application Publication No. 20030228302 published on December 11, 2003, the entire disclosure of which is hereby expressly incorporated by reference. In brief, this patent publication describes libraries of a prototype immunoglobulin of interest, in which a single predetermined amino acid has been substituted in one or more positions in one or more complementarity-determining regions of the immunoglobulin of interest. Subsets of such libraries include mutated immunoglobulins in which the predetermined amino acid has been substituted in one or more positions in one or more of the six complementarity-determining regions of the immunoglobulin in all possible combinations. Such mutations can be generated, for example, by walk-through mutagenesis, as described in U.S. Patent Nos. 5,798,208, 5,830,650, 6,649, 340, and in U.S. Patent Application Publication No. 20030194807, the entire disclosures of which are hereby expressly incorporated by reference. In walk-through mutagenesis, a library of immunoglobulins is generated in which a single predetermined amino acid is incorporated at least once into each position of a defined region, or several defined regions, of interest in the immunoglobulin, such as into one or more complementarity determining regions (CDRs) or framework (FR) regions of the immunoglobulins. The resultant mutated immunoglobulins differ from the prototype immunoglobulin, in that they have the single predetermined amino acid incorporated into one or more positions within one or more regions (*e.g*., CDRs or FR region) of the immunoglobulin, in lieu of the "native" or "wild-type" amino acid which was present at the same position or positions in the prototype immunoglobulin. The set of mutated immunoglobulins includes individual mutated immunoglobulins for each position of the defined region of interest; thus, for each position in the defined region of interest (*e.g*., the CDR or FR) each mutated immunoglobulin has either an amino acid found in the prototype immunoglobulin, or the predetermined amino acid, and the mixture of all mutated immunoglobulins contains all possible variants.

Specific sublibraries of antibody heavy and light chains with various mutations can be combined to provide the framework constructs for the antibodies of the present invention, which is followed by introducing diversity in the CDRs of both heavy and light chains. This diversity can be achieved by methods known in the art, such as, for example, by Kunkel mutagenesis, and can be repeated several times in order to further increase diversity. Thus, for example, diversity into the heavy and light chain CDR1 and CD2 regions, separately or simultaneously, can be introduced by multiple rounds of Kunkel mutagenesis. If necessary, the various Kunkel clones can be segregated by CDR lengths and/or clones lacking diversity in a targeted CDR (*e.g*., CDR1 or CDR3) can be removed, *e.g*., by digestion with template-specific restriction enzymes. Upon completion of these steps, the size of the library should exceed about 10⁹ members, but libraries with lesser members are also useful.

In a specific embodiment, both immunized antibody libraries and universal antibody libraries are used for identifying the neutralizing antibodies of the present invention. The two types of libraries are fundamentally different. The universal antibody libraries are retrospectively synthesized collections of human-like antibodies with the predicted ability to bind proteins and peptides, while an immunized repertoire will contain sequences to specifically recognize avian H5 hemagglutinin, and/or H1, H2, or H3 hemagglutinin, as the case may be. Thus, the immunized repertoires are theoretically optimized to recognize critical components of targeted influenza subtype(s). As a result these differences the two methods produce a different set of antibodies and thus provide a more efficient approach for identifying the desired neutralizing antibodies.

### Hyperimmunized non-human primate antibody libraries

In this method, an antibody library is rescued from hyperimmunized non-human primates, such as, for example, macaque or baboons. Specifically, non-human primates are immunized with various subtypes of the influenza A virus or with various hemagglutinin (H) proteins. Animals developing titers of antibody recognizing the influenza A virus subtype or hemagglutinin they were immunized with are sacrificed and their spleens harvested. Blood or bone marrow of the immunized animals is collected, and antibodies produced are collected and amplified as described above for the comprehensive influenza antibody libraries.

### Strategies for isolating neutralizing antibodies of the invention

Regardless of the type of antibody library or libraries used, antibodies with dual specificities, such as, for example, showing reactivity with two different influenza A subtypes and/or with two strains (isolates) of the same subtype, and/or with human and non-human isolates, can be discovered and optimized through controlled cross-reactive selection and/or directed combinatorial and/or mutagenic engineering.

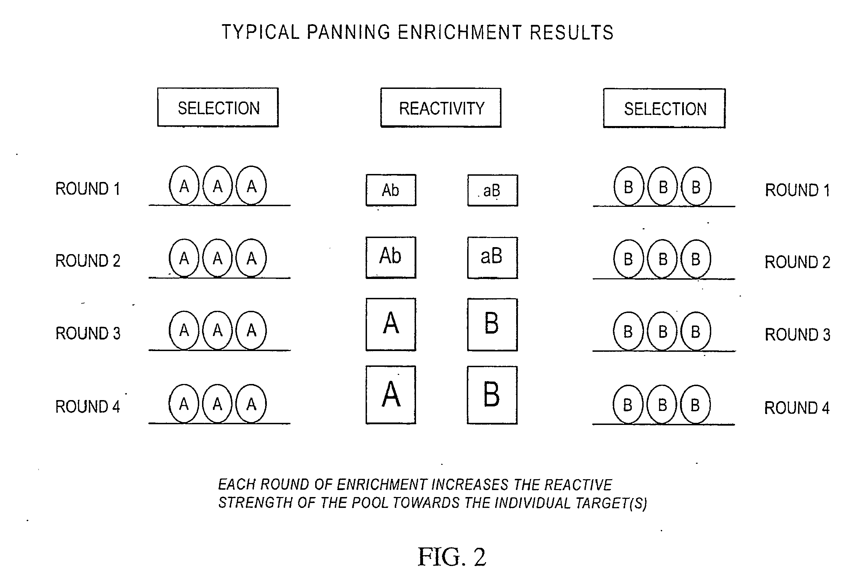

In a typical enrichment scheme, illustrated in Figure 2, a library including antibodies showing cross-reactivity to two targets, designated as targets A and B, are subjected to multiple rounds of enrichment. If enrichment is based on reactivity with target A, each round of enrichment will increase the reactive strength of the pool towards target A. Similarly, if enrichment is based on reactivity with target B, each round of enrichment will increase the reactive strength of the pool towards target B. Although Figure 2 refers to panning, which is the selection method used when screening phage display libraries (see below), the approach is equally applicable to any type of library discussed above, other otherwise known in the art, and to any type of display technique. Targets A and B include any targets to which antibodies bind, including but not limited to various isolates, types and sub-types of influenza viruses.

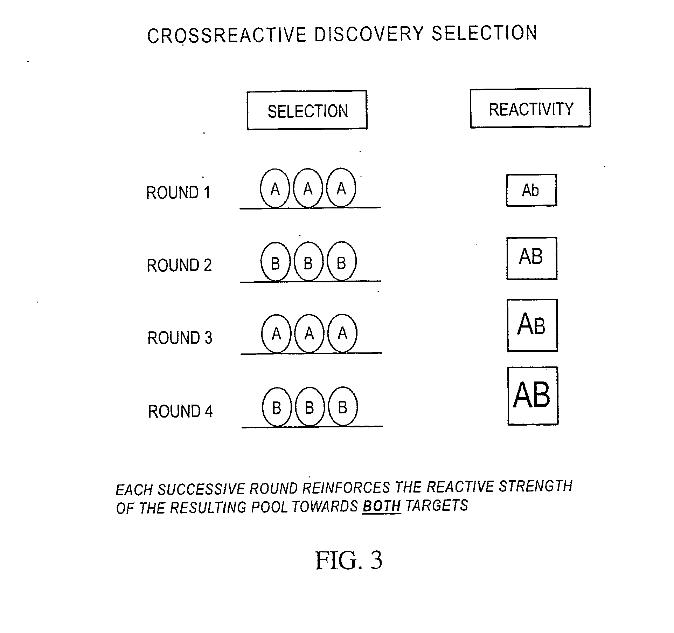

Since the goal of the present invention is to identify neutralizing antibodies with multiple specificities, a cross-reactive discovery selection scheme has been developed. In the interest of simplicity, this scheme is illustrated in Figure 3 showing the selection of antibodies with dual specificities. In this case, an antibody library including antibodies showing reactivity with two targets, targets A and B, is first selected for reactivity with one of the targets, *e.g*., target A, followed by selection for reactivity with the other target, *e.g*., target B. Each successive selection round reinforces the reactive strength of the resulting pool towards both targets. Accordingly, this method is particularly useful for identifying antibodies with dual specificity. Of course, the method can be extended to identifying antibodies showing reactivity towards further targets, by including additional rounds of enrichment towards the additional target(s). Again, if the library screened is a phage display library, selection is performed by cross-reactive panning, but other libraries and other selection methods can also be used.

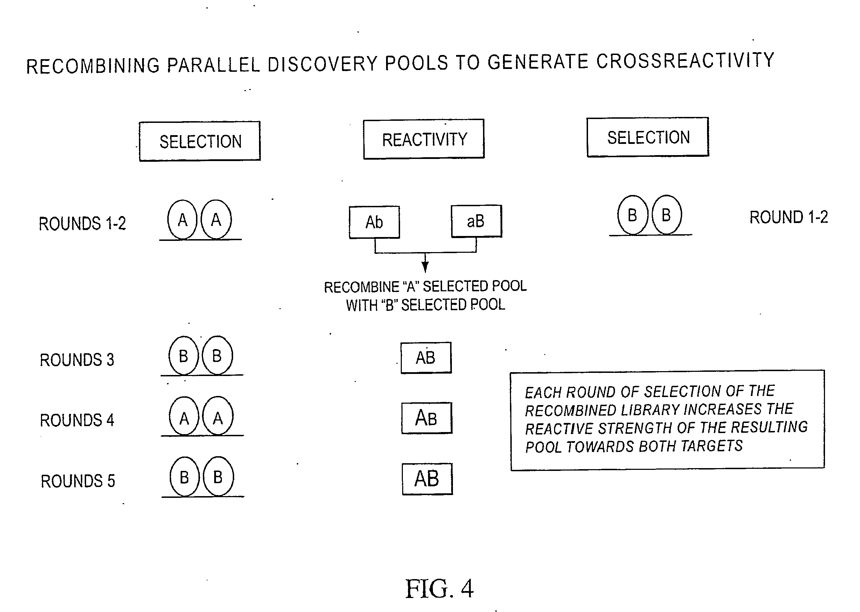

A combination of the two methods discussed above includes two separate enrichment rounds for reactivity towards target A and target B, respectively, recombining the two pools obtained, and subsequent cross-reactive selection rounds, as described above. This approach is illustrated in Figure 4. Just as in the pure cross-reactive selection, each round of selection of the recombined library increases the reactive strength of the resulting pool towards both targets.

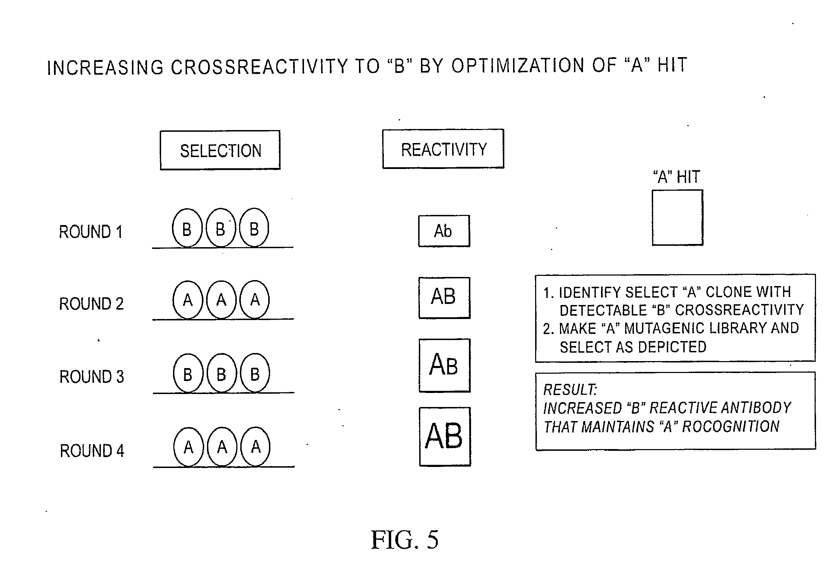

In a further embodiment, illustrated in Figure 5, first a clone showing strong reactivity with a target A, and having detectable cross-reactivity with target B is identified. Based on this clone, a mutagenic library is prepared, which is then selected, in alternating rounds, for reactivity with target B and target A respectively. This scheme will result in antibodies that maintain strong reactivity with target A, and have increased reactivity with target B. Just as before, selection is performed by panning, if the libraries screened are phage display libraries, but other libraries, other display techniques, and other selection methods can also be used, following the same strategy.

As discussed above, targets A and B can, for example, be two different subtypes of the influenza A virus, two different strains (isolates) of the same influenza A virus, subtypes or isolates from two different species, where one species is preferably human. Thus, for example, target A may be an isolate of the 2004 Vietnam isolate of the H5N1 virus, and target B may be a 1997 Hong Kong isolate of the H5N1 virus. It is emphasized that these examples are merely illustrative, and antibodies with dual and multiple specificities to any two or multiple targets can be identified, selected and optimized in an analogous manner.

Alternatively, if an antibody library such as the UAL that allows segregation of discrete frameworks and CDR lengths is used to find an antibody to target A, then an antigen B could be screened for and the library could be restricted to a diverse collection of similar parameters. Once an antibody to antigen B is found then chimeric or mutagenic antibodies based upon the respective A and B antibodies could be used to engineer a dual specific collection.

### Phage display

In a particular embodiment, the present invention utilizes phage display antibody libraries to functionally discover neutralizing monoclonal antibodies with multiple (including dual) specificities. Such antibodies can, for example, be monoclonal antibodies capable of neutralizing more than one influenza A virus subtype, including the H5, H7 and/or H9 subtypes, such as the H5 and H1; H5 and H2; H5 and H3; H5, H1, and H2; H5, H1, and H3; H5, H2 and H3; H1, H2 and H3, etc., subtypes, and/or more than one strain (isolate) of the same subtype.

To generate a phage antibody library, a cDNA library obtained from any source, including the libraries discussed above, is cloned into a phagemid vector.

Thus, for example, the collection of antibody heavy and light chain repertoires rescued from lymphocytes or bone marrow by RT-PCR as described above, is reassembled as a scFv library fused to m13 pIII protein. The combinatorial library will contain about more than 10⁶, or more than 10⁷, or more than 10⁸, or more than 10⁹ different members, more than 10⁷ different members or above being preferred. For quality control random clones are sequenced to assess overall repertoire complexity.

Similarly, following the initial PCR rescue of heavy and light chain variable regions from a naïve or immunized human, or hyperimmunized nonhman primate antibody library, the PCR products are combined with linker oligos to generate scFv libraries to clone directly in frame with M13 pIII coat protein. The library will contain about more than 10⁶, or more than 10⁷, or more than 10⁸, or more than 10⁹ different members, more than 10⁷ different members or above being preferred. As a quality control step, random clones are sequenced in order to assess overall repertoire size and complexity.

Antibody phage display libraries may contain antibodies in various formats, such as in a single-chain Fv (scFv) or Fab format. For review see, *e.g*., Hoogenboom, Methods Mol. Biol. 178:1-37 (2002).

### Screening

Screening methods for identifying antibodies with the desired neutralizing properties have been described above. Reactivity can be assessed based on direct binding to the desired hemagglutinin proteins.

### Hemagglutinin (HA) protein production

Hemagglutinin (HA) proteins can be produced by recombinant DNA technology. In this method, HA genes are cloned into an appropriate vector, preferably a baculovirus expression vector for expression in baculovirus-infected insect cells, such as *Spodoptera frugiperda* (Sf9) cells.

The nucleic acid coding for the HA protein is inserted into a baculovirus expression vector, such as Bac-to-Bac (Invitrogen), with or without a C-terminal epitope tag, such as a poly-his (hexahistidine tag). A poly-his tag provides for easy purification by nickel chelate chromatography.

In general the cloning involves making reference cDNAs by assembly PCR from individually synthesized oligos. Corresponding isolate variant HA proteins are made by either substituting appropriate mutant oligos into additional assembly PCRs or by mutagenesis techniques, such as by Kunkel mutagenesis. Two clusters of HA protein sequences exist for H5, the 1997 and 2004 subtype isolates. Therefore, a single reference protein is made for each cluster. Similarly, reference proteins are generated for 1918 Spanish flu (H1), 1958 Asian Flu (H2), 1968 Hong Kong Flu (H3), and current H1, H2, H3 isolates.

Recombinant baculovirus is generated by transfecting the above Bacmid into Sf9 cells (ATCC CRL 1711) using lipofectin (commercially available from Gibco-BRL). After 4-5 days of incubation at 28°C, the released viruses are harvested and used for further amplifications. Viral infection and protein expression are performed as described by O'Reilley et al., Baculovirus Expression Vectors: A Laboratory Manual (Oxford: Oxford University Press, 1994).

Expressed poly-His-tagged HA polypeptides can then be purified, for example, by Ni²⁺-chelate affinity chromatography as follows. Supernatents are collected from recombinant virus-infected Sf9 cells as described by Rupert et al., Nature 362:175-179 (1993). A Ni²⁺-NTA agarose column (commercially available from Qiagen) is prepared with a bed volume of 5 mL, washed with 25 mL of water, and equilibrated with 25 mL of loading buffer. The filtered cell extract is loaded onto the column at 0.5 mL per minute. The column is washed to baseline A₂₈₀ with loading buffer, at which point fraction collection is started. Next, the column is washed with a secondary wash buffer (50 mM phosphate; 300 mM NaCI, 10% glycerol, pH 6.0), which elutes non-specifically bound protein. After reaching A₂₈₀ baseline again, the column is developed with a 0 to 500 mM imidazole gradient in the secondary wash buffer. One-mL fractions are collected and analyzed by SDS-PAGE and silver staining or Western blot with Ni²⁺-NTA-conjugated to alkaline phosphatase (Qiagen). Fractions containing the eluted His₁₀-tagged HA polypeptide are pooled and dialyzed against loading buffer.

Alternatively, purification of an IgG-tagged (or Fc-tagged) HA polypeptide can be performed using known chromatography techniques, including, for instance, Protein A or protein G column chromatography.

As an alternative to using Sf9 cells HA proteins can also be produced in other recombinant host cells, prokaryote, yeast, or higher eukaryote cells. Suitable prokaryotes include but are not limited to eubacteria, such as Gram-negative or Gram-positive organisms, for example, *Enterobacteriaceae* such as *Escherichia, e.g., E. coli, Enterobacter, Erwinia,* Klebsiella, *Proteus, Salmonella, e.g., Salmonella typhimurium, Serratia, e.g., Serratia marcescans,* and *Shigella,* as well as *Bacilli* such as *B. subtilis* and *B. lichenifortnis* (*e.g., B. lichenifonnis* 41P disclosed in DD 266,710 published 12 April 1989), *Pseudomonas* such as *P. aeruginosa,* and *Streptomyces.* Various *E. coli* strains are publicly available, such as *E. coli* K12 strain MM294 (ATCC 31,446); *E*. *coli* X1776 (ATCC 31,537); *E. coli* strain W3110 (ATCC 27,325); and K5 772 (ATCC 53,635).

In addition to prokaryotes, eukaryotic microbes such as filamentous fungi or yeast are suitable cloning or expression hosts for vectors containing nucleic acid encoding an HA polypeptide. *Saccharomyces cerevisiae* is a commonly used lower eukaryotic host microorganism. However, a number of other genera, species, and strains are commonly available and useful herein, such as *Schizosaccharomyces pombe* (Beach and Nurse, Nature 290: 140 (1981); EP 139,383 published 2 May 1985); *Kluyveromyces* hosts (U.S. Patent No. 4,943,529; Fleer et al., Bio/Technology 9:968-975 (1991)) such as, *e.g., K. lactis* (MW98-8C, CBS683, CBS4574; Louvencourt et al., J. Bacterial, 737 (1983)), *K. fragilis* (ATCC 12,424), *K. bulgaricus* (ATCC 16,045), *K. wickeramii* (ATCC 24,178), *K waltii* (ATCC 56,500), *K. drosophilarum* (ATCC 36,906; Van den Berg et al., Bio/Technology 8:135 (1990)), *K. thermotolerans,* and *K. marxianus; yarrowia* (EP 402,226); *Pichia pastoris* (EP 183,070; Sreekrishna et al., J. Basic Microbiol. 28:265-278 (1988)); *Candida; Trichoderma reesia* (EP 244,234); *Neurospora crassa* (Case et al., Proc. Natl. Acad. Sci. USA 76:5259-5263 (1979)); *Schwanniomyces* such as *Schwanniomyces occidentalis* (EP 394,538 published 31 October 1990); and filamentous fungi such as, *e.g., Neurospora, Penicillium, Tolypocladium* (WO 91/00357 published 10 January 1991), and *Aspergillus* hosts such as A. *nidulans* (Ballance et al., Biochem. Biophys. Res. Commun. 112:284-289 (1983); Tilburn et al., Gene 26:205-221 (1983); Yelton et al., Proc. Natl. Acad. Sci. USA 81:1470-1474 (1984)) and A. *niger* Kelly and Hynes, EMBO J. 4:475-479 (1985). Methylotropic yeasts are suitable herein and include, but are not limited to, yeast capable of growth on methanol selected from the genera consisting of *Hansenula, Candida, Kloeckera, Pichia, Saccharomyces, Torulopsis,* and *Rhodotorula.* A list of specific species that are exemplary of this class of yeasts may be found in C. Anthony, The Biochemistry of Methylotrophs 269 (1982).

Suitable host cells for the expression of HA proteins include cells of multicellular organisms. Examples of invertebrate cells include the above-mentioned insect cells such as Drosophila S2 and Spodoptera Sf9, as well as plant cells. Examples of useful mammalian host cell lines include Chinese hamster ovary (CHO) and COS cells. More specific examples include monkey kidney CV1 line transformed by SV40 (COS-7, ATCC CRL 1651); human embryonic kidney line (HEK 293 or HEK 293 cells subcloned for growth in suspension culture (Graham et al., J. Gen Virol. 36:59 (1977)); Chinese hamster ovary cells/-DHFR (CHO, Urlaub and Chasin, Proc. Natl. Acad. Sci. USA 77:4216 ( 1980)); mouse sertoli cells (TM4, Mather, Biol. Reprod. 23:243-25 (1980)); human lung cells (W138, ATCC CCL 75); human liver cells (Hep G2, HB 8065); and mouse mammary tumor (MMT 060562, ATCC CCL51). The selection of the appropriate host cell is deemed to be within the skill in the art.

### Hemagglutinin (HA) protein panning

HA protein is immobilized on to the surface of microtiter wells or magnetic beads to pan the described above libraries. In a particular embodiment, each library is allowed to bind the H5 protein at 4 degrees for two hours and then washed extensively with cold PBS, before eluting HA specific binding clones with 0.2M glycine-HC1 buffer (pH2.5). The recovered phage is pH neutralized and amplified by infecting a susceptible host *E. coli.* Subsequently, phagemid production can be induced to repeat the enrichment of positive clones and subsequent clones isolation for triage. Upon sufficient enrichment the entire pool is transferred by infection into a non amber suppressor *E. coli* strain such as HB2151 to express soluble scFv proteins. Alternatively the pool(s) could be subcloned into a monomeric scFv expression vector, such as pBAD, and recombinant soluble scFv proteins are expressed for *in vitro* analysis and characterization, as described below.

### Characterization

H5 clones are first tested for binding affinity to an H5 protein produced as described above. In a particular example, binding is tested to a 2004 H5 protein (Refseq AAS65618, Isolate; A/Thailand/2(SP-33)/2004(H5N1)), and in parallel test to a 1997 H5 protein (Refseq AAF74331, Isolate; A/Hong Kong/486/97(H5N1)), but other isolates can also be used alone or in any combination. The positive clones obtained with the 2004 and the 1997 H5 proteins will fall into two broad categories: 2004 selective and 2004/1997 nonselective. The typical functional test for neutralization involves hemagglutination inhibition assays using whole virus binding to red blood cells. Due to safety concerns, alternative hemagglutination assays with recombinant protein and red blood cells are preferred. In order to eliminate the need for whole blood, the hemagglutinin binding inhibition assay can be preformed on airway epithelial cells. The binding assay can be performed in any configuration, including, without limitation, any flow cytometric or cell ELISA (cELISA) based assays. Using cELISA is advantageous in that it obviates the use of expensive flow cytometry equipment and can provide for more automated clonal assessment and greater data collection. On the other hand, flow cytometry may provide greater sensitivity, consistency, and speed.

H1 clones can be tested for binding to any H1 proteins, including binding to the current 2004 H1 and, in parallel, for binding to 1918 and 1976 proteins. The positive clones will fall into two broad categories: 2004 selective and 2004 nonselective. Once again it is critical to test for neutralization, using methodologies similar to those described above.

Other HA proteins, such as H2 and H3, can be characterized in an analogous manner.

### Optimization

For the efficient management of influenza epidemics and pandemics, including a potential pandemic associated with human infections caused by an avian (H5) virus, antibodies that effectively neutralize current isolates of the H proteins, such as the H5 protein, as well as future mutations, are needed. In order to achieve this goal, diverse H (e.g., H5) neutralizing clones need to be identified that bind all known isolates of the targeted hemagglutinin subtype(s).

If desired, cross-reactivity can be further improved by methods known in the art, such as, for example, by Look Through Mutagenesis (LTM), as described in US. Patent Application Publication No. 20050136428, published June 23, 2005, the entire disclosure of which is hereby expressly incorporated by reference.

Look-through mutagenesis (LTM) is a multidimensional mutagenesis method that simultaneously assesses and optimizes combinatorial mutations of selected amino acids. The process focuses on a precise distribution within one or more complementarity determining region (CDR) domains and explores the synergistic contribution of amino acid side-chain chemistry. LTM generates a positional series of single mutations within a CDR where each wild type residue is systematically substituted by one of a number of selected amino acids. Mutated CDRs are combined to generate combinatorial single-chain variable fragment (scFv) libraries of increasing complexity and size without becoming prohibitive to the quantitative display of all variants. After positive selection, clones with improved properties are sequenced, and those beneficial mutations are mapped. To identify synergistic mutations for improved HA binding properties, combinatorial libraries (combinatorial beneficial mutations, CBMs) expressing all beneficial permutations can be produced by mixed DNA probes, positively selected, and analyzed to identify a panel of optimized scFv candidates. The procedure can be performed in a similar manner with Fv and other antibody libraries.

Mutagenesis can also be performed by walk-through mutagenesis (WTM), as described above.

Another useful mutagenic method to intentionally design cross-reactivity of the antibodies herein with more than one influenza A subtype and/or more than one isolate of the same subtype, is referred herein as "destinational" mutagenesis. Destinational mutagenesis can be used to rationally engineer a collection of antibodies based upon one or more antibody clones, preferably of differing reactivities. In the context of the present invention, destinational mutagenesis is used to encode single or multiple residues defined by analogous positions on like sequences such as those in the individual CDRs of antibodies. In this case, these collections are generated using oligo degeneracy to capture the range of residues found in the comparable positions. It is expected that within this collection a continuum of specificities will exist between or even beyond those of the parental clones. The objective of destinational mutagenesis is to generate diverse multifunctional antibody collections, or libraries, between two or more discrete entities or collections. In the case of influenza this method can be utilized to use two antibodies that recognize two distinct epitopes, isolates, or subtypes and morph both functional qualities into a single antibody. As an example, a first influenza A antibody can be specific to a Vietnam isolate of the H5 subtype and a second antibody is specific to a Thailand or Turkish isolate of the H5 subtype of the influenza A virus. To create a destinational mutagenesis library, the CDR sequences for both antibodies are first attained and aligned. Next all positions of conserved identity are fixed with a single codon to the matched residue. At non-conserved positions a degenerate codon is incorporated to encode both residues. In some instances the degenerate codon will only encode the two parental residues at this position. However, in some instances additional co-products are produced. The level of co-product production can be dialed in to force co-product production or eliminate this production dependent upon size limits or goals.

Thus, for example, if the first position of the two antibodies respectively are threonine and alanine, the degenerate codon with A/G-C- in the first two positions would only encode threonine or alanine, irrespective of the base in the third position. If, for example, the next position residues are lysine and arginine the degenerate codon A-A/G-A/G will only encode lysine or arginine. However, if the degenerate codon A/C-A/G-A/G/C/T were used then asparagine, histidine, glutamine, and serine coproducts will be generated as well.

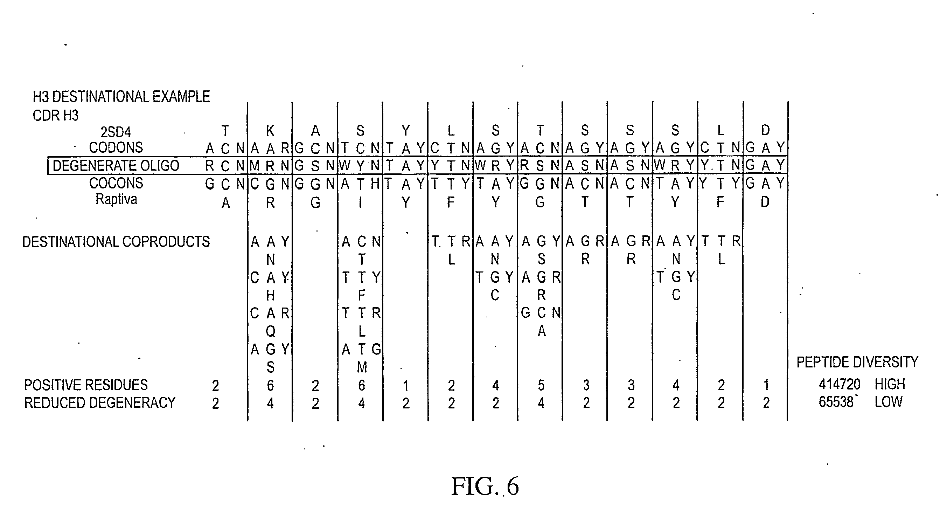

As a convenience it is simpler to use only antibodies with matched CDR lengths. One way to force this is to screen a size restricted library for the second antigen, based on the CDR length and potentially even framework restrictions imparted by the initially discovered antibody. It is noted, however, that using CDRs of equal length is only a convenience and not a requirement. It is easy to see that, while this method will be useful to create large functionally diverse libraries of influenza A virus neutralizing antibodies, its applicability is much broader. This mutagenesis technique can be used to produce functionally diverse libraries or collections of any antibody. Thus, Figure 6 is included herein to illustrate the use of the destinational mutagenesis method using CDRs of a TNF- α antibody and a CD11a antibody as the parental sequences mutagenized.

Other exemplary mutagenesis methods include saturation mutagenesis and error prone PCR.

Saturation mutagenesis (Hayashi et al., Biotechniques 17:310-315 (1994)) is a technique in which all 20 amino acids are substituted in a particular position in a protein and clones corresponding to each variant are assayed for a particular phenotype. (See, also U.S. Patent Nos. 6,171,820; 6,358,709 and 6,361,974.)

Error prone PCR (Leung et al., Technique 1:11-15 (1989); Cadwell and Joyce, PCR Method Applic. 2:28-33 (1992)) is a modified polymerase chain reaction (PCR) technique introducing random point mutations into cloned genes. The resulting PCR products can be cloned to produce random mutant libraries or transcribed directly if a T7 promoter is incorporated within the appropriate PCR primer.

Other mutagenesis techniques are also well known and described, for example, in In Vitro Mutagenesis Protocols, J. Braman, Ed., Humana Press, 2001.

In the present case, one of the main goals is to engineer an antibody (or antibodies) to effectively treat current H5 (or H7 or H9) isolates as well as future mutations. To engineer an antibody with tolerances capable of recognizing mutations in new isolates H5 neutralizing clones that bind a variety of H5 isolates, including, for example, both recent 2004 isolates and previous 1997 isolates are to be identified. It is expected that if a clone is selected on a 2004 isolate it will bind/neutralize a 1997 isolate to a lesser degree. In this case the goal is to improve 1997 recognition dramatically within the context of improving (or at least maintaining) 2004 isolate binding. Therefore, selection is first done for improvements on 1997 reference protein followed by selection on the 2004 protein. Doing so provides a greater selective pressure on the new strain, while maintaining pressure on the second parameter.

Optimization can be based on any of the libraries discussed above, or any other types of libraries known in the art, alone or in any combination. In a particular embodiment, optimization can begin by screening three types of LTM libraries; triple mutagenized light chain library, triple mutagenized heavy chain library, and hextuple mutagenized (light + heavy chain) library. H5 is panned essentially as described above, although minor modifications might be desirable. For example, prior to glycine-HCl elution one can select for improved binding by increasing washing stringencies at each round by either or both of the following methods: extensive washing at RT or 37 degrees, or prolonged incubation in presence of excess soluble parent scFv. These selection modifications should improve off-rate kinetics in the resulting clones. After 3-4 rounds of selection we will sequence random clones and test for binding by ELISA. Following sequence analysis of the improved clones, all the allowable improved mutations are combined into a combinatorial beneficial mutagenesis (CBM) library to select for synergistic improvements to binding of both subtype H5 isolates. The CBM library is made by synthesizing degenerate oligo nucleotides to represent all improved and original parental residues at all positions. The resulting library is selected under increasing stringencies, similarly to LTM screening. Following sufficient selection the pool is subcloned into a pBAD expression vector to express and purify monomeric scfv protein from *E. coli* for binding and neutralization assays, described above.

H1 neutralizing antibodies can be optimized in an analogous manner. In this case one can select and optimize using any reference protein sequences from 1918, 1976, and current as either a starting point or destination.

In addition, intertype recognition is tested with the neutralizing antibody clones. An example of intertype recognition is coincidental or engineered H1 binding from an H5 sourced or optimized clone.

Once neutralizing antibodies with the desired properties have been identified, it might be desirable to identify the dominant epitope or epitopes recognized by the majority of such antibodies. Methods for epitope mapping are well known in the art and are disclosed, for example, in Morris, Glenn E., Epitope Mapping Protocols, Totowa, N.J. ed., Humana Press, 1996; and Epitope Mapping: A Practical Approach, Westwood and Hay, eds., Oxford University Press, 2001.

The handling of antibody libraries, such as libraries from various donors or characterized by reactivity to different isolates of subtypes of a virus, including but not limited to influenza viruses, can be greatly facilitated by applying unique barcodes distinguishing the various antibody collections. The barcodes preferably are selected such that they are capable of propagating along with the clone(s) labeled.

Thus the barcodes can be non-coding DNA sequences of about 1-24 non-coding nucleotides in length that can be deconvoluted by sequencing or specific PCR primers. This way, a collection of nucleic acids, such as an antibody repertoire, can be linked at the cloning step.

In another example, the barcodes are coding sequences of silent mutations. If the libraries utilize restrition enzymes that recognize interrupted palidromes (e.g. Sfi GGCCNNNNNGGCC), distinct nucleotides can be incorporated in place of the "N's" to distinguish various collections of clones, such as antibody libraries. This barcoding approach has the advantage that the repertoire is linked at the amplification step.

In a different example, the barcodes are coding sequences that encode immunologically distinct peptide or protein sequences fused to phage particles. Examples include, for example, epitope (e.g. Myc, HA, FLAG) fusions to pIII, pVIII, pVII, or pIX phages. The epitopes can be used singly or in various combinations, and can be provided in cis (on the library-encoding plasmid) or in trans (specifically modified helper phage) configuration.

Other examples of possible barcodes include, without limitation, chemical and enzymatic phage modifications (for phage libraries) with haptens or fluorescent chromophores. Such tags are preferred for a single round of selection.

While barcoding is illustrated herein for distinguishing antibody libraries, one of ordinary skill will appreciate that the described approaches are broadly applicable for uniquely labeling and distinguishing nucleic acid molecules and collections of nucleic acids in general.

### Production of neutralizing antibodies

Once antibodies with the desired neutralizing properties are identified, such antibodies, including antibody fragments can be produced by methods well known in the art, including, for example, hybridoma techniques or recombinant DNA technology.

In the hybridoma method, a mouse or other appropriate host animal, such as a hamster, is immunized to elicit lymphocytes that produce or are capable of producing antibodies that will specifically bind to the protein used for immunization. Alternatively, lymphocytes may be immunized in vitro. Lymphocytes then are fused with myeloma cells using a suitable fusing agent, such as polyethylene glycol, to form a hybridoma cell (Goding, Monoclonal Antibodies: Principles and Practice, pp.59-103 (Academic Press, 1986)).

The hybridoma cells thus prepared are seeded and grown in a suitable culture medium that preferably contains one or more substances that inhibit the growth or survival of the unfused, parental myeloma cells. For example, if the parental myeloma cells lack the enzyme hypoxanthine guanine phosphoribosyl transferase (HGPRT or HPRT), the culture medium for the hybridomas typically will include hypoxanthine, aminopterin, and thymidine (HAT medium), which substances prevent the growth of HGPRT-deficient cells.

Preferred myeloma cells are those that fuse efficiently, support stable high-level production of antibody by the selected antibody-producing cells, and are sensitive to a medium such as HAT medium. Among these, preferred myeloma cell lines are murine myeloma lines, such as those derived from MOPC-21 and MPC-11 mouse tumors available from the Salk Institute Cell Distribution Center, San Diego, California USA, and SP-2 or X63-Ag8-653 cells available from the American Type Culture Collection, Rockville, Maryland USA. Human myeloma and mouse-human heteromyeloma cell lines also have been described for the production of human monoclonal antibodies (Kozbor, J. Immunol. 133:3001 (1984); and Brodeur et al., Monoclonal Antibody Production Techniques and Applications, pp. 51-63 (Marcel Dekker, Inc., New York, 1987)).

Culture medium in which hybridoma cells are growing is assayed for production of monoclonal antibodies directed against the antigen. Preferably, the binding specificity of monoclonal antibodies produced by hybridoma cells is determined by immunoprecipitation or by an in vitro binding assay, such as radioimmunoassay (RIA) or enzyme-linked immunoabsorbent assay (ELISA).

Recombinant monoclonal antibodies can, for example, be produced by isolating the DNA encoding the required antibody chains and co-transfecting a recombinant host cell with the coding sequences for co-expression, using well known recombinant expression vectors. Recombinant host cells can be prokaryotic and eukaryotic cells, such as those described above.

The choice of human variable domains, both light and heavy, to be used in making the humanized antibodies is very important to reduce antigenicity. According to the so-called "best-fit" method, the sequence of the variable domain of a rodent antibody is screened against the entire library of known human variable-domain sequences. The human sequence which is closest to that of the rodent is then accepted as the human framework region (FR) for the humanized antibody (Sims et al., J. Immunol. 151:2296 (1993); Chothia et al., J. Mol. Biol. 196:901 (1987)). It is important that antibodies be humanized with retention of high affinity for the antigen and other favorable biological properties. To achieve this goal, according to a preferred method, humanized antibodies are prepared by a process of analysis of the parental sequences and various conceptual humanized products using three-dimensional models of the parental and humanized sequences.

In addition, human antibodies can be generated following methods known in the art. For example, transgenic animals (e.g., mice) can be made that are capable, upon immunization, of producing a full repertoire of human antibodies in the absence of endogenous immunoglobulin production. See, e.g., Jakobovits et al., Proc. Natl. Acad. Sci. USA 90:2551 (1993); Jakobovits et al., Nature 362:255-258 (1993); Bruggermann et al., Year in Immuno. 7:33 (1993); and U.S. Patent Nos. 5,591,669, 5,589,369 and 5,545,807.

### Use of neutralizing antibodies

The influenza neutralizing antibodies of the present invention can be used for the prevention and/or treatment of influenza type A infections. For therapeutic applications, the antibodies or other molecules, the delivery of which is facilitated by using the antibodies or antibody-based transport sequences, are usually used in the form of pharmaceutical compositions. Techniques and formulations generally may be found in Remington's Pharmaceutical Sciences, 18th Edition, Mack Publishing Co. (Easton, Pa. 1990). See also, Wang and Hanson "Parenteral Formulations of Proteins and Peptides: Stability and Stabilizers," Journal of Parenteral Science and Technology, Technical Report No. 10, Supp. 42-2S (1988).

Antibodies are typically formulated in the form of lyophilized formulations or aqueous solutions. Acceptable carriers, excipients, or stabilizers are nontoxic to recipients at the dosages and concentrations employed, and include buffers such as phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (*e*.*g*., Zn-protein complexes); and/or non-ionic surfactants such as TWEEN™, PLURONICS™ or polyethylene glycol (PEG).

The antibodies also may be entrapped in microcapsules prepared, for example, by coacervation techniques or by interfacial polymerization (for example, hydroxymethylcellulose or gelatin-microcapsules and poly-(methylmethacylate) microcapsules, respectively), in colloidal drug delivery systems (for example, liposomes, albumin microspheres, microemulsions, nano-particles and nanocapsules), or in macroemulsions. Such techniques are disclosed in Remington's Pharmaceutical Sciences, *supra*.

The neutralizing antibodies disclosed herein may also be formulated as immunoliposomes. Liposomes containing the antibody are prepared by methods known in the art, such as described in Epstein et al., Proc. Natl. Acad. Sci. USA 82:3688 (1985); Hwang et al., Proc. Natl Acad. Sci. USA 77:4030 (1980); U.S. Patent Nos. 4,485,045 and 4,544,545; and WIO97138731 published October 23, 1997. Liposomes with enhanced circulation time are disclosed in U.S. Patent No. 5,013,556.

Particularly useful liposomes can be generated by the reverse phase evaporation method with a lipid composition comprising phosphatidylcholine, cholesterol and PEG-derivatized phosphatidylethanolamine (PEG-PE). Liposomes are extruded through filters of defined pore size to yield liposomes with the desired diameter. Fab' fragments of the antibody of the present invention can be conjugated to the liposomes as described in Martin et al. J. Biol. Chem. 257:286-288 (1982) via a disulfide interchange reaction. A chemotherapeutic agent is optionally contained within the liposome. See Gabizon et al. J. National Cancer Inst. 81(19)1484 (1989).

For the prevention or treatment of disease, the appropriate dosage of antibody will depend on the type of infection to be treated the severity and course of the disease, and whether the antibody is administered for preventive or therapeutic purposes. The antibody is suitably administered to the patient at one time or over a series of treatments. Depending on the type and severity of the disease, about 1 µg/kg to about 15 mg/kg of antibody is a typical initial candidate dosage for administration to the patient, whether, for example, by one or more separate administrations, or by continuous infusion.

Further details of the invention are illustrated by the following nonlimiting Example.

### Example

### Antibody Libraries from Survivors of Prior Bird Flu Outbreaks and Preparation of Neutralizing Antibodies

### Materials and Methods

### Bone Marrow Protocol and Sera Preparation

Blood was obtained by standard venopuncture, allowed to clot, and processed to recover serum. The serum was stored at -20 °C for 3-4 days until they were shipped on dry ice. Donors were anaesthetized with an injection of a local anesthetic and 5ml of bone marrow was removed from the pelvic bone of each H5N1 survivor. Next the 5ml of bone marrow was placed into a sterile 50-ml tube containing 45 ml RNAlater (Ambion). The mixture was gently inverted approximately 8-20 times, until there were no visible clumps and the marrow and RNAlater were mixed well. Next the specimen was refrigerated the between 2-10 °C overnight. Following the overnight refrigeration, the specimens were stored at -20 °C for 3-4 days until they were shipped on dry ice. Upon receipt the RNAlater/marrow and sera containing tubes were stored at -80 °C until processed.

### Serology: HA ELISA

ELISA plates (Thermo, Immulon 4HBX 96W) were coated with 100 µl of 100 ng/mL H5 hemagglutinin (Protein Sciences, A/Vietnam/1203/2004) in 1X ELISA Plate Coating Solution (BioFX) by overnight incubation at room temperature. The next day plates were washed three times with 300 µl PBS/ 0.05% Tween-20 (PBST). Following the wash, 300 µl of a blocking solution (4% Non-Fat dry Milk in PBS/ 0.05% Tween-20) was added and incubated for 1 hour at RT. Following the blocking step, the plates were washed three times with 300 µl PBS/ 0.05% Tween-20. Next, 100 µl serum samples diluted 1:20,000 in PBS/ 0.05% Tween were incubated for 1-2 hours at RT and then washed three times with 300 µl PBS/ 0.05% Tween-20. 100 µl of an anti-human Fc-HRP conjugate diluted 1:5,000 in PBS/ 0.05% Tween was incubated for 1-2 hours at RT and then washed three times with 300 µl PBS/ 0.05% Tween-20. Following this final wash, 100 µl of chromogenic substrate solution was added (TMB1 Substrate, BioFx) and after sufficient amount of time terminated by the addition of 100 µl of STOP Solution (BioFx). Absorbances at 450nm were read on a plate reader (Molecular Devices Thermomax microplate reader with Softmax Pro software), data recorded, and subsequently plotted using Excel (Microsoft).

### Bane Marrow: RNA Extraction and mRNA Purification

Bone marrow (-2.5 ml in 20 ml RNA later), previously stored at -80 °C, was recovered by centrifugation to remove RNA later and then resuspended in 11.25 ml TRI BD reagent (Sigma) containing 300 µl Acetic Acid. The pellet was then vortexed vigorously. Next 1.5 ml BCP (1-bromo-3-chloropropane, Sigma) was added, mixed by vortexing, incubated at RT for 5 min, and then centrifuged at 12000 x g for 15 min at 4°C. The aqueous phase was carefully removed to not disturb the interface. Total RNA from the aqueous phase was next precipitated by addition of 25 ml isopropanol, incubation at RT for 10 minutes, and centrifugation at 12000 x g for 10 min at 4°C. Following the addition of isopropanol, two phases were formed due to residual RNAlater, resulting in the precipitated RNA settling at the interface. To eliminate the residual RNAlater and allow maximal recovery of RNA, 5 ml aliquots of 50% isopropanol in H₂0 were added and mixed until no phase separation was noticeable, at which point the RNA was pelleted by centrifugation at 12000 x g for 10 min at 4°C. The RNA pellet was washed with 75% EtOH, transferred to an RNAse-free 1.6 ml microcentrifuge tube, and again recovered by centrifugation. Finally the RNA pellet was resuspended in 100 µl 1mM Na-phosphate, pH 8.2 and the A₂₆₀ and A₂₈₀ were read to assess RNA purity.

Prior to reverse transcription mRNA was purified from total RNA according to Qiagen Oligotex mRNA purification kit. Briefly, 50-200 µg bone marrow RNA was brought to 250 µl with RNase-free water and mixed with 250 µl of OBB buffer and Oligotex suspension followed by incubation for 3 min at 70°C. Hybridization between the oligo dT₃₀ of the Oligotex particle and the mRNA poly-A-tail was carried out at room temperature for 10 min. The hybridized suspensions were then transferred to a spin column and centrifuged for 1 min. The spin column was washed twice with 400 µl Buffer OW2. Purified mRNA was then eluted twice by centrifugation with 20 µl hot (70°C) Buffer OEB. Typical yields were 500 ng to 1.5 µg total RNA.

### Reverse transcription using N9 and Oligo dT on bone marrow mRNA

Reverse transcription (RT) reactions were accomplished by mixing together 75-100 ng mRNA with 2 µl 10X Accuscript RT Buffer (Stratagene), 0.8 µl 100 mM dNTPs, and either N9 (300 ng) or oligo dT primer (100 ng) and then brought to a final volume of 17 µl with water. The mixtures were heated at 65°C for 5 min, and then allowed to cool to room temperature. Next 2 µl DTT, 0.5 µl RNase Block (Stratagene), 0.5 µl AccuScript RT (Stratagene) were added to each reaction. Next, the N9 primed reactions were incubated for 10 minutes at room temperature and the oligo-dT primed reactions were incubated on ice for 10 minutes. Finally, both reactions were incubated at 42°C for 60 minutes followed by 70°C for 15 minutes to kill the enzyme.

### PCR from bone marrow-derived cDNA

Antibody heavy and light chain repertoires were amplified from bone marrow cDNA essentially using previously described methods and degenerate primers (O'Brien, P.M., Aitken R. Standard protocols for the construction of scFv Libraries. Antibody Phage Display - Methods and Protocols, vol 178, 59-71, 2001, Humana Press) based upon human germline V and J regions.

Briefly, PCR reactions using Oligo dT primed cDNA (from 75 ng mRNA) for lambda light chains and N9 primed cDNA (from 75 ng mRNA for kappa light chains, from 100 ng mRNA for heavy chains) were mixed together with 5 µl10X amplification buffer (Invitrogen), 1.5 µl dNTPs (10 mM), 1 µl MgSO4 (50 mM), 2.5 µl V_{regioo} primers (10 uM) and 2.5 µl J_{region} primers (10 uM) -10 uM for V_{H}, 0.5 µl Platinum Pfx Polymerase (Invitrogen), and sterile dH₂O to final volume of 50 µl. PCR parameters were as follows: step 1-95°C 5 minutes, step 2- 95°C 30 seconds, step 3-58°C 30 seconds, step 4- 68°C 1 minute, step 5- cycle step 2-4 40 times, step 6- 68°C 5 minutes. Light chain PCR products were cleaned up using Qiagen PCR Cleanup kit. Heavy chains PCR products were gel purified from 1.5% agarose gel using Qiagen Gel Extraction Kit and then reamplified. Heavy chain reamplification was carried out as follows: Mixed 10 µl 10X amplification buffer (Invitrogen), 3 µl dNTPs (10mM), 2 µl MgSO4 (50 mM), 5 µl each V_{H} primers (10 uM) and J_{H} primers (10 uM), 5 µl Heavy chain Primary PCR product, 1 µl Platinum Pfx, volume adjusted to 100 µl with water. Cycling parameters were as follows: step 1-95°C 5 minutes, step 2- 95°C 30 seconds, step 3-58°C 30 seconds, step 4- 68°C 1 minute, step 5- cycle step 2-4 20 times, step 6- 68°C 5 minutes. Reamplified heavy chain PCR products were cleaned up from a 1.5% agarose-TAE gel using Qiagen Extraction Kit.

### Antibody phage library construction

Separate antibody libraries for each individual bird flu survivor were constructed using unique identifying 3-nucleotide barcodes inserted in the untranslated region following the terminal pIII stop codon.

### Light Chain cloning:

1 µg each of pooled kappa light chain and pooled lambda light chain per donor were digested with NotI and BamHI and gel purified from a 1.5% agarose-TAE gel using Qiagen Gel Extraction Kit. 5 µg of each vector was digested with NotI and BamHI and gel purified from a 1% agarose-TAE gel using Qiagen Gel Extraction Kit. Library ligations were performed with 200 ng of gel purified Kappa or Lambda inserts and 1 µg of gel purified vector in 60 µl for 1 hour at RT or overnight at 14°C. Ligations were desalted using Edge BioSystem Perfroma spin columns. The library was transformed in five electroporations in 80 µl TG-1 or XL-1 Blue aliquots, each recovered in 1 ml SOC, pooled and outgrown for one hour at 37°C. Total number of transformants was determined following this outgrowth by plating an aliquot from each of the transformations. The remaining electroporation was amplified by growing overnight at 37°C in 200 ml 2YT + 50 µg/ml Ampicillin + 2% glucose. The subsequent light chain library was recovered by plasmid purification from these overnight cultures using a Qiagen High Speed Maxiprep Kit.

### Heavy Chain Cloning:

1.5-2 µg each of the donor-specific heavy chains (V_{H}1, V_{H} 2, 5, 6 pool, V_{H} 3, and V_{H} 4) were digested with a 40 Unit excess/µg DNA with SfiI and XhoI and gel purified from a 1.5% agarose-TAE gel using Qiagen Gel Extraction Kit. 15 µg of each light chain library vector was digested with 40 Unit/µg DNA with SfiI and XhoI and gel purified from a 1% agarose-TAE gel using Qiagen Gel Extraction Kit. Library ligations were set up by combining 1.2 µg SfiI/XhoI digested, gel purified heavy chain donor collections and 5 µg of each light chain library (kappa and lambda) overnight at 14°C. The library ligations were then desalted with Edge BioSystem Pefroma spin columns and then transformed through 20 electroporations per library in 80 µl TG-1 aliquots, each recovered in 1 ml SOC, pooled and outgrown for one hour at 37°C. Again following this outgrowth an aliquot of each was used to determine the total number of transformants with the remainder transferred to 1L 2YT + 50 µg/ml Ampicillin + 2% glucose and grown at 37C with vigorous aeration to an OD₆₀₀ of ∼0.3. Next M13K07 helper phage was then added at a multiplicity of infection (MOI) of 5:1 and incubated for 1 hour at 37°C, with no agitation. Next the cells were harvested by centrifugation and resuspended in 1L 2YT + 50 µg/ml Ampicillin, 70 µg/ml Kanamycin and grown overnight at 37°C with vigorous aeration to allow for scFv phagemid production. The next morning the cells were collected by centrifugation and supernatant containing phagemid was collected. The phagemids were precipitated from the supernatant by the addition of 0.2 volumes 20% PEG/5 M NaCl solution and incubation for 1 hour on ice. The phagemid library stocks were then harvested by centrifugation and resuspended in 20 ml sterile PBS. Residual bacteria were removed by an additional centrifugation and the final phagemid libraries were stored at -20°C in PBS+50% glycerol.

### Phagemid panning and amplification

ELISA plates (Immulon 4HBX flat bottom, Nunc) were coated with 100 µl of 100 ng/mL H5 hemagglutinin protein(Protein Sciences, A/Vietnam/1203/2004) in ELISA Coating Solution (BioFX) by overnight incubation at room temperature. The next day plates were washed three times with 300 µl ST. Following the wash, 300 µl of a blocking solution (4% Non-Fat dry Milk in PBS/ 0.05% Tween-20) was added and incubated for 30 mins on ice. Following the blocking step, the plates were washed three times with 300 µl PBST. Just prior to phage panning, the glycerol was removed from the frozen phagemid stocks using Millipore Amicon Ultra columns and then blocked in 4% nonfat dry milk for 15 minutes. Next, 100 µl aliquots of phagemid were distributed into 8 wells (total phage ∼1x10¹² CFU) and incubated for 2 hours at 4°C followed by washing 6-8 times with 300 µl PBST. Phagemid were collected following a 10 min at room temperature in 100 µl/well Elution buffer (0.2M glycine-HCl, pH 2.2, 1 mg/ml BSA). The eluate was then neutralized by the addition of 56.25 µl 2M Tris base per ml eluate. Following neutralization, 5 ml TG1 cells (OD₆₀₀ ∼0.3) were infected with 0.5 ml neutralized phage at 37°C for 30 minutes in 2-YT with no shaking. Following this step some cells were plated onto LB AMP Glucose plates to determine total phagemid recovery. The remaining inoculum was placed into 10 ml 2-YTAG (final concentration 2% glucose and 50ug/ml ampicillin) and grown at 37°C with vigorous aeration to OD₆₀₀ ∼0.3. Next the cultures were infected with M13K07 helper phage at an MOI of 5:1 and incubated at 37°C for 30-60 minutes with no shaking. The cells were collected by centrifugation and resuspended in 25ml 2-YTAK (Ampicillin 50 µg/ml, Kanamycin 70 µg/ml), transferred to a fresh culture flask, and grown ON at 37°C with shaking. Subsequent rounds were similarly recovered and amplified.

### scFv ELISA

Individual colonies of *E. coli* HB2151 transformed cells from biopanned phage were grown overnight at 37°C in 1 ml of 2YT+ 100 µg/ml AMP. The following morning the cells were harvested by centrifugation and resuspended in 1.5 ml periplasmic lysis buffer (1ml BBS (Teknova) + 0.5 ml 10mg/ml lysozyme + EDTA to 10 mM final concentration). The cells were again pelleted by centrifugation and the scFv containing periplasmic lysates were collected. The scFv lysates were combined 1:1 with dilution buffer (PBS/0.05% BSA) and 100 µl was added to wells that had been previously antigen coated with and blocked with dilution buffer. The samples were incubated for 2 hours at room temperature and then washed three times with PBS/ 0.05% Tween. Next 100 µl of 1:5000 diluted Biotin Anti-Histidine mouse (Serotec) in dilution buffer was added to each well and incubated for 1 hr at room temperature. Following this incubation the wells were washed three times with PBS/ 0.05% Tween and then to each well 100 µl of 1:2500 Streptavidin:HRP (Serotec) was added and incubated for 1 hr at room temperature and then washed three times with PBS/ 0.05% Tween. Following this final wash, 100 µl of chromogenic substrate solution was added (TMB1 Substrate, BioFx) and after sufficient amount of time terminated by the addition of 100 µl of STOP Solution (BioFx). Absorbances at 450nm were read on a plate reader (Molecular Devices Thermomax microplate reader with Softmax Pro software), data recorded, and subsequently plotted using Excel (Microsoft).

### Sequencing

To deduce the heavy and light chain sequences, individual clones were grown and plasmid DNA extracted (Qiagen). The plasmid DNA was subjected to standard DNA sequencing.

### Hemagglutinin Inhibition (HAI) Assays

Hemagglutination Inhibition was performed essentially following the method of Rogers et a/., Virology 131:394-408 (1983), in round bottom microtiter plates (Coming) using 4 HAU (hemagglutinating units) of virus or protein/well. For HAI determinations 25 µl samples of purified single chain variable fragments (scFv) were mixed with 25 µl of PBS containing 4 HAU of the test virus in each microtiter well. Following a preincubation of 15 minutes at room temperature, 25 µl of 0.75% human erythrocytes were added, and mixed. HAI antibody activity was determined by visual inspection following a 60 min incubation at room temperature.

### Results

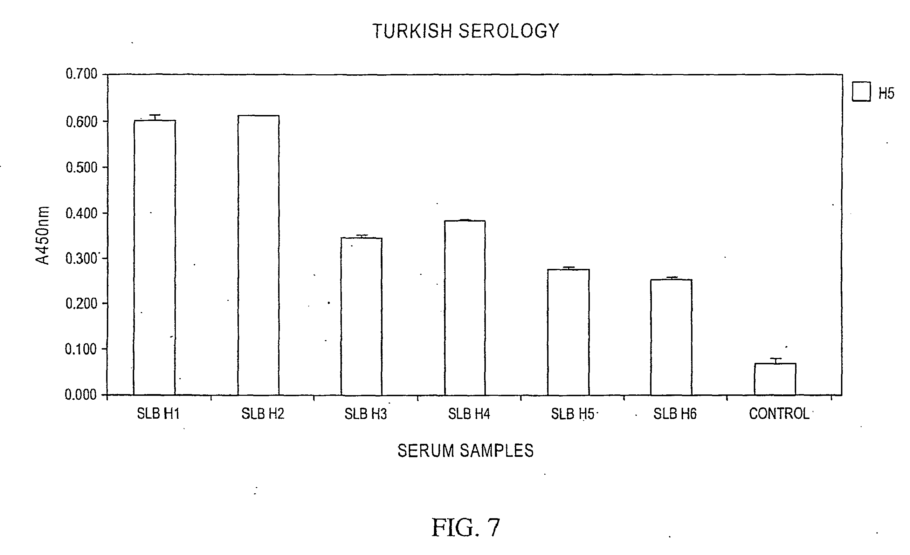

Bone marrow and blood samples were collected from six survivors of the H5N1 bird flu outbreak that had taken place in Turkey in January 2006, approximately four months after the outbreak. For all six survivors the initial diagnosis of bird flu was made following by physical examination, clinical laboratory testing, and molecular diagnostic determination, sanctioned by the Turkish Ministry of Health. Four of these survivors were additionally confirmed by the World Health Organization (WHO). Serum samples were analyzed to confirm the presence of antibodies to H5 hemagglutinin (A/Vietnam/1203/2004) using the serology protocol described above. As shown in Figure 7, the blood samples of all six patients (designated SLB H1-H6, respectively) demonstrated the presence of antibodies to the H5 antigen. Following this confirmation, RNA was extracted from the bone marrow samples of these individuals, and bone marrow mRNA was purified and reverse transcribed using the protocols described above. The antibody heavy and light chain repertoires were then amplified from the bone marrow cDNA as described above, and individual antibody heavy and light chain phage libraries were cloned separately for each survivor, using the above-described three-nucleotide bar coding to distinguish the individual libraries.

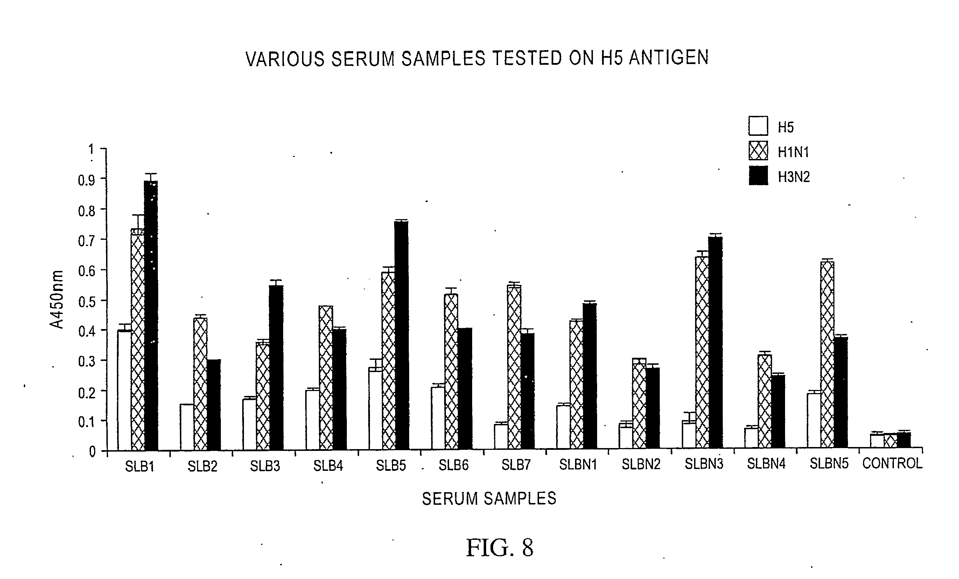

Bone marrow and blood samples were also collected from twelve local donors who were treated for flu symptoms in the year of 2006. Serology was performed as described above to confirm the presence of antibodies to H1, H3 and H5 hemagglutinin, respectively. As shown in Figure 8, all serum samples tested positive for antibodies to H1 and/or H3 hemagglutinins, where the dominance of a certain subtype depended on the influenza A virus subtype to which the particular donor was exposed most throughout his or her lifetime. Interestingly, there were donors whose serum contained a significant level of antibodies of H5 hemagglutinin as well (donors SLB 1 and SLB5 in Figure 8). Following this confirmation, RNA was extracted from the bone marrow samples of the donors, and bone marrow mRNA was purified and reverse transcribed using the protocols described above. The antibody heavy and light chain repertoires were then amplified from the bone marrow cDNA as described above, and individual antibody heavy and light chain phage libraries were cloned separately for each donor, using the above-described three-nucleotide bar coding to distinguish the individual libraries.

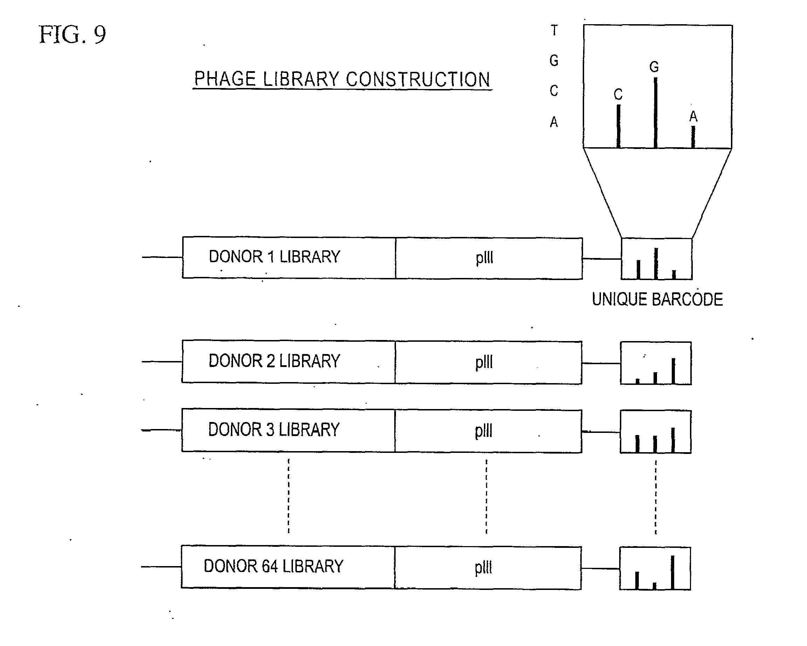

As illustrated in Figure 9, using three of the available four nucleotides allows the creation of 64 unique barcodes.

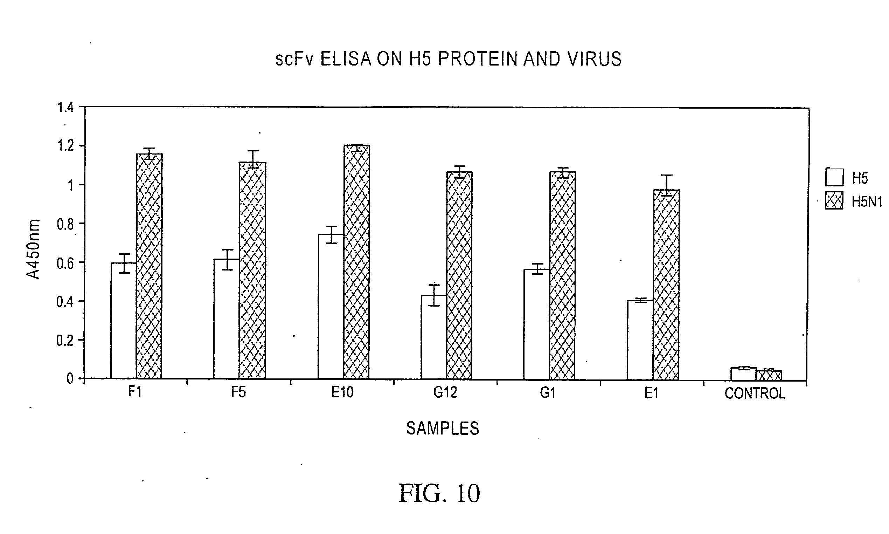

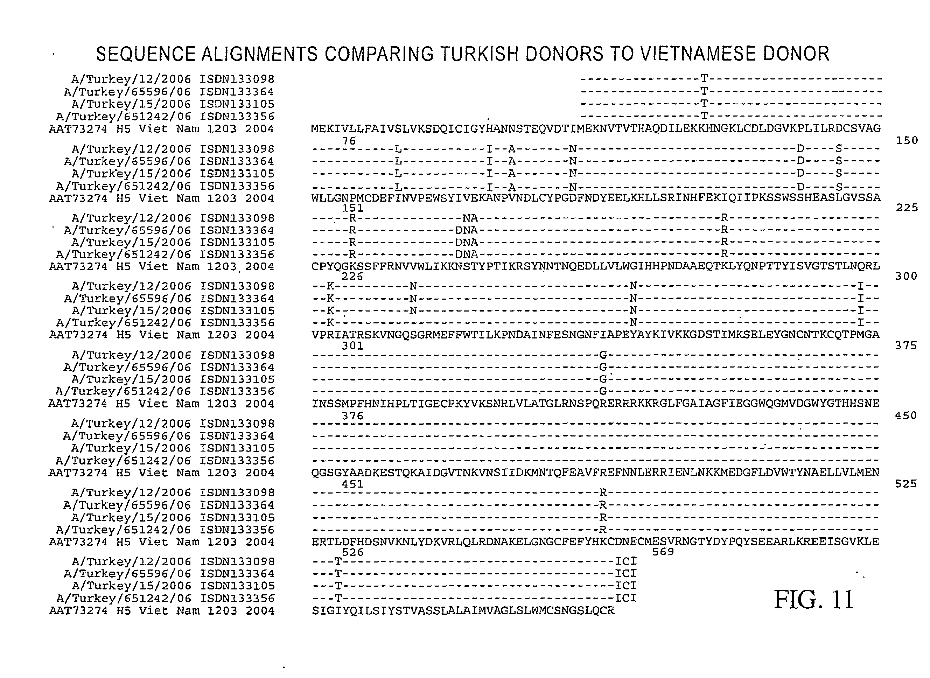

Out of 48 random clones obtained after three rounds of panning of pooled antibody libraries prepared from the bone marrow samples of Turkish bird flu survivors, 40 were tested by ELISA for binding to the H5 hemagglutinin protein (Protein Sciences, A/Vietnam/1203/2004), and to inactivated Vietnamese H5N1 virus (CBER, A/Vietnam/1203/2004). The clones were sequenced. Of the 40 clones, five were found to be different. As shown in Figure 10, all five distinct clones (clones F5 and G1 have the same sequences) were binding both to the H5 protein and the Vietnamese H5N1 virus. Figure 11 shows sequence alignments comparing the sequences of H5 hemagglutinin proteins from Turkish donors to the H5 hemagglutinin sequence of the Vietnamese isolate used in the above experiments. The results of these experiments show that, despite differences in the sequences, the antibodies tested bound both the Turkish and the Vietnamese H5 proteins and viruses, and thus showed cross-reactivity with more than one isolate of the H5N1 virus.

Four additional unique clones were identified from among 12 clones produced by the second round of panning.

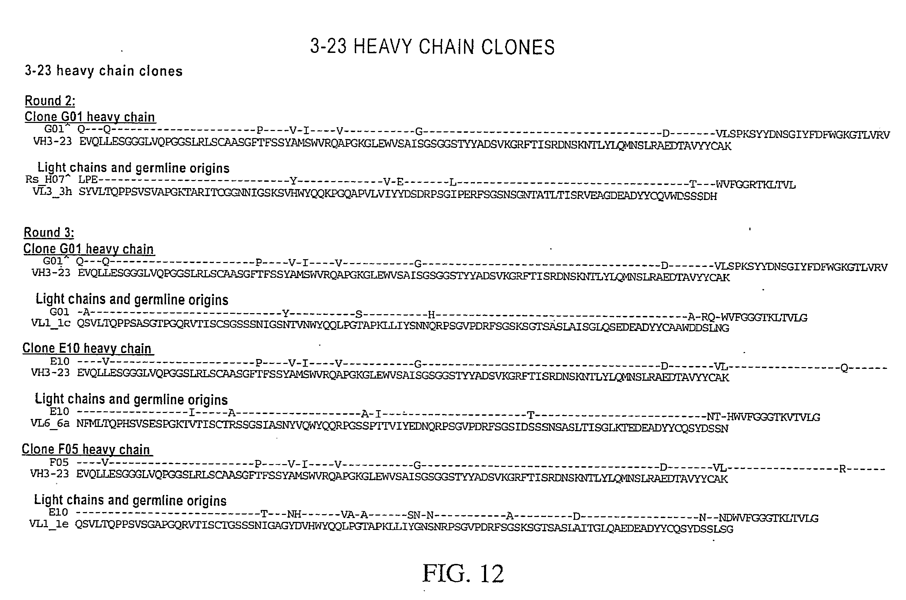

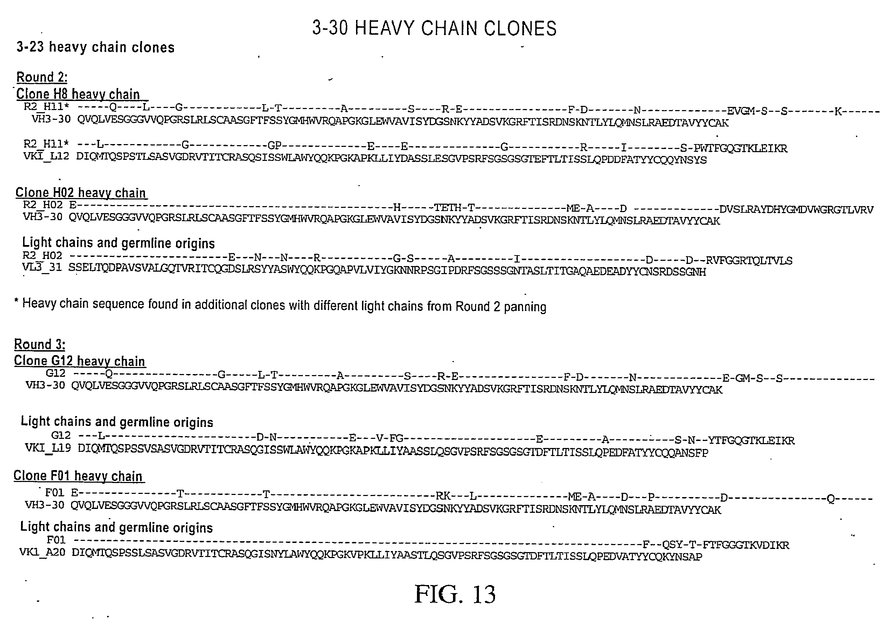

The heavy chain variable region sequences of the unique clones identified in the pooled antibody libraries of Turkish donors, along with the corresponding light chain and germline origin sequences, are shown in Figure 12 and 13. In particular, the sequences shown in Figure 12 (3-23 heavy chain clones) originate from a pooled library of all heavy and light chains of all Turkish donors after three rounds of panning. The sequences shown in Figure 13 (3-30 heavy chain clones) originate from a pooled library of all heavy and light chains of all Turkish donors after two rounds of panning.

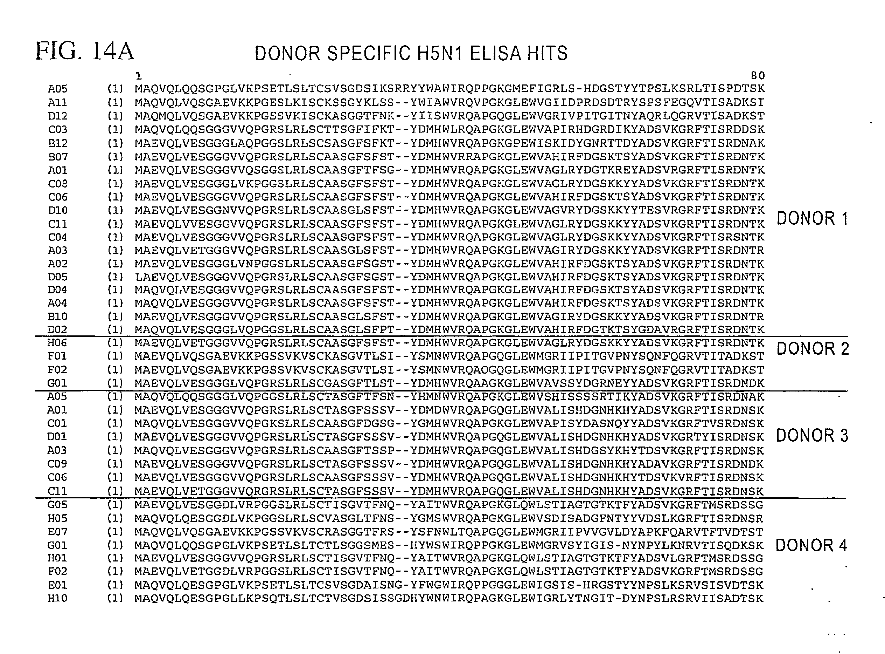

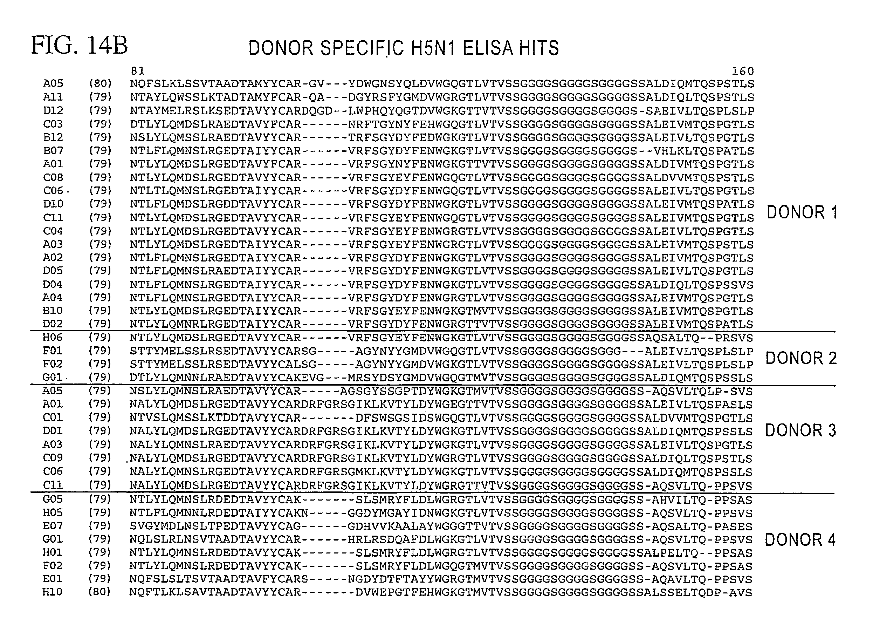

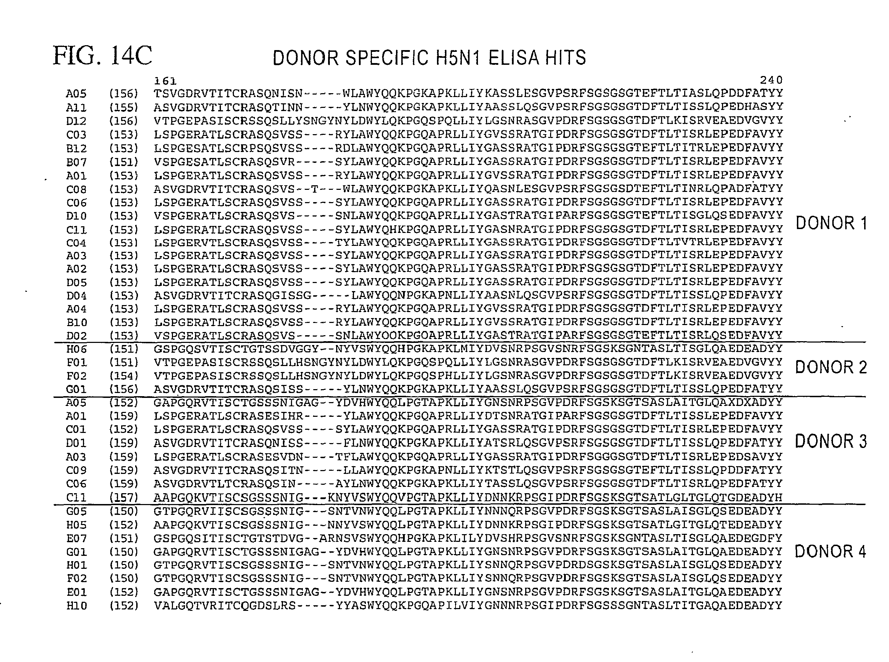

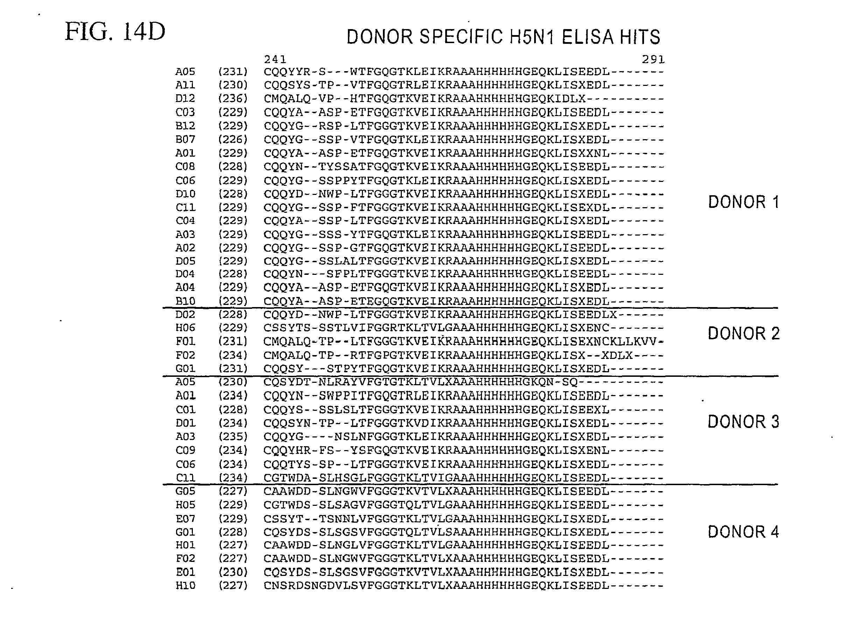

Additional unique H5N1 specific antibody heavy chain variable region sequences were identified from antibody libraries of individual Turkish donors, using the ELISA protocol described above, after four rounds of panning. The sequences of these H5N1 ELISA positive clones are shown in Figures 14A-D.

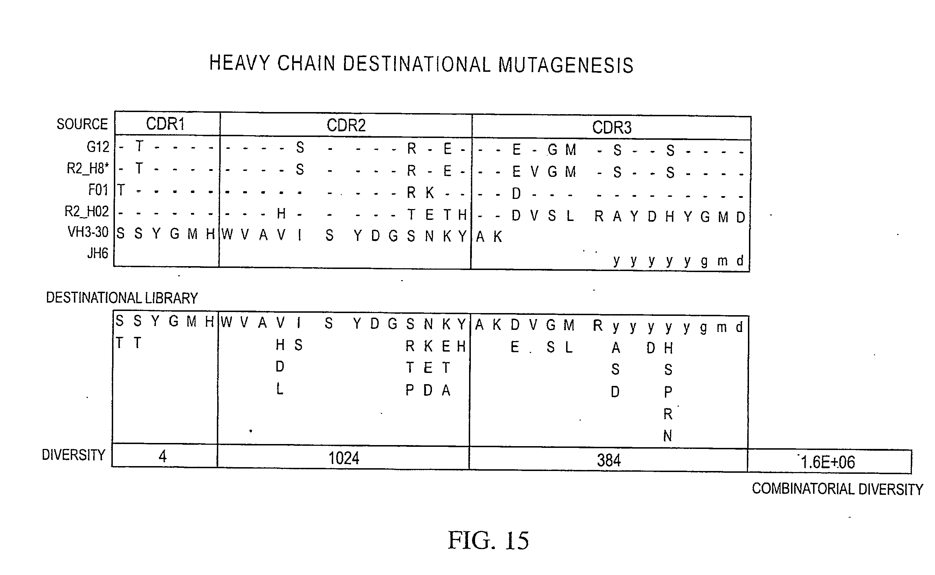

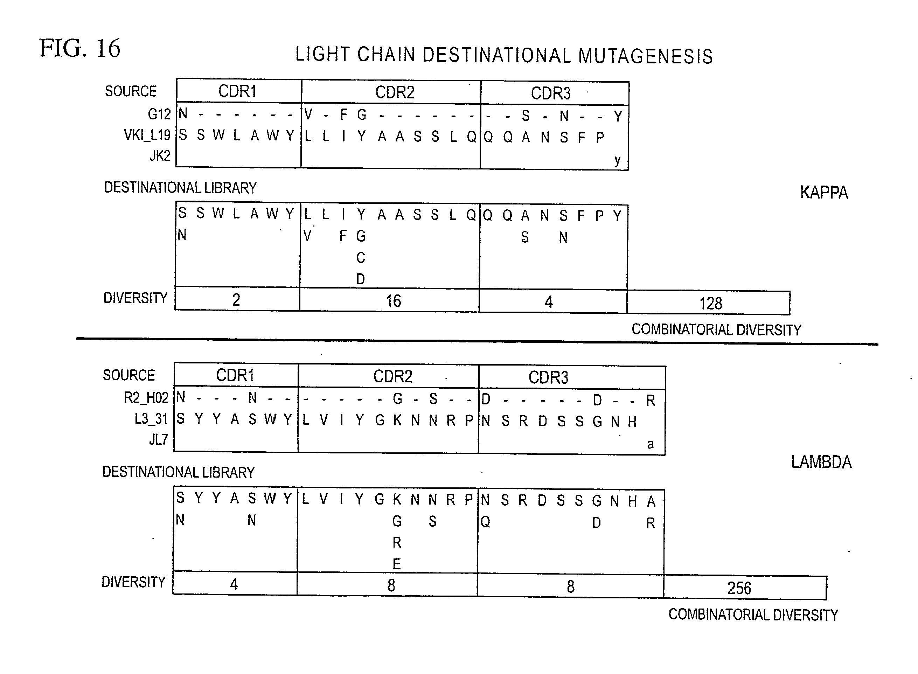

Figures 15 and 16 illustrate the use of destinational mutagenesis to create diverse antibody heavy and light chain libraries using the antibody heavy (Figure 15) and light chain (Figure 16) sequences identified by analysis of sera and bone marrow of Turkish bird flu survivors as described above.

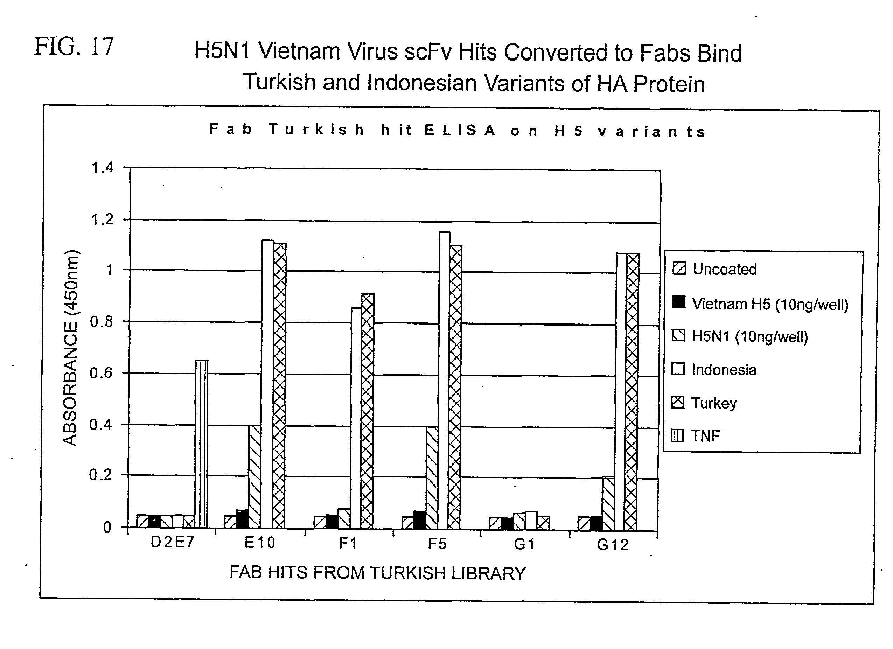

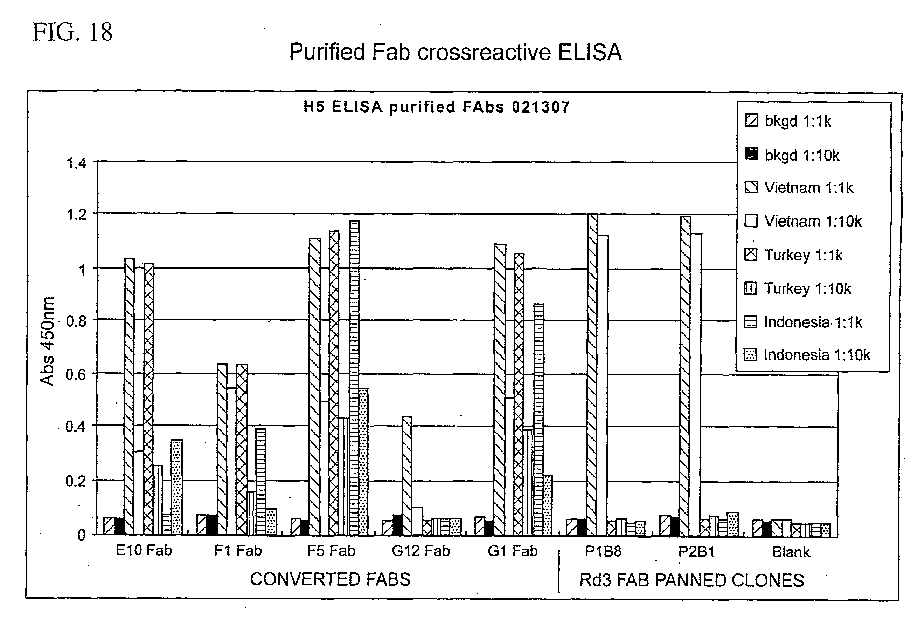

Figures 17 and 18 show ELISA results confirming cross-reactivity of certain Fab fragments obtained from an H5N1 Vietnam virus scFv antibody with Turkish and Indonesian variants of the HA protein.

Although in the foregoing description the invention is illustrated with reference to certain embodiments, it is not so limited. Indeed, various modifications of the invention in addition to those shown and described herein will become apparent to those skilled in the art from the foregoing description and fall within the scope of the appended claims.

All references cited throughout the specification are hereby expressly incorporated by reference.
The following numbered paragraphs (paras.) contain statements of broad combinations of the inventive technical features herein disclosed:-
1. A neutralizing antibody neutralizing more than one isolate of an influenza A virus subtype and/or more than one subtype of the influenza A virus.
2. The neutralizing antibody of para. 1 neutralizing more than one isolate of influenza A virus H1 subtype.
3. The neutralizing antibody of para. 1 neutralizing more than one isolate of influenza A virus H3 subtype.
4. The neutralizing antibody of para. 1 neutralizing influenza A virus HI and H3 subtypes.
5. The neutralizing antibody of para. 4 neutralizing more than one isolates of influenza A virus H1 and/or H3 subtypes.
6. The neutralizing antibody of para. 1 neutralizing substantially all isolates of an influenza A virus subtype.
7. The neutralizing antibody of para. 1 or para. 6 wherein said subtype is selected from the group consisting of H5, H7 and H9 subtypes.
8. The neutralizing antibody of para. 7 wherein said subtype is the H5 subtype.
9. The neutralizing antibody of para. 8 wherein said antibody neutralizes substantially all isolates of the influenza A virus H5 subtype.
10. The neutralizing antibody of para. 7 wherein said subtype is the H7 subtype.
11. The neutralizing antibody of para. 10 wherein said antibody neutralizes substantially all isolates of the influenza A virus H7 subtype.
12. The neutralizing antibody of para. 7 wherein said subtype is the H9 subtype.
13. The neutralizing antibody of para. 12 wherein said antibody neutralizes substantially all isolates of the influenza A virus H9 subtype.
14. The neutralizing antibody of para. 7 which further neutralizes at least one additional H subtype of influenza A virus.
15. The neutralizing antibody of para. 14 wherein said additional H subtype is selected from the group consisting of H1, H2 and H3 subtypes.
16. The neutralizing antibody of para. 15 neutralizing more than one isolate of said additional H subtype of influenza A virus.
17. The neutralizing antibody of para. 1 neutralizing the H5N1 subtype of influenza virus A.
18. The neutralizing antibody of para. 17 neutralizing more than one isolate of the H5N1 subtype of influenza virus A.
19. The neutralizing antibody of para. 18 wherein at least one of said isolates has the ability to infect humans.
20. The neutralizing antibody of para. 19 wherein at least one of said isolates has been obtained from a human subject.
21. The neutralizing antibody of para. 20 wherein said human subject is diseased.
22. The neutralizing antibody of para. 20 wherein said human subject recovered from infection with the H5N1 subtype of influenza virus A.
23. The neutralizing antibody of para. 19 wherein at least one of said isolates has been obtained from a non-human animal.
24. The neutralizing antibody of para. 23 wherein said non-human animal is a bird.
25. The neutralizing antibody of para. 24 wherein said non-human animal is a wild-fowl.
26. The neutralizing antibody of para. 24 wherein said non-human animal is a chicken.
27. The neutralizing antibody of para. 18 neutralizing substantially all isolates of the H5N1 subtype of influenza virus A.
28. The neutralizing antibody of para. 17 neutralizing the H5N1 subtype and at least one additional subtype selected from the group consisting of H1N1, H2N2, and H3N2 subtypes.
29. The neutralizing antibody of para. 28 neutralizing more than isolates of the H5N1 subtype of influenza virus A.
30. The neutralizing antibody of para. 29 neutralizing substantially all isolates of the H5N1 subtype of influenza virus A.
31. The neutralizing antibody of para. 30 neutralizing more than one isolate of said additional subtype.
32. The neutralizing antibody of para. 31 neutralizing substantially all isolates of said additional subtype.
33. The neutralizing antibody of para. 1 wherein said antibody binds to an H5 protein.
34. The neutralizing antibody of para. 33 wherein said antibody binds to more than one variant of the H5 protein.
35. The neutralizing antibody of para. 34 wherein said antibody binds to all variants of the H5 protein.
36. The neutralizing antibody of para. 35 wherein said antibody binds to at least one additional H protein.
37. The neutralizing antibody of para. 36 wherein said additional H protein is selected from the group consisting of HI, H2, and H3 proteins.
38. The neutralizing antibody of para. 37 wherein said antibody binds to more than one variant of said additional H protein.
39. The neutralizing antibody of para. 38 wherein said antibody binds to substantially all variants of said additional H protein.
40. A composition comprising a neutralizing antibody according to any one of paras. 1-39.
41. A method for identifying an antibody capable of neutralizing more than one isolate of an influenza A virus subtype or more than one subtype of an influenza A virus, comprising identifying, in an antibody library, antibodies that react with both a first and a second isolate of said influenza A virus subtype or with a first and a second subtype of said influenza A virus, and subjecting the antibodies identified to successive alternating rounds of selection, based on their ability to bind said first and second isolates, or said first and second subtypes, respectively.
42. The method of para. 41 comprising at least two rounds of selection.
43. The method of para. 41 wherein said first and second isolates are different isolates of the H5N1 subtype of said influenza A virus.
44. The method of para. 41 wherein said antibodies that react with both a first and a second influenza A virus subtype isolate have been identified by at least two rounds of separate enrichment of antibodies reacting with the first isolate and the second isolate, respectively, and recombining the antibodies identified.
45. The method of para. 41 wherein said antibody that can react with both said first and said second influenza A subtype isolate is subjected to mutagenesis prior to being subjected to said successive alternating rounds of selection, based on their ability to bind said first and second isolate, respectively.
46. The method of para. 41 wherein said antibody library is a phage display library.
47. The method of para. 46 wherein selection is performed by biopanning.
48. The method of para. 41 wherein said influenza A virus subtype is an H5N1 subtype.
49. The method of para. 48 wherein said first isolate in a 2006 Turkish isolate of the H5N1 virus.
50. The method of para. 48 wherein said first isolate is a 2003/2004 Vietnam isolate of the H5N1 virus.
51. The method of para. 48 wherein said second isolate is a 2003/2004 Vietnam isolate of the H5N1 virus.
52. The method of para. 50 wherein said second isolate is a 1997 Hong Kong isolate of the H5N1 virus.
53. The method of para. 48 wherein said first and said second isolates originate from different species.
54. The method of para. 53 wherein at least one of said species is human.
55. The method of para. 53 wherein at least one of said species is a bird.
56. The method of para. 41 wherein said antibodies capable of binding said first and said second isolates are additionally selected based on their ability to bind more than one influenza A subtype.
57. A collection of sequences shared by the neutralizing antibodies identified by the method of any one of paras. 41 to 56.
58. A collection of sequences comprising one or more of the unique heavy and/or light chain sequences shown in Figures 11, 12, 13, and 14A-D or a consensus or variant sequence based on said sequences.
59. A neutralizing antibody identifiable by the method of any one of paras. 41 to 56, or a fragment thereof.
60. The neutralizing antibody of para. 59 comprising a heavy and/or light chain sequence selected from the unique sequences shown in Figures 11, 12, 13, and 14A-D, or a consensus or variant sequence based on said sequences, or a fragment thereof.
61. The neutralizing antibody or antibody fragment of para. 59 or para. 60 capable of conferring passive immunity to an avian or mammalian subject against an influenza A virus infection.
62. The neutralizing antibody or antibody fragment of para. 61 wherein said mammalian subject is a human.
63. The neutralizing antibody or antibody fragment of para. 62 wherein said influenza A virus infection is caused by a virus selected from the group consisting one H5N1, HINI, H2N2, and H3N2 subtypes.
64. A method for the prevention and/or treatment of an influenza A infection in a subject comprising administering to said subject an effective amount of a composition of para. 40.
65. A method for treating influenza A infection in a subject comprising administering to said subject an effective amount of a neutralizing antibody of para. 59.
66. The method of para. 64 or para. 65 wherein said subject is a human patient..
67. A method for preventing influenza A infection comprising administering to a subject at risk of developing influenza A infection an effective amount of a composition of para. 40.
68. A method for preventing influenza A infection comprising administering to a subject at risk of developing influenza A infection an effective amount of a neutralizing antibody of para. 59.
69. The method of para. 67 or para. 68 wherein said subject is a human patient.
70. A method for producing a diverse multifunctional antibody collection, comprising (a) aligning CDR sequences of at least two functionally different antibodies, (b) identifying amino acid residues conserved between the CDR sequences aligned, (c) performing mutagenesis of multiple non-conserved amino acid residues in at least one of the CDR sequences aligned, using degenerate oligonucleotide probes encoding at least the amino acid residues present in the functionally different antibodies at the non-conserved positions mutagenized to produce multiple variants of the aligned CDR sequences, and, if desired, repeating steps (b) and (c) with one or more of said variants until said antibody collection reaches a desired degree of diversity or size.
71. The method of para. 70 wherein the CDR sequences aligned have the same lengths.
72. The method of para. 70 wherein the mutagenized variants produced in step (c) retain all conserved residues present in at least two of the CDR sequences aligned.
73. The method of para. 70 wherein the mutagenized variants produced in step (c) retain all conserved residues present in all of the CDR sequences aligned.
74. The method of para. 70 wherein said functionally different antibodies bind to different epitopes on a target antigen.
75. The method of para. 70 wherein said functionally different antibodies bind to different target antigens.
76. The method of para. 75 wherein said different target antigens are variants of the same antigen.
77. The method of para. 70 wherein said functionally different antibodies have different binding affinities.
78. The method of para. 70 wherein said functionally different antibodies have different biological properties.
79. The method of para. 70 wherein said functionally different antibodies bind to an influenza A virus.
80. The method of para. 79 wherein at least two of said functionally different antibodies bind to different epitopes on the same influenza A virus.
81. The method of para. 79 wherein said functionally different antibodies bind to different influenza A virus subtypes.
82. The method of para. 79 wherein at least two of said functionally different antibodies bind to different isolates of the same influenza A virus subtype.
83. The method of para. 79 wherein at least two of said functionally different antibodies bind to different isolates of the same influenza A virus subtype and different influenza A virus subtypes.
84. The method of any one of paras. 70 to 83 wherein at least two of said functionally different antibodies have different binding affinities.
85. The method of any one of paras. 70 to 83 wherein at least two of said functionally different antibodies differ in their ability to neutralize the influenza A virus to which they bind.
86. An antibody collection comprising a plurality of neutralizing antibodies which differ from each other in at least one property.
87. The antibody collection of para. 86 which comprises at least about 100 neutralizing antibodies.
88. The antibody collection of para. 87 prepared by the method of any one of paras. 70 to 83.
89. A method for uniquely identifying nucleic acids in a collection comprising labeling said nucleic acids with a unique barcode linked to or incorporated in the sequences of the nucleic acid present in said collection.
90. The method of para. 89 wherein said barcode is a noncoding nucleotide sequence of one to about 24 nucleotides in length.
91. The method of para. 90 wherein said noncoding nucleotide sequence is linked to the 3' noncoding region of the nucleic acid sequences labeled.
92. The method of para. 89 wherein said barcode is the coding sequence of one or more silent mutations incorporated into the nucleic acid sequences labeled.
93. The method of para. 89 wherein said barcode is a peptide or polypeptide sequence.

## Claims

1. A neutralizing human monoclonal antibody binding to a hemagglutinin (HA) of an influenza A virus, said antibody neutralizing more than one isolate of an H5 influenza A virus subtype.

2. A method for uniquely identifying nucleic acids in a collection comprising labeling said nucleic acids with a unique barcode linked to or incorporated in the sequences of the nucleic acid present in said collection.

3. The neutralizing antibody of claim 1 wherein said antibody binds to an H5 protein.

4. The neutralizing antibody of claim 3 wherein said antibody additionally binds to an H1 protein.

5. The neutralizing antibody of any one of claims 1, 3 or 4 wherein said antibody neutralizes all isolates of the influenza A virus H5 subtype.

6. The neutralizing antibody of any one of claims 1 and 3-5 neutralizing the H5N1 subtype of influenza virus A.

7. The neutralizing antibody of claim 6 neutralizing more than one isolate of the H5N1 subtype of influenza virus A.

8. The neutralizing antibody of any one of claim 1 and 3-7, wherein said antibody neutralizes more than one isolate of the influenza A virus H1 subtype.

9. A composition comprising a neutralizing antibody according to any one of claims 1 and 3 to 8.

10. The composition of claim 9, for use in a method for the prevention and/or treatment of an influenza A infection in a subject.

11. A neutralizing antibody of any one of claims 1 and 3-9, for use in the prevention and/or treatment of an influenza A infection in a subject.

12. A composition according to claim 10 or neutralizing antibody according to claim 11 wherein the subject is a human subject.
